(19) Europäisches Patentamt
European Patent Office
Office européen des brevets

(11) EP 2 789 352 A1

(12) EUROPÄISCHE PATENTANMELDUNG

(43) Veröffentlichungstag:
**15.10.2014 Patentblatt 2014/42**

(21) Anmeldenummer: **14169413.3**

(22) Anmeldetag: **21.12.2011**

(51) Int Cl.:
***A61L 15/32*** (2006.01)
***A61L 27/24*** (2006.01)
***A61K 8/65*** (2006.01)
***A61Q 19/00*** (2006.01)
***A61K 8/02*** (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.12.2010 EP 10196934**

(62) Dokumentnummer(n) der früheren Anmeldung(en) nach Art. 76 EPÜ:
**11194805.5 / 2 468 307**

(71) Anmelder: **MedSkin Solutions Dr. Suwelack AG**
**48721 Billerbeck (DE)**

(72) Erfinder:
- **Malessa, Ralf**
  **45134 Essen (DE)**
- **Kassner, Anja**
  **48161 Münster (DE)**

(74) Vertreter: **Gille Hrabal**
**Brucknerstrasse 20**
**40593 Düsseldorf (DE)**

Bemerkungen:
Diese Anmeldung ist am 22-05-2014 als Teilanmeldung zu der unter INID-Code 62 erwähnten Anmeldung eingereicht worden.

# (54) Degradationsstabilisierte, biokompatible Collagenmatrices

(57) Die vorliegende Erfindung betrifft degradationsstabilisierte, biokompatible Collagenmatrices, die sich insbesondere durch ihren Anteil an löslichen Collagen- und Peptidbestandteilen auszeichnen, Verfahren zur Herstellung solcher Collagenmatrices, die insbesondere die chemische Vernetzung mit einem Epoxy-funktionellen Vernetzungsmittel umfassen, sowie die Verwendung der erfindungsgemäßen Collagenmatrices als kosmetisches oder pharmazeutisches Mittel, insbesondere zur topischen Anwendung sowie als Mittel zur Wundbehandlung, als Implantat oder als Mittel zur Blutstillung bei Menschen oder Tieren, sowie als Gerüststruktur zur Zellbesiedelung im Bereich Biotechnologie, Grundlagenforschung und Tissue Engineering.

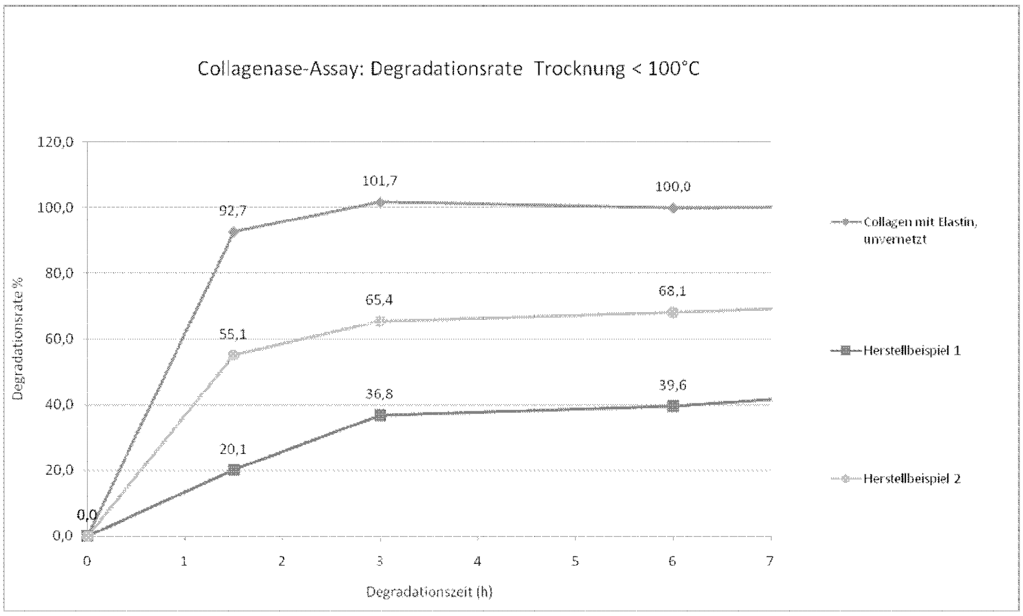


Abb. 2a

EP 2 789 352 A1

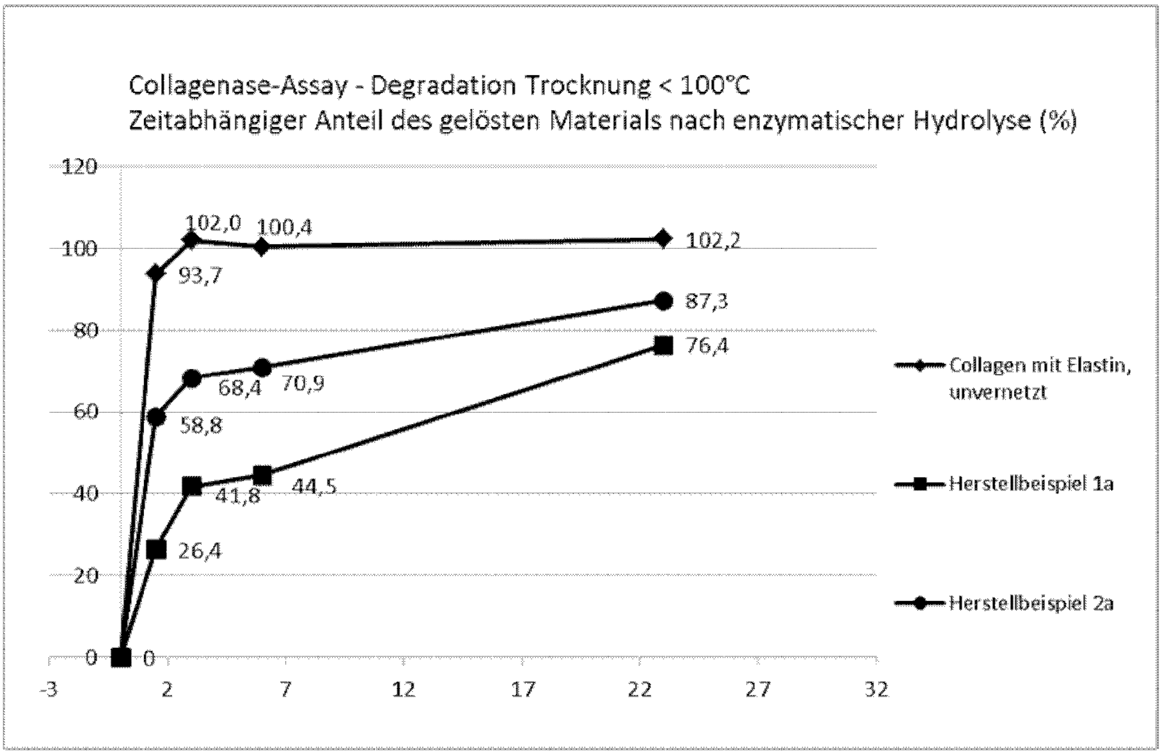
Collagenase-Assay - Degradation Trocknung < 100°C
Zeitabhängiger Anteil des gelösten Materials nach enzymatischer Hydrolyse (%)
120
100
80
60
40
20
0
-3
2
7
12
17
22
27
32
102,0
100,4
102,2
93,7
87,3
76,4
68,4
70,9
58,8
41,8
44,5
26,4
0
Collagen mit Elastin, unvernetzt
Herstellbeispiel 1a
Herstellbeispiel 2a

Abb. 2b

**Beschreibung**

EINLEITUNG UND STAND DER TECHNIK

Die vorliegende Erfindung betrifft degradationsstabilisierte, biokompatible Collagenmatrices, die neben unlöslichen Collagenfasern insbesondere einen Anteil an löslichen Collagen- und Peptidbestandteilen aufweisen, Verfahren zur Herstellung solcher Collagenmatrices, die insbesondere die chemische Vernetzung mit einem Epoxy-funktionellen Vernetzungsmittel umfassen, sowie die Verwendung der erfindungsgemäßen Collagenmatrices als kosmetisches oder pharmazeutisches Mittel, insbesondere zur topischen Anwendung sowie als Mittel zur Wundbehandlung, als Implantat oder als Mittel zur Blutstillung bei Menschen oder Tieren, sowie als Gerüststruktur zur Zellbesiedelung im Bereich Biotechnologie, Grundlagenforschung und Tissue Engineering.

Im Bereich der Collagenmaterialien nimmt die Bereitstellung von mechanisch stabilen, flexiblen, mit ausreichender Stabilität gegen biologische Abbaubarkeit (Degradation) ausgerüsteten Materialien und Gerüststrukturen ("scaffolds") einen hohen Stellenwert ein. Insbesondere im Bereich der Wundbehandlung, der biologischen Implantate sowie im Bereich des Tissue Engineering und der Zellbesiedelung von Gerüststrukturen, aber auch im Bereich hochsorptiver Materialen im Bereich Wundheilung und Blutstillung kommt solchen Produktanforderungen mehr und mehr Bedeutung zu. Im kosmetischen Bereich sind dabei zusätzlich insbesondere eine ansprechende Haptik und Optik bei gleichzeitiger hoher Hautverträglichkeit erwünscht. Desweiteren stellt auch die Bereitstellung von Wirkstoffen über solche degradationsstabilisierten Träger- oder Grundmaterialien, die außerdem über eine hohe mechanische Stabilität verfügen, sowohl in der kosmetischen als auch in der pharmazeutischen Anwendung sowie im Tissue Engineering einen wichtigen Aspekt dar. Die Kombination beispielsweise von "scaffolds" (Gerüsten) des Tissue Engineering mit Wirkstoffen ist eine Strategie, die sich in zahlreichen Publikationen widerspiegelt. Wirkstoffe, welche adsorptiv bzw. kovalent am "scaffold" gebunden oder kontinuierlich aus einen Depot freigesetzt ihre biologische Aktivität über einen größeren Zeitraum entfalten, gehören zum Stand der Technik. Die kontinuierliche Freisetzung aus einem entsprechenden Wirkstoffdepot eröffnet insbesondere bei Wirkstoffen, die eine kurze biologische Halbwertzeit besitzen, gezielte Eingriffe in die Wundheilung. So werden über die Genexpression viele kritische Phänomene, z.B. die Zelldifferenzierung, die Zellproliferation und -aggregation, durch Signalproteine beeinflusst. Die Verfügbarkeit und Differenzierung, die Regeneration oder der Ersatz einzelner Zellen, die Zellteilung und Regulierung stellen Hauptforschungsziele dar (Rui Miguel Paz, Dissertation RWTH Aachen, 2004). Das Ausrüsten von Biomaterialien mit biologisch aktiven Substanzen zur Verbesserung der Funktionalität veranschaulicht die Entwicklung des rein passiven Implantatmaterials zum aktiven Implantat, das mit dem umliegenden Gewebe oder der Gewebeflüssigkeit in gezielte Wechselwirkung tritt. Die Kombinationen von biologisch aktiven Substanzen mit Biomaterialien sind in der Medizin weit verbreitet und spielen zunehmend z.B. bei der Verhinderung implantatbedingter Infektionen eine Rolle. So spielt im Bereich der Wundauflagen ein verbessertes Wundmilieu, eine Reduzierung von Infektionen sowie die Anregung des Zellwachstums durch Zusetzen von Wirkstoffen aber auch durch gezielte Beeinflussung der Zelldifferenzierung und des Expressionsmusters der Zellen über die steuerbare Anpassung der physikalischen Materialeigenschaften, wie insbesondere der Steifigkeit des Matrixmaterials, eine zunehmend wichtigere Rolle. Auch das sogenannte Wundexsudat-Management, welches insbesondere bei chronischen Wunden die Beeinflussung und Interaktion in exsudierenden, d.h. nässenden Wunden, sowohl auf physikalischer Ebene, über rein adsorptive Funktionen, Abführung des Wundexsudats etc., als auch auf pharmakologischer Ebene, Freisetzung von Wirkstoffen, betrifft, hat in den vergangenen Jahren an Bedeutung zugenommen.

Die Beladung von Collagenmatrices mit Protein-Wirkstoffen, wie Wachstumsfaktoren ist seit langem bekannt und wird beispielsweise in der WO 85/04413 oder in der US 5219576 (EP 0428541 B1) beschrieben.

Um darüber hinaus jedoch auch die Produktanforderungen an eine hohe mechanische Stabilität der Trägermaterialien erreichen zu können, sowie eine Verringerung eines unerwünschten zu schnellen biologischen Abbaus oder Degradation zu erzielen, beispielsweise in einer Wunde oder bei Implantaten im Körper, ist die Methode der Vernetzung, insbesondere der Vernetzung mit chemischen Vernetzungsmittel bekannt und weit verbreitet. Insbesondere ist auch bekannt, eine chemische Vernetzung von Collagen mit Epoxy-funktionellen Vernetzungsmitteln vorzunehmen.

Dabei sind vornehmlich Verfahren bekannt, worin die Vernetzung mit dem Epoxy-Vernetzer an einem festen, trockenen, in der Regel einem gefriergetrockneten Collagenmaterial erfolgt. So beschreibt beispielsweise die DE 69533285 Meniskus-Prothesen aus Biopolymerfasern die chemisch vernetzt oder teilvernetzt sein können, wobei die Vernetzung an den bereits gefriergetrockneten Collagenfasern erfolgt.

Auch die WO 2003/053490 A1 (EP 1455855 A1) hat gefriergetrocknete Collagenmaterialien zum Gegenstand, die chemisch vernetzt werden können. Auch die chemische Vernetzung von gefriergetrockneten Collagen-Elastin-Materialien wird hier beschrieben, wobei als chemisches Vernetzungsmittel Glutaraldehyd verwendet wird und Epoxide lediglich allgemein als Vernetzungsmittel genannt werden.

Die DE 102006006461 A1 und die DE 102004039537 A1 haben mit Hautzellen besiedelte Collagenmatrices (Vollhautmodelle) zum Gegenstand, worin die besiedelten Collagenmaterialien einer chemischen Vernetzung mit Glutaraldehyd unterworfen werden. Weitere zahlreiche chemische Vernetzungsmittel werden lediglich allgemein aufgeführt.

Auch hier wird lediglich eine Vernetzung der bereits gefriergetrockneten Materialien beschrieben.

Üblicherweise erfolgt die chemische Vernetzung bei diesen Verfahren durch Tränken der festen Collagenmaterialien, insbesondere solcher in Form von Bögen oder Schichten, mit einer Lösung des Vernetzungsmittels. Die schicht- oder bogenförmigen festen Collagenmaterialien (Layer) werden dabei in der Regel zunächst durch Trocknen (z.B. Gefriertrocknung) von Collagenmaterialien nach üblichen Verfahren erhalten und nach dem Tränken mit dem Vernetzungsmittel einem erneuten Trocknungsschritt (z.B. einer erneuten Gefriertrocknung) unterworfen. In der Regel wird dabei in einem zusätzlichen Schritt das überschüssige, nicht gekoppelte Vernetzungsmittel nach erfolgter Vernetzungsreaktion durch intensives Spülen aus dem Material ausgewaschen, was insbesondere in Hinblick auf die Biokompatibilität bzw. die Verringerung der durch Vernetzungsmittelreste bestehenden Toxizität des vernetzten Materials notwendig ist.

Ein entsprechendes Verfahren wird insbesondere beschrieben in "Cross-linking of Collagen-based materials" (Dissertation R. Zeeman, 1998) sowie von Zeeman et al. in J Biomed Mater Res, 46, 424-433, 1999, in J Biomed Mater Res, 47, 270-277, 1999, in Biomaterials, 20, 921-931, 1999, sowie in J Biomed Mater Res, 51, 541-548, 2000. Darin wird beispielsweise durch Gefriertrocknung erhaltenes schichtförmiges dermales Schafscollagen, sogenanntes "dermal sheep collagen" (DSC-layer) in saurem oder alkalischem pH mit einer Lösung eines Epoxy-funktionellen Vernetzungsmittels wie 1,4-Butandioldiglycidylether (BDDGE) getränkt und bei Raumtemperatur (20 - 30 °C) über einen Zeitraum von mehreren Tagen vernetzt. Anschließend werden die vollständig vernetzten Collagen-Layer gewaschen und erneut gefriergetrocknet, um die Epoxy-vernetzten Collagenmaterialien zu erhalten. In diesen Publikationen wird beschrieben, dass der zur Vernetzung verwendete pH-Wert einen maßgeblichen Einfluss auf die Flexibilität und Elastizität des vernetzten Materials hat, wobei Materialien, die bei einem sauren pH-Wert < 6 (pH 4 - 6) vernetzt wurden, eine höhere Flexibilität und Elastizität zeigen gegenüber den im Alkalischen vernetzten Materialien.

Auch die EP 0898973 B1 beschreibt Verfahren zur chemischen Vernetzung von Collagen. Als mögliches Ausgangsmaterial für die chemische Vernetzung wird hierin prinzipiell auch eine Collagensuspension genannt. Dabei besteht das beschriebene Vernetzungsverfahren jedoch im Wesentlichen aus mindestens zwei Vernetzungsschritten und wird in der Regel bei pH-Werten von 4-9 durchgeführt. Das einzige konkrete Ausführungsbeispiel, worin eine Vernetzung mit Epoxid-Vernetzern wie BDDGE erfolgt, betrifft bogenförmiges dermales Schafscollagen (DSC), wie vorstehend beschrieben, welches mit BDDGE-Lösung getränkt und nach einer Reaktionszeit von 7 Tagen gewaschen und darauf erneut gefriergetrocknet wird.

Nachteilig an solchen Verfahren, worin die Vernetzung durch Tränken eines festen, getrockneten (gefriergetrockneten) Collagenmaterials und anschließender erneuter Trocknung erfolgt, ist einerseits die Notwendigkeit der Durchführung mehrerer Trocknungsschritte, was insbesondere aus verfahrensökonomischen Gründen nachteilig ist, sowie andererseits der hohe Zeitaufwand der insbesondere mit den über mehrere Tage verlaufenden Vernetzungszeiten zusammenhängt. Auch zeigt sich, dass bei erneuter Durchführung einer Gefriertrocknung eines vernetzten, bereits gefriergetrockneten Materials eine unerwünschte Materialveränderung, insbesondere in Form einer Schrumpfung des schichtförmigen Materials beobachtet werden kann. Durch die erhöhte thermische Belastung des Collagenmaterials, die zwangsläufig durch den zusätzlichen zweiten Trocknungsprozess bedingt ist, ist außerdem mit nachteiligen Auswirkungen auf die Struktur des Collagenmaterials zu rechnen, wie beispielsweise einer erhöhten Denaturierung oder anderen Strukturveränderungen in der Collagenpeptidstruktur, insbesondere in Collagenmaterialien, worin das Collagen als natives, biologisches Strukturprotein vorliegt.

Dahingegen ist beispielsweise aus der EP 0793511 A1 ein Verfahren zur Herstellung zusammengesetzter Biopolymerschäume, umfassend unter anderem auch Polymermischungen aus Collagen und Elastin, bekannt, worin die Zugabe des Vernetzungsmittels zu einer Polymersuspension erfolgt und erst abschließend die Durchführung eines einzigen Trocknungsschritts, insbesondere eine Gefriertrocknung, erfolgt. Eine Vernetzung solcher Polymermischungen mit Epoxy-funktionellen Vernetzern wird dabei jedoch nicht offenbart.

Auch in der EP 0680990 A1 wird die Zugabe des Vernetzungsmittels zu einer Polymersuspension beschrieben, wobei hierin Polymermischungen aus Collagen und einem synthetischen hydrophilen Polymer wie insbesondere einem funktionell aktivierten synthetischen hydrophilen Polymer wie beispielsweise einem Glykol, vorzugsweise ein difunktionell aktivierten Polyethylenglycol (PEG) der Vernetzungsreaktion unterworfen werden. Epoxid als Vernetzungsmittel wird lediglich allgemein als ein mögliches unter zahlreichen Vernetzungsmitteln aufgelistet.

Die US 4,883,864 beschreibt chemisch modifizierte (vernetzte) Collagenzusammensetzungen, wobei es sich hierin um mittels Pepsin löslich gemachtes Collagen handelt, welches anschließend durch Zugabe des Vernetzungsmittels vernetzt wird. Epoxid wird in dieser Druckschrift ebenfalls lediglich allgemein als ein mögliches Vernetzungsmittel genannt. Ausführungsbeispiel 18 erwähnt, dass die erfindungsgemäßen Collagenmaterialien gefriergetrocknet und als chirurgisches Schwamm-Material verwendet werden können. Die Verwendung von unlöslichem nativem Collagen oder die konkrete Verwendung von Epoxid, insbesondere im Zusammenhang mit dem Schritt der Gefriertrocknung wird auch hierin nicht offenbart.

Aus der DE 10350654 A1, einer älteren Patentanmeldung der Anmelderin, sind bereits chemisch vernetzte Collagenmaterialien, insbesondere solche aus säureunlöslichem nativem Collagen, bekannt. Darin werden bevorzugt wässrige Collagensuspensionen mit einem pH-Wert von 2-4 eingesetzt. Außerdem wird die Möglichkeit der Zugabe von

Vernetzungsmitteln zu solchen wässrigen Collagensuspensionen und anschließende Gefriertrocknung genannt. Weiterhin wird erwähnt, dass insbesondere bei der Verwendung von Vernetzungsmitteln die im Sauren reagieren die Vernetzungsbehandlung vor der Gefriertrocknung erfolgen kann. Als mögliche Vernetzungsmittel werden verschiedene Gruppen polyfunktioneller Vernetzungsmittel aufgelistet, wobei diese allgemeine Auflistung auch Diepoxide wie 1,4-Butandioldigycidylether (BDDGE) beinhaltet. Bevorzugt wird jedoch eine Dehydrothermalvernetzung durchgeführt. Für Materialien, die mittels EDC (1-Ethyl-3-(3-dimethylaminopropyl)carbodiimid) vernetzt sind, wird außerdem die überraschende Verbesserung der angiogenetischen Eigenschaften des Collagenmaterials erwähnt. Im Zusammenhang mit der darin als bevorzugt hervorgehobenen Dehydrothermalvernetzung werden Gefriertrocknungstemperaturen von mindestens 50 bis 180 °C genannt, wobei für die Dehydrothermalvernetzung höhere Temperaturen über 80 °C als bevorzugt hervorgehoben sind. Eine konkrete Auswahl von Epoxiden als Vernetzungsmittel insbesondere in Kombination mit einer konkreten Verfahrensführung, die beispielsweise einen konkreten pH-Wert < 4 und einen ausgewählten Gefriertrocknungstemperaturbereich berücksichtigt, ergibt sich hieraus nicht. In diesem Dokument wird außerdem die Aufarbeitung und Herstellung einer Collagensuspension aus faserförmigem nativ-unlöslichem Collagen beschrieben. Es ergeben sich dabei jedoch keine Hinweise darauf, die im Rahmen dieser Aufarbeitung vermutlich anfallenden säure-löslichen Collagen- und Peptidbestandteile freisetzbar im Collagenmaterial zu erhalten. Insbesondere im Zusammenhang mit der lediglich allgemein erwähnten chemischen Vernetzung wird weder die Notwendigkeit des Erhalts solcher löslicher Bestandteile noch eine Möglichkeit dafür erwähnt. Im Gegenteil verweist dieses Dokument explizit auf die gewünschte Verwendung von säure-unlöslichem Collagenmaterial und säurelösliches Collagen wird explizit als nachteilig bezeichnet. Die Einbringung beispielsweise proteinogener Wirkstoffe (Wachstumsfaktoren etc.) wird hierin über eine Verkapselung und Einarbeitung von Microsphären erreicht.

Als nativ säure-unlösliches Collagen bezeichnet man üblicherweise die in saurer Lösung unlösliche, durch Zentrifugation präzipitierbare Fraktion einer Collagensuspension, die lichtmikroskopisch sichtbare Fasern enthält, im Sinne der vorliegenden Erfindung umfasst nativ säureunlösliches Collagen insbesondere die in saurer Lösung von pH <4 unlösliche, durch Zentrifugation bei 16.000 g präzipitierbare Fraktion einer reinen Collagensuspension, die Fasern enthält, welche im Lichtmikroskop sichtbar sind (Faserdicke ab 0.2 $\mu$m).

Dahingegen bezeichnet nativ-lösliches bzw. säurelösliches Collagen den Anteil an Collagen, der in saurer Lösung bei pH <4 eine klare Lösung bildet, welche keine unter dem Lichtmikroskop erkennbaren Faserstrukturen beinhaltet.

Das nativ-lösliche bzw. säurelösliche Collagen kann durch Fraktionierung, beispielsweise mittels bekannten Verfahren der SEC (Size-Exclusion-Chromatographie), in höhermolekulare, native, vollständige Collagenmoleküle mit einem Molekulargewicht >250 kDa und niedermolekulare Collagenpeptide mit einem Molekulargewicht <250 kDa getrennt werden. Dabei können die niedermolekularen Collagenpeptide keinem vollständigen Collagenmolekül zugeordnet werden.

Prinzipiell ist das Vorliegen löslicher vollständiger Collagenmoleküle sowie niedermolekularer Peptid-Bestandteile durch Analyse der löslichen Bestandteile mittels bekannten Methoden, beispielsweise SDS-PAGE (Sodium Dodecyl Sulfate-Polyacrylamide Gel Electrophoresis / Natriumdodecylsulfat-Polyacrylamidgelelektrophorese), wie in den nachfolgenden Beispielen dargestellt, qualitativ nachweisbar.

Die säure-löslichen Collagenbestandteile und Collagenpeptide weisen verschiedene vorteilhafte Wirkungen auf. So verfügen sie über filmbildende Eigenschaften und können bei topischer Applikation bzw. Freisetzung aus einem Trägermaterial auf der Haut über diese Filmbildung das Wasserhaltevermögen der Haut verbessern und entsprechend den transepidermalen Wasserverlust verringern. Desweiteren stellen lösliche Collagenfragmente wichtige Signalbotenstoffe und Matrixbestandteile dar, die positive Eigenschaften in der Wundbehandlung sowie in der Steuerbarkeit der Zellbesiedelung / Zellreaktion bewirken, was insbesondere im Bereich des Tissue Engineering in Hinblick auf das sogenannte Expressionsprofil der Zellbesiedelung vorteilhaft und erwünscht ist. Daher liegt ein wichtiger Aspekt der vorliegenden Erfindung darin, solche säure-löslichen Collagenbestandteile und -peptide sowie andere niedermolekulare Peptid-Bestandteile in der gefriergetrockneten, degradationsstabilisierten (chemisch vernetzten) Collagenmatrix zu erhalten, so dass diese bei der Anwendung aus der Matrix freigesetzt werden und am Applikationsort ihre vorteilhafte Wirkung erzielen können.

Die aus dem Stand der Technik bekannten chemisch vernetzten und darüber degradationsstabilisierten Collagenmaterialien enthalten die säure-löslichen Collagenfraktionen, -fragmente und andere Peptidbestandteile entweder aufgrund der grundsätzlichen Materialbeschaffenheit, z.B. bei Spalthautcollagen oder den oben beschriebenen dermal sheep collagen (DSC) Layern, oder aufgrund des durchgeführten Epoxid-Vernetzungsverfahrens und dabei angewendeten Vernetzungsbedingungen nicht mehr. Die im Stand der Technik beschriebenen Epoxy-Vernetzungsverfahren werden unter Verwendung eines deutlichen Überschusses an Vernetzungsmittel in einem hohen Volumenanteil von Lösungsmittel über einen langen Zeitraum von mindestens 72 Stunden durchgeführt. Die im Roh-Material möglicherweise vorhandenen löslichen Fraktionen werden dabei durch den hohen Lösungsmittelanteil freigesetzt und somit ausgewaschen. Durch den hohen Verdünnungseffekt sind lösliche Peptid- und Collagenanteile nur noch geringfügig im Material vorhanden. Die in geringen Maßen noch vorhandenen löslichen, Peptid- und Collagenbestandteile werden durch die

hohen Vernetzungsmittelkonzentrationen und die langen Vernetzungszeiten mit den Collagensträngen vernetzt und damit im vernetzten Material unlösbar gebunden. Da die beschriebenen Verfahren außerdem als zwingenden Schritt das Auswaschen des Vernetzungsmittels aus dem vernetzten Material umfassen, werden spätestens mit diesem Wasch-Schritt möglicherweise noch nicht gebundene lösliche Collagenfraktionen bzw. lösliche Collagenfragmente oder andere niedermolekulare lösliche Peptid-Bestandteile aus dem vernetzten Material ausgewaschen. Damit stehen aus dem Stand der Technik keine chemisch vernetzten Collagenmaterialien zur Verfügung, die gleichzeitig neben einer hohen Degradationsstabilität und mechanischen Festigkeit aufgrund der chemischen Vernetzung auch über eine hohe Biokompatibilität (geringe Toxizität) sowie durch aus dem vernetzten Material freisetzbare säure-lösliche Collagenfraktionen, -fragmente und/oder Peptidbestandteile verfügen.

## AUFGABENSTELLUNG

Die Aufgabe der vorliegenden Erfindung lag somit darin, gefriergetrocknete Collagenmatrices bereitzustellen, die sowohl eine hohe biologische Abbaustabilität (Degradationsstabilität, Hydrolysestabilität) aufweisen als auch über eine für kosmetische und medizinische Applikationen geeignete mechanische Reißfestigkeit, insbesondere in hydratisiertem Zustand (Nassreißfestigkeit) verfügen. Darüber hinaus sollten die Collagenmatrices über eine hohe Biokompatibilität, gekennzeichnet durch eine geringe Toxizität, verfügen sowie die Freisetzung löslicher Collagen- und Peptidbestandteile aus der vernetzten Matrix bei der Anwendung ermöglichen. Darüber hinaus sollte eine hohe Flüssigkeitsaufnahmekapazität und Befeuchtungsgeschwindigkeit der vernetzten Collagenmatrices gegeben sein und die Collagenmatrices sollten über eine hohe Flexibilität und Elastizität sowie über angenehme haptische und optische Eigenschaften (z. B. hohe optische Dichte) verfügen, um insbesondere als kosmetische oder pharmazeutische Mittel, speziell zur Verwendung als kosmetische Auflagen oder Masken sowie als Wundbehandlungsmittel, Implantat oder Blutstillungsmittel sowie als Gerüststruktur zur Zellbesiedelung / Zellkultivierung im Tissue Engineering geeignet zu sein. In einem weiteren Aspekt sollten Collagenmatrices bereitgestellt werden, die durch eine gesteuerte Anpassung der Steifigkeit eine gezielte Beeinflussung der Zelldifferenzierung und des Expressionsmusters der Zellen im Bereich Biotechnologie, Grundlagenforschung und Tissue Engineering ermöglichen. Eine stabile und in Hinblick auf die Beeinflussung des Expressionsmusters von Zellen optimierte Gerüststruktur und hohe Biokompatibilität des Collagenmaterials ist Voraussetzung für ein erleichtertes Einwachsen neuer Zellen und deren Verhalten, beispielsweise bei der Verwendung als Implantat sowie bei der Verwendung als Zellgerüststruktur im Tissue Engineering.

Nicht zuletzt sollte das Verfahren im Vergleich zum Stand der Technik in ökonomischer Sicht überlegen sein.

## BESCHREIBUNG DER ERFINDUNG

Die Erfinder fanden, dass ein derartiges spezifisches, verbessertes Collagenmaterial erhalten werden kann, indem einer wässrigen Suspension aus Collagen, umfassend neben faserförmigem säureunlöslichem nativem Collagen auch Fraktionen nativ-löslichen (säurelöslichen) Collagens und Collagen-Peptide, wie vorstehend definiert, und/oder Strukturbildner bzw. Wirkstoffe aus der Gruppe der Matrixproteine, extrazellulären Matrixbestandteile, proteinogenen Wirkstoffe und löslichen Protein- oder Peptidbestandteile, bei einem pH-Wert $< 4$ ein Epoxy-funktionelles Vernetzungsmittel hinzugefügt wird, diese Mischung anschließend eingefroren und bei einer Temperatur $< 100$ °C gefriergetrocknet wird. Insbesondere zeigte sich überraschend, dass durch dieses neue Verfahren der Gehalt an Epoxid-Vernetzungsmittel gegenüber aus dem Stand der Technik bekannten Vernetzer-Gehalten drastisch verringert werden konnte, ohne Einbußen im Vernetzungsgrad zu erhalten. Ein niedriger pH-Wert $< 4$ sowie reduzierte Vernetzungsmittelgehalte sind dabei insbesondere in Hinblick auf die Epoxid-Restaktivität nach der Gefriertrocknung und den Erhalt der unvernetzten, in der Anwendung freisetzbaren löslichen Collagen- und Peptidbestandteile und damit in Hinblick auf die Toxizität und Biokompatibilität des vernetzten Collagenmaterials vorteilhaft. Geringere Gefriertrocknungstemperaturen als solche, die bei einer Dehydrothermalvernetzung üblicherweise angewendet werden, können einerseits aus ökonomischen Gesichtspunkten vorteilhaft sein, insbesondere sind sie jedoch bekanntermaßen für eine schonende Verarbeitung und den Schutz Temperatur-empfindlicher Inhaltsstoffe (z.B. instabiler Wirkstoffe) essentiell. Ein weiterer Vorteil des erfindungsgemäßen Verfahrens liegt in der Anwendung eines sogenannten Ein-Topf-Verfahrens durch Zugabe des Vernetzers zur Collagensuspension und anschließender direkter Gefriertrocknung zum vernetzten Endprodukt bei geringer Standzeit (Topfzeit) der Collagen-Vernetzer-Mischung von vorzugsweise maximal 24 Stunden vor der abschließenden Gefriertrocknung. Dadurch ist insgesamt eine deutlich ökonomischere Verfahrensführung möglich.

Dadurch konnte die obige Aufgabenstellung insbesondere gelöst werden durch die Bereitstellung eines Verfahrens zur Herstellung mechanisch stabiler und degradationsstabilisierter, biokompatibler Epoxyvernetzter Collagenmatrices, die unvernetzte, in der Anwendung freisetzbare lösliche Collagen- und Peptidbestandteile enthalten, wobei das Verfahren die Schritte umfasst:

a) Herstellen einer wässrigen Collagensuspension

b) Einstellen des pH-Wertes der Collagensuspension aus Schritt a) auf pH < 4,

c) gegebenenfalls Hinzufügen weiterer Strukturbildner, Wirk- und/oder Hilfsstoffe,

d) Hinzufügen eines Epoxy-funktionellen Vernetzungsmittels, wobei die Reihenfolge der Schritte c) und d) variabel ist,

e) Einfrieren der aus Schritt d) erhältlichen Collagenmischung,

f) Gefriertrocknung der gefrorenen Mischung aus Schritt e) bei einer Gefriertrocknungstemperatur < 100 °C

g) optional Einstellen des so erhältlichen gefriergetrockneten vernetzten Collagenmaterials auf einen Feuchtigkeitsgehalt von < 25 Gew.-%, bezogen auf das gefriergetrocknete Collagenmaterial, und

h) gegebenenfalls Überführen der aus Schritt g) erhältlichen Materialien in die gewünschte Form, Sterilisieren und/oder Konfektionieren.

Desweiteren sind Gegenstand der vorliegenden Erfindung auch die nach diesem Verfahren erhältlichen mechanisch stabilen und degradationsstabilisierten, vernetzten, biokompatiblen Collagenmatrices, die unvernetzte, in der Anwendung freisetzbare lösliche Collagen- und Peptidbestandteile enthalten.

Das erfindungsgemäß zur Herstellung der vernetzten Collagenmatrices verwendete Collagen ist insbesondere bovinen, porcinen, equinen, humanen Ursprungs oder es handelt sich um gentechnisch hergestelltes Collagen. Besonders bevorzugt handelt es sich um Collagen bovinen Ursprungs. Die Herstellung eines besonders bevorzugten Collagens ist in der DE 4048622 A1 und in der DE 10350654 A1 der Anmelderin beschrieben. Das dort beschriebene Verfahren zur Herstellung von Collagenschwämmen beinhaltet:

- das Unterwerfen eines Collagenrohmaterials einer Alkalibehandlung,
- Waschen des resultierenden Collagenmaterials,
- das Unterwerfen des resultierenden Collagens einer Säurebehandlung,
- Waschen des resultierenden Collagenmaterials, und
- Zerkleinern des resultierenden Collagenmaterials, insbesondere mit einer Kolloidmühle,

wobei jeder der genannten Schritte ggf. mehrfach wiederholt werden kann.

Dabei wird eine wässrige Collagensuspension von sogenanntem nativ-säureunlöslichem Collagen in Form von Fasern und Fibrillen erhalten, welches außerdem messbare Anteile säurelöslichen Collagens und säurelöslicher Peptid-Bestandteile umfasst. Der qualitative Nachweis solcher säurelöslicher Anteile kann beispielsweise mittels SDS-PAGE, wie hierin beschrieben, erfolgen. Diese Collagensuspension kann dann mit dem erfindungsgemäßen Verfahren durch Einstellung des pH-Wertes auf pH < 4, gegebenenfalls Zugabe weiterer Strukturbildner, kosmetischer oder pharmazeutischer Wirkstoffe sowie Hilfsstoffe und anschließender Zugabe des Epoxy-funktionellen Vernetzungsmittels durch Gefriertrocknung bei einer Gefriertrocknungstemperatur < 100 °C zu dem erfindungsgemäßen Produkt verarbeitet werden.

Die Verwendung eines ausschließlich säurelöslichen Collagenmaterials, wie vielfach im Stand der Technik beschrieben (siehe beispielsweise US 4,883,864) ist gegenüber den in dem erfindungsgemäßen Verfahren verwendeten Collagensuspensionen mit einem überwiegenden Anteil säureunlöslichen Collagens in Form von Fasern und Fibrillen nachteilig, da im Vergleich bei ausschließlich säurelöslichem Collagen deutlich höhere Vernetzungsmittelmengen notwendig sind, um einen ausreichenden Vernetzungsgrad zu erzielen. Es ist bekannt, dass aus rein säurelöslichem Collagen hergestellte Materialien per se im feuchtem Zustand eine unzureichende mechanische Festigkeit (Nassreißfestigkeit) und geringe Degradationsstabilität aufweisen gegenüber solchen, die aus säureunlöslichem Collagen erhältlich sind. Dies ist im Wesentlichen darauf zurückzuführen, dass säurelösliches Collagen aus vereinzelten Collagenmolekülen besteht, wohingegen säureunlösliches Collagen im Wesentlichen aus Collagenfibrillen besteht, welche aus lateral angeordneten, miteinander bereits natürlicherweise vernetzten Collagenmolekülen aufgebaut sind. Diese Fibrillen sind wiederum über natürliche Vernetzung lateral zu größeren Aggregaten, den Collagenfasern, verbunden. Eine Degradation sowohl von bereits natürlich vernetzten als auch von chemisch vernetzten Collagenfasern findet aufgrund der geringeren relativen Oberfläche und Angreifbarkeit von einzelnen Proteinketten im Faserverbund deutlich verlangsamt statt.

Bei dem erfindungsgemäßen Collagenmaterial handelt es sich im Wesentlichen um Collagen des Typs I, III und V, hauptsächlich I.

Die in dem erfindungsgemäßen Verfahren eingesetzte Collagensuspension, die beispielsweise nach den aus der DE 3203957 A1 bekannten Verfahren erhältlich ist, enthält das vorbehandelte und aufgereinigte Collagenmaterial vorzerkleinert, homogenisiert und zerfasert und liegt als eine wässrige Dispersion vor, die das Ausgangsmaterial für die Herstellung der erfindungsgemäßen vernetzten Collagenmatrices darstellt.

Das Trockengewicht der Dispersion sollte etwa 1 bis 4 Gew.-%, vorzugsweise 1,5 bis 2,5 Gew.-% betragen. Der pH-Wert der wässrigen Dispersion sollte < 4 betragen, andernfalls muss der pH-Wert auf pH<4 eingestellt werden. Die Einstellung des pH-Wertes erfolgt bevorzugt mit verdünnter Salzsäure. Bevorzugt wird der pH-Wert der wässrigen Collagensuspension auf pH 2,5 bis 3,5, bevorzugter auf pH 2,7 bis 3,3, ganz besonders bevorzugt auf pH 3 eingestellt.

Die so erhältliche Collagensuspension enthält üblicherweise aufgrund der vorstehend beschriebenen Aufbereitung, insbesondere aufgrund des Zerkleinerungsschritts, neben dem faserförmigen nativsäureunlöslichen Collagen

vorzugsweise auch über die Kolloidvermahlung freigesetzte säure-lösliche Collagen- und Peptidbestandteile, die erfindungsgemäß erwünscht sind. Der Anteil solcher säurelöslichen Bestandteile kann durch die Verfahrensführung gesteuert und auf den gewünschten Anteil eingestellt werden. Bevorzugt ist ein Anteil säurelöslicher Collagen- und Peptidbestandteile in der Collagensuspension von bis zu 7 Gew.-% bezogen auf die Trockenmasse der Collagensuspension, bestimmt durch den Gewichtsanteil der nach Zentrifugation bei 16.000 g erhaltenen, anschließend lyophilisierten löslichen Bestandteile.

Die Zugabe wahlweise vorhandener weiterer Strukturbildner oder gegebenenfalls von kosmetischen bzw. pharmazeutischen Wirkstoffen sowie gegebenenfalls von Hilfsstoffen wie unten beschrieben, in die erfindungsgemäße Collagen-Zusammensetzung kann an dieser Stelle durch Zugeben zur wässrigen Collagensuspension erfolgen. Durch Zugabe weiterer Strukturbildner bzw. Wirkstoffe aus der Gruppe der Matrixproteine, extrazellulären Matrixbestandteile, proteinogenen Wirkstoffe und löslichen Protein- oder Peptidbestandteile etc. kann damit der Anteil der oben genannten löslichen Collagen- und Peptidbestandteile in der Collagensuspension naturgemäß erhöht werden, insbesondere ist es dadurch auch möglich, deutlich höhere Anteile von > 7 Gew.-% (in Trockenmasse) zu erreichen. Wird eine Collagensuspension eingesetzt, die selbst nur geringe Anteile säurelöslicher Collagene und Peptide aufweist, so können solche Bestandteile bevorzugt an dieser Stelle in der gewünschten Menge in die Suspension eingemischt werden.

Anschließend erfolgt die Zugabe des Epoxid-Vernetzungsmittels zu der Collagensuspension. Es ist jedoch genauso möglich, das Epoxid-Vernetzungsmittel vor der Zugabe wahlweise vorhandener weiterer Strukturbildner, Wirk- oder Hilfsstoffe einzumischen. Damit sind diese Schritte im Verfahrensablauf variabel und grundsätzlich gegeneinander austauschbar.

Das Epoxy-funktionelle Vernetzungsmittel wird ausgewählt aus der Gruppe der Epoxidverbindungen, (Epoxide), umfassend insbesondere Diepoxide sowie Polyepoxy-Verbindungen wie Polyglycerinpolyglycidylether mit einem Polymerisationsgrad von 1 bis 3, Polyolpolyglycidylether, Glycoldiglycidylether, Glycerindiglycidylether, Glycerintriglycidylether, Diglycerintetraglycidylether, Ethylenglycolglycidylether, Butandioldiglycidylether, wie insbesondere 1,4-Butandioldiglycidylether, Dicarbonsäurediglycidylester etc.. Die Epoxyfunktionellen Vernetzungsmittel können insbesondere ausgewählt sein aus der Gruppe der Polyethylengycoldiglycidylether gemäß der allgemeinen Formel

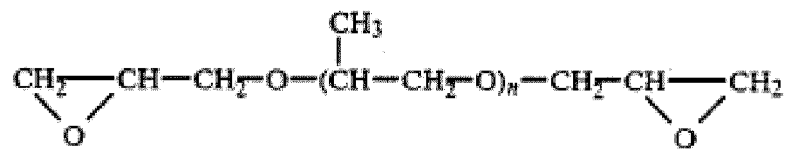

,

oder aus der Gruppe der Polyglycidylether-funktionalen Moleküle von Polyethylenglycol, Polypropylenglycol und Polyethylenpropylenglycol, worin das Diglycidylether-Derivat dargestellt ist durch die allgemeine Formel

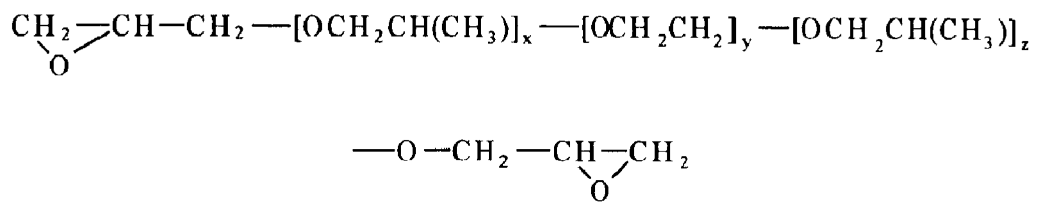

mit x + z = 0 - 70 und y = 0 - 90.

Aus der Gruppe der Diepoxide sind insbesondere solche umfasst, entsprechend der allgemeinen Formel

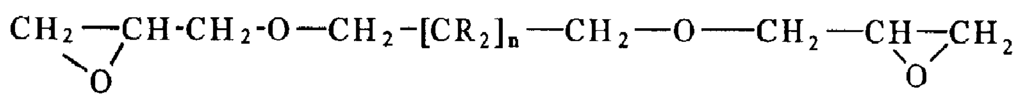

worin R irgendein Substituent sein kann, der nicht mit dem Vernetzungsprozess interferiert und/oder die Wasserlöslichkeit der Vernetzungssubstanz in wässriger Lösung verringert und worin n = 1 - 6, vorzugsweise n = 1 - 4 ist.

Grundsätzlich können die maßgeblichen Eigenschaften der erfindungsgemäßen Collagenmatrices durch die Wahl des geeigneten Epoxid-Vernetzungsmittels, beispielsweise in Hinblick auf dessen Bifunktionalität, Kettenlänge etc. gesteuert werden.

Bevorzugt werden wasserlösliche Epoxid-Vernetzungsmittel, besonders bevorzugt ist das Epoxid-Vernetzungsmittel aus der Gruppe der Diepoxide ausgewählt, wobei 1,4-Butandioldiglycidylether (BDDGE) ganz besonders bevorzugt ist. Die Epoxid-Verbindungen können unter geeigneten Bedingungen sowohl säurekatalysierte als auch basenkatalysierte Reaktionen mit einer Vielzahl von funktionellen Gruppen durchlaufen, einschließlich Amin- und Carboxylgruppen. Im Stand der Technik (z.B. Zeeman et al.) wird beschrieben, dass bei Vernetzung im sauren pH-Bereich (pH 4-6) primär eine Vernetzung der Carboxyl-Gruppen erfolgt, wohingegen bei der alkalischen Vernetzung (pH 9) primär an den Amid-Gruppen vernetzt wird.

Zur Einarbeitung des Vernetzungsmittels sowie möglicher weiterer Inhaltsstoffe aus der Gruppe der löslichen Protein- und Peptidbestandteile, sowie von Wirk- und/oder Hilfsstoffen wird die Collagensuspension bevorzugt auf Temperaturen unterhalb von Raumtemperatur (23 °C) abgekühlt. Insbesondere erfolgt das Einmischen bei einer Temperatur < 20 °C, bevorzugt bei einer Temperatur < 10 °C, besonders bevorzugt bei ≤ 5 °C, wobei an dieser Stelle im Verfahrensablauf die Temperatur der Collagensuspension / -mischung natürlich noch nicht bis zum Gefrieren der Masse abgesenkt wird.

Die so erhaltene, vorstehend beschriebene wässrige Collagensuspension, die das Vernetzungsmittel sowie gegebenenfalls weitere Strukturbildner, Wirkstoffe und/oder Hilfsstoffe enthält (Collagenmischung) und einen pH-Wert < 4 aufweist, wird anschließend eingefroren, vorzugsweise innerhalb von 24 Stunden, so dass die Standzeit (Topfzeit) der wässrigen Collagenmischung 24 Stunden möglichst nicht überschreitet.

Die bevorzugte Temperatur der wässrigen Collagenmischung während dieser Standzeiten (Topfzeiten) entspricht den vorstehend definierten verringerten Temperaturen des Einmischvorgangs und entsprechend wird die Collagenmischung während etwaiger Standzeiten auf einer Temperatur < 20 °C, bevorzugt < 10 °C, besonders bevorzugt ≤ 5 °C, ohne dass die Collagenmischung gefriert, gehalten.

Die verringerten Temperaturen während des Einmischens und der Standzeiten der Collagenmischung vor dem Einfrieren wirken sich überraschenderweise vorteilhaft auf die mechanische Stabilität (Nassreißfestigkeit) der erfindungsgemäßen gefriergetrockneten Collagenmatrices aus. So zeigte sich überraschend, dass mit einer Abnahme der Temperatur während der Standzeit eine Verbesserung der mechanischen Nassreißfestigkeit erzielt werden kann.

Bevorzugt erfolgt das anschließende Einfrieren über einen Zeitraum von 0,5 bis 4 Stunden, vorzugsweise 1 bis 3 Stunden bei einer Temperatur von -10 bis -60 °C.

Überraschend wurde gefunden, dass längere Standzeiten von mehreren Tagen, wie sie im Stand der Technik beschrieben sind, offensichtlich nicht zwingend zur Erzielung einer vollständigen und zufriedenstellenden Vernetzung notwendig sind. Im Gegenteil zeigte sich überraschend, dass sich im erfindungsgemäßen Verfahren längere Standzeiten vor dem Einfrieren nachteilig auf den Vernetzungsgrad und damit auf die Reißfestigkeit (Nassreißfestigkeit) und die Degradationsrate auswirken. Insofern wurde es als vorteilhaft gefunden, in dem erfindungsgemäßen Verfahren die wässrige Collagenmischung unmittelbar nach der Herstellung, innerhalb von maximal 24 Stunden, bevorzugter innerhalb von maximal 18 Stunden, besonders bevorzugt innerhalb von weniger als 12 Stunden, einzufrieren. Neben den vorteilhaften Effekten in Hinblick auf die Materialeigenschaften sind kurze Standzeiten auch aus verfahrensökonomischen Gründen bevorzugt.

Es wird angenommen, dass durch das unmittelbare Einfrieren der wässrigen Suspension die Vernetzungsreaktion zwischen den Peptidmolekülen und den Vernetzungsmittelmolekülen in der wässrigen Mischung unterbunden wird, was aufgrund der wie nachfolgend dargestellten Theorie bezüglich des ablaufenden Reaktionsmechanismus vorteilhaft ist:

> Bei Zugabe des Epoxy-funktionellen Vernetzungsmittels zu der wässrigen Collagensuspension läuft prinzipiell eine Konkurrenzreaktion zwischen Epoxid-Hydrolyse (Epoxid ⇆ $H_2O$) einerseits und Vernetzungsreaktion zwischen Epoxid und Protein (Epoxid ⇆ Protein) andererseits ab. Durch das sofortige Einfrieren werden die Reaktionsbestandteile immobilisiert und die Reaktionen quasi "eingefroren". Außerdem friert dabei das Wasser in Reinstform aus und drängt die gelösten Bestandteile (Epoxid) an die $H_2O$-Kristallperipherie, wodurch das Epoxid in relativer Nähe zu den zu vernetzenden Proteinen konzentriert lokalisiert wird. Durch diese entstehende relative Nähe und erhöhte Konzentration des Epoxids zu den Proteinen ergibt sich vermutlich eine vorteilhafte Reaktionskinetik mit den Collagenfasern und die Konkurrenzreaktion wird in Richtung "Epoxid ⇆ Protein" verschoben. Es wird weiter angenommen, dass mit dem Beginn des Gefriertrocknungsprozesses über den dabei erfolgenden Energieeintrag, durch den die gefrorenen Wassermoleküle durch Sublimation unmittelbar in den Gaszustand überführt werden, auch die Aktivierungsenergie für die Reaktion Epoxid ⇆ Protein zur Verfügung gestellt wird, durch die diese in Gang gesetzt wird. Da darüber hinaus das Wasser durch die Sublimation nicht mehr in den Zwischenzustand der flüssigen Phase gelangt, wird außerdem die konkurrierende Hydrolysereaktion Epoxid ⇆ $H_2O$ unterdrückt.

Somit ist es erfindungsgemäß besonders bevorzugt, die Reaktionszeiten in der wässrigen Suspension möglichst kurz zu halten und diese möglichst schnell in eine gefrorene Form zu überführen.

Aufgrund der vorstehenden Ausführungen ist es insbesondere auch möglich, in dem erfindungsgemäßen Verfahren extrem geringe Mengen des Epoxy-Vernetzers einzusetzen. Erfindungsgemäß werden bevorzugt Epoxy-Konzentrationen bis maximal 50 Gew.-%, bevorzugt bis 20 Gew.-%, bevorzugter bis 10 Gew.-%, besonders bevorzugt bis 7 Gew.-%, jeweils bezogen auf die Trockenmasse der Collagensuspension bzw. bis maximal 1 Gew.-%, bevorzugt bis 0,4 Gew.-%, bevorzugter bis 0,2 Gew.-%, besonders bevorzugt bis 0,14 Gew.-%, jeweils bezogen auf die wässrige Collagensuspension (die ggf. auch weitere Strukturbildner, Wirk- und Hilfsstoffe umfasst) eingesetzt.

Um einen zufriedenstellenden Vernetzungsgrad (Nassreißfestigkeit / Degradationsrate / Hydrolysestabilität) zu erzielen wird das Vernetzungsmittel bevorzugt in einer Menge von mindestens 0,5 Gew.-%, bevorzugter mindestens 1 Gew.-%, noch bevorzugter mindestens 3 Gew-%, jeweils bezogen auf die Trockenmasse der wässrigen Collagensuspension bzw. von mindestens 0,01 Gew.-%, bevorzugt mindestens 0,02 Gew.-%, bevorzugter mindestens 0,06 Gew.-%, jeweils bezogen auf die wässrige Collagensuspension (die ggf. auch weitere Strukturbildner, Wirk- und Hilfsstoffe umfasst), eingesetzt. Dabei hängt die Wahl der geeigneten Vernetzungsmittelkonzentration maßgeblich von den gewünschten Materialeigenschaften und dem jeweiligen Anwendungsgebiet ab.

Im Gegensatz dazu werden im Stand der Technik um ein Vielfaches höhere Epoxid-Konzentrationen eingesetzt. Die Verwendung derart geringer Epoxid-Konzentrationen wirkt sich insbesondere auch besonders vorteilhaft auf die Biokompatibilität, entsprechend der Restaktivität des Epoxid-Vernetzungsmittels (und damit einer geringen Toxizität) im Endprodukt, aus und ermöglicht außerdem den gewünschten Erhalt freisetzbarer säurelöslicher Collagen- und Peptidbestandteile bei der Anwendung des Endproduktes.

Die Epoxid-Restaktivität stellt auf Grund des damit einhergehenden toxischen Potentials ein Maß für die Biokompatibilität der vernetzten Matrices dar. Die Bestimmung der Epoxy-Restaktivität kann mittels eines modifizierten NBP-Assays (Nitrobenzyl-Pyridin-Assays), basierend auf "Detection of Epoxides with 4-(p-Nitrobenzylpyridine" von Agarwal et al. (1979) Bull. Environm. Contam. Toxicol. 23, p. 825-829, modifiziert nach Zocher et al. (2000) "Epoxide hydrolase activity of Streptomyces strains" J. Biotechnol. Feb 17; 77(2-3), p. 287-92, wie hierin im Detail beschrieben, erfolgen.

Die geringe Epoxy-Restaktivität und damit hohe Biokompatibilität der erfindungsgemäßen Collagenmatrices ist vermutlich auch auf den verfahrenstechnisch bevorzugten geringen pH-Wert von < 4 zurückzuführen, da niedrige pH-Werte im Endprodukt eine schnelle Hydrolyse des verbliebenen Rest-Epoxids bewirken und damit dessen Abbaubarkeit im Endprodukt beschleunigen. Dieser Effekt wird maßgeblich auch durch den Feuchtigkeitsgehalt der vernetzten Collagenmatrices (im Endprodukt) beeinflusst, wie nachfolgend weiter ausgeführt.

Weiterhin wurde überraschend gefunden, dass die erfindungsgemäß bevorzugten geringen Epoxid-Konzentrationen einen maßgeblichen Einfluss auf die Reißfestigkeit, die Hydrolysestabilität sowie auf die enzymatische Degradation (z.B. Collagenaseabbau) haben. Optimale Ergebnisse wurden hier in den vorstehend definierten Vorzugsbereichen erzielt.

Das Einfrieren der nach vorstehend beschriebenen Schritten erhältlichen Suspension erfolgt bevorzugt in Form von Platten, prinzipiell sind jedoch auch jegliche anderen denkbaren geometrischen, natürlichen Formen nachempfundene Ausgestaltungen oder physiologische Formen möglich. Die Dicke der resultierenden Platten kann 0,5 bis 5,0 cm, bevorzugt 1,0 bis 3,0 cm, besonders bevorzugt 1,5 bis 2,0 cm betragen.

Bei den nach den erfindungsgemäßen Verfahren erhältlichen Collagenmatrices handelt es sich um poröse Collagenmaterialien, wodurch insbesondere auch deren hohe Adsorptions- oder Feuchtigkeitsaufnahmekapazität und Benetzungsgeschwindigkeit bedingt wird. Auch für die Eignung der erfindungsgemäßen Collagenmatrices als Gerüste zur Zellbesiedelung im Tissue Engineering stellt deren Porosität eine maßgebliche Eigenschaft dar.

Der Porositätsgrad der erfindungsgemäßen Collagenmaterialien ist dabei im wesentlichen eine Funktion zweier Parameter, der Materialdichte und der Eiskristallgröße. Hohe Feststoffgehalte in der wässrigen Suspension erhöhen die Materialdichte im gefriergetrockneten Endprodukt und verringern die Kontaktfläche Rehydratisierungsmittel / Feststoff. Hohe Einfriergradienten führen zu kleinen Eiskristallen, welche zu großen inneren Materialoberflächen führen, was wiederum die Rehydratisierung begünstigt. Niedrige Einfriergradienten hingegen bedingen große Eiskristalle, was wiederum in einer großporigen Materialstruktur im Endprodukt führt. Durch die Steuerung der Einfriergeschwindigkeit kann somit die Porengröße der erfindungsgemäßen Collagenmatrices gezielt beeinflusst werden. Es ist außerdem möglich, die Porengröße zusätzlich durch Hinzufügen oberflächenaktiver Substanzen zu beeinflussen, was jedoch weniger bevorzugt ist.

Die erhaltenen Platten können gegebenenfalls bei -3°C bis -35 °C zwischengelagert werden. Vorzugsweise werden die eingefrorenen Platten mindestens 24 Stunden lang gelagert.

Nach dem Einfrieren und gegebenenfalls Zwischenlagern gelangen die Platten zur Gefriertrocknung.

Überraschend hat sich gezeigt, dass in dem erfindungsgemäßen Verfahren die Gefriertrocknungstemperatur 100 °C nicht übersteigen sollte, um vernetzte Collagenmatrices mit den gewünschten optimalen Eigenschaften hinsichtlich mechanischer Reißfestigkeit, Hydrolysestabilität, Biokompatibilität entsprechend geringer Epoxy-Restaktivität sowie des Erhalts freisetzbarer löslicher Collagen- und Peptidbestandteile bzw. freisetzbarer Strukturbildner bzw. Wirkstoffe aus der Gruppe der Matrixproteine, extrazellulären Matrixbestandteile, proteinogenen Wirkstoffe und löslichen Protein- oder Peptidbestandteile etc. im Endprodukt sowie der haptischen Erscheinung in Form von Flexibilität, Elastizität und Weichheit des Materials zu erhalten.

Aus dem Stand der Technik ist bekannt, relativ hohe Gefriertrocknungstemperaturen von 50 bis 180 °C und

bevorzugter Temperaturen > 80 °C zur Erzielung einer Dehydrothermalvernetzung von Collagenmatrices anzuwenden. Dabei ist weiter bekannt, dass der Vernetzungsgrad und damit die Reißfestigkeit mit der Gefriertrocknungstemperatur zunimmt. Besonders hohe Vernetzungsgrade werden nach dem Stand der Technik beispielsweise mit Gefriertrocknungstemperaturen zwischen 80 und 150 °C bzw. mit Temperaturen über 110 bis 150 °C erzielt. Entsprechend würde man annehmen, dass höhere Gefriertrocknungstemperaturen auch im Zusammenhang mit der Verwendung von Vernetzungsmitteln zu entsprechend besseren Ergebnissen hinsichtlich des Vernetzungsgrades und damit der Reißfestigkeit führen. Es wurde jedoch im Gegenteil gefunden, dass bei dem erfindungsgemäßen Epoxid-Vernetzungsverfahren höhere Gefriertrocknungstemperaturen zu schlechteren Ergebnissen hinsichtlich der Reißfestigkeit (Nassreißfestigkeit) führen. Insbesondere konnte auch festgestellt werden, dass eine dehydrothermale Nachvernetzung bei Temperaturen > 100 °C einen nachteiligen Effekt auf die Nassreißfestigkeit hat. Dieser Effekt war insofern überraschend, als der Fachmann einen additiven Effekt von chemischer Vernetzung und dehydrothermaler Vernetzung erwarten würde.

Es wird angenommen, dass der Vorteil der erfindungsgemäßen Gefriertrocknungstemperaturen von <100°C durch die damit einhergehende Verlängerung des Sublimationsprozesses bedingt wird. Durch die vergleichsweise niedrigeren Gefriertrocknungstemperaturen ist ein verlängerter Sublimationsprozess bis zur vollständigen Trocknung des Materials notwendig, wodurch bedingt ein verlängerter Zeitablauf für die Abreagierung von Vernetzungsmittel und Peptidmolekülen zur Verfügung steht. Entsprechend ist eine Vervollständigung der Vernetzungsreaktion über diesen verlängerten Sublimationsprozess möglich, was zu höheren Vernetzungsgraden führt, wohingegen hohe Gefriertrocknungstemperaturen zu einer beschleunigten Vernetzungsreaktion führt, die dann jedoch inhomogen und ungleichmäßig erfolgt, was zu Endprodukten mit geringerer Reißfestigkeit führt. Entsprechend wird das erfindungsgemäße Verfahren mit einer Gefriertrocknungstemperatur < 100 °C durchgeführt, bevorzugter ist eine Gefriertrocknungstemperaturen < 85 °C, noch bevorzugter < 80 °C.

Wie vorstehend bereits ausgeführt, sind Gefriertrocknungstemperaturen > 100 °C, wie sie für die Erzielung einer zufriedenstellenden Dehydrothermalvernetzung vorteilhaft sind, nachteilig in Hinblick auf den Erhalt freisetzbarer löslicher Collagen- und Peptidbestandteile und/oder Strukturbildner bzw. Wirkstoffe aus der Gruppe der Matrixproteine, extrazellulären Matrixbestandteile, proteinogenen Wirkstoffe und löslichen Protein- oder Peptidbestandteile, da diese einerseits mit vernetzt und damit unlösbar im Material gebunden werden und andererseits bei zu hohen Temperaturen eine unerwünschte - zumindest partielle - Hitze-Denaturierung dieser proteinogenen Bestandteile erfolgen kann.

Nach den im Stand der Technik beschriebenen Vernetzungsverfahren, worin Gefriertrocknungstemperaturen im Bereich von 20 - 30 °C angewendet werden, sind vor der Gefriertrocknung vergleichsweise lange Vernetzungszeiten, bis 44 Stunden bzw. über mehrere Tage (bis zu 7 Tage), notwendig. Dagegen fallen im erfindungsgemäßen Verfahren keine zusätzlichen Vernetzungszeiten vor der Gefriertrocknung an, die Standzeit der Collagensuspension vor der Durchführung der Gefriertrocknung beträgt maximal 24 Stunden und die Vernetzung findet im Wesentlichen anschließend im Verlauf des Gefriertrocknungs-prozesses statt. Insbesondere wurde gefunden, dass eine Gefriertrocknungstemperatur ≥ 40 °C, bevorzugt ≥ 50 °C, bevorzugter ≥ 60 °C vorteilhaft für eine optimale Verfahrensführung ist. Da im erfindungsgemäßen Verfahren die Vernetzungsreaktion im Wesentlichen während der Gefriertrocknung stattfindet, ermöglichen höhere Vernetzungstemperaturen, wie vorstehend definiert, besonders kurze Gefriertrocknungs- und damit besonders kurze Vernetzungszeiten, ohne nachteilige Auswirkungen auf den Vernetzungsgrad und damit die resultierende Materialstabilität zu haben. Darüber hinaus ergibt sich der Zeitvorteil auch aus der Durchführung der Vernetzungsreaktion und der Gefriertrocknung in einem einzigen Schritt.

Optional wird in dem erfindungsgemäßen Verfahren anschließend der Feuchtigkeitsgehalt des erhaltenen gefriergetrockneten vernetzten Collagenmaterials auf einen Feuchtigkeitsgehalt bis maximal 25 Gew.-%, bezogen auf das gefriergetrocknete Collagenmaterial, eingestellt (Rückbefeuchtung). Bevorzugter erfolgt ein Einstellen auf einen Feuchtigkeitsgehalt bis 20 Gew.-%, noch bevorzugter bis 15 Gew.-%, jeweils bezogen auf das gefriergetrocknete Collagenmaterial, Bevorzugt erfolgt eine Rückbefeuchtung bzw. Einstellung des Feuchtigkeitsgehaltes auf mindestens 3 Gew.-%, bevorzugter auf mindestens 5 Gew.-%, noch bevorzugter auf mindestens 7 Gew.-%, jeweils bezogen auf das gefriergetrocknete Collagenmaterial, Insbesondere ist es günstig, den Feuchtigkeitsgehalt auf einen Gehalt zwischen 3 und 25 Gew.-%, bevorzugter zwischen 5 und 20 Gew.-%, noch bevorzugter zwischen 7 und 15 Gew.-%, jeweils bezogen auf das gefriergetrocknete Collagenmaterial, einzustellen. Die Rückbefeuchtung bzw. Einstellung des Feuchtigkeitsgehaltes kann dabei grundsätzlich nach bekannten Verfahren zur Klimatisierung bzw. Feuchtigkeitseinstellung erfolgen. Beispielsweise können die erfindungsgemäßen gefriergetrockneten Collagenmaterialien durch Lagerung bei geeigneten, klimakontrollierten Umgebungsbedingungen z. B. bei einer Temperatur zwischen 10 - 25 °C, einer relativen Luftfeuchtigkeit zwischen 40 - 95 %, bevorzugter zwischen 50-75 % über einen geeigneten Zeitraum beispielsweise zwischen 5 - 120 h, bevorzugter zwischen 12 - 80 h entsprechend eingestellt bzw. konditioniert werden. Grundsätzlich ist ein Fachmann in der Lage, die genannten Parameter Temperatur, Luftfeuchtigkeit und Lagerungszeit in geeigneter Weise zu kombinieren, um bestmögliche Ergebnisse hinsichtlich der gewünschten Feuchtigkeitseinstellung zu erzielen.

Überraschend wurde gefunden, dass eine derartige Einstellung des Feuchtigkeitsgehaltes der gefriergetrockneten Collagenmaterialien vorteilhaft in Hinblick auf die Restaktivität des Epoxid-Vernetzungsmittel im gefriergetrockneten Material ist. Insbesondere konnte mittels der hierin definierten Bestimmungsmethode gezeigt werden, dass durch

geeignete Wahl der Rückbefeuchtungbedingungen (Feuchtigkeitsgehalt, Lagerzeit) die Reduzierung der Epoxy-Restaktivität auf ein nicht mehr nachweisbares Maß deutlich beschleunigt werden konnte. Insbesondere ist es damit möglich, durch die Wahl der Rückbefeuchtungsparameter den Abbau der Epoxy-Restaktivität gezielt zu steuern und damit den Verfahrensablauf ökonomisch vorteilhaft zu beeinflussen.

Die so erhaltenen erfindungsgemäßen Epoxy-vernetzten gefriergetrockneten Collagenmatrices stellen vorzugsweise poröse, schwammartige Formkörper dar, die gegebenenfalls in eine gewünschte Form geschnitten, sterilisiert und konfektioniert werden können.

Dabei erfolgt das Überführen der Collagenmaterialien in die gewünschte Form gemäß Schritt h) zweckmäßig durch Zuschneiden. Dabei kann das Zuschneiden prinzipiell in jegliche gewünschte geometrische Form oder Dicke mit üblichen, bekannten Verfahren erfolgen.

Insbesondere in der therapeutischen Anwendung ist die Sterilisation der erfindungsgemäßen Collagenmatrices von Bedeutung, wobei auch hier auf übliche Verfahren verwiesen wird. Bevorzugt ist eine Sterilisierung mittels gamma-Röntgenstrahlung.

Konfektionieren umfasst ggf. das Bedrucken, Prägen, Stanzen, Perforieren und/oder Laminieren sowie eventuelle Gravur (Veränderung der Oberflächenstruktur) mittels Laser der erfindungsgemäßen Collagenmatrices, sowie das Verpacken.

Die erfindungsgemäßen porösen Collagenmatrices werden bevorzugt in Form eines schichtförmigen Materials bereitgestellt, dass eine Dicke (Dimension, die die geringste Längenausdehnung besitzt) von bevorzugt etwa 0,1 bis 30 mm aufweist, bevorzugt 0,5 - 20 mm, noch bevorzugter 1-10 mm.

Die erfindungsgemäßen vernetzten Collagenmatrices können neben Collagen wahlweise mindestens einen weiteren Bestandteil enthalten, der aus der Gruppe ausgewählt wird, die aus weiteren Strukturbildnern, kosmetischen oder pharmazeutischen Wirkstoffen und/oder Hilfsstoffen besteht.

Aus der Gruppe weiterer Strukturbildner werden bevorzugt Alginate, Elastin, Hyaluronsäure ausgewählt.

Kosmetische Wirkstoffe im Sinne der Erfindung umfassen insbesondere solche Wirkstoffe, die dazu bestimmt sind, äußerlich am Menschen zur Reinigung, Pflege, oder zur Beeinflussung des Aussehens oder des Körpergeruchs, oder zur Vermittlung von Geruchseindrücken angewendet zu werden, es sei denn, dass sie überwiegend dazu bestimmt sind, Krankheiten, Leiden, Körperschäden oder krankhafte Beschwerden zu lindern oder zu beseitigen. In diesem Sinne handelt es sich bei den erfindungsgemäßen Materialien zur kosmetischen Verwendung beispielsweise um Badepräparate, Hautwasch- und -reinigungsmittel, Hautpflegemittel, insbesondere Gesichtshautpflegemittel, Augenkosmetika, Lippenpflegemittel, Nagelpflegemittel, Fußpflegemittel, Haarpflegemittel, insbesondere Haarwaschmittel, Haarkonditionierungsmittel, Haarweichspüler etc., Lichtschutzmittel, Hautbräunungs- und -auf-hellungsmittel, Depigmentierungsmittel, Deodorants, Antihydrotika, Haarentfernungsmittel, Insektenrepellents etc. oder derartige Mittel in Kombination. Insbesondere ist die Verwendung als kosmetische Auflage oder Maske bevorzugt.

Beispiele kosmetisch gegebenenfalls auch z.B. dermatologischer, therapeutisch wirksamer Verbindungen schließen ein: Antiaknemittel, antimikrobielle Mittel, Antitranspirationsmittel, adstringierende Mittel, desodorierende Mittel, Enthaarungsmittel, Konditionierungsmittel für die Haut, hautglättende Mittel, Mittel zur Steigerung der Hauthydratation wie z. B. Dexpanthenol (Panthenol, Pantothenol), Glycerin oder Harnstoff sowie andere NMFs (Natural Moisturing Factors) wie z.B. Pyrrolidoncarbonsäure, Milchsäure und Aminosäuren, Sonnenschutzmittel, Keratolytika, Radikalfänger für freie Radikale, Antioxidationsmittel, Antiseborrhöika, Antischuppenmittel, antiseptische Wirkstoffe, Wirkstoffe zur Behandlung der Anzeichen der Hautalterung und/oder Mittel, die die Differenzierung und/oder Proliferation und/oder Pigmentierung der Haut modulieren, Inhibitoren von Proteasen, z.B. MMP (Matrix-Metallo-Proteinase)-Hemmer, Glycations-Inhibitoren zur Verringerung der Entstehung von AGE (Advanced Glycation Endproduct)-Substanzen, Vitamine wie Vitamin C (Ascorbinsäure) und ihre Derivate, wie beispielsweise Glycoside wie Ascorbylglucosid, oder Ester der Ascorbinsäure wie Natrium- oder Magnesium-Ascorbylphosphat oder Ascorbylpalmitat und -stearat L-Ascorbinsäurephosphatester, Alkalimetallsalze wie Natrium- und Kaliumsalze von L-Ascorbinsäurephosphatestern; Erdalkalimetallsalze wie Magnesium- und Calciumsalze von L-Ascorbinsäurephosphatestern; trivalente Metallsalze wie Aluminiumsalze von L-Ascorbinsäurephosphatestern; Alkalimetallsalze von L-Ascorbinsäuresulfatestern wie Natrium- und Kaliumsalze von L-Ascorbinsäuresulfatestern; Erdalkalimetallsalze wie Magnesium- und Calciumsalze von L-Ascorbinsäuresulfatestern; trivalente Metallsalze wie Aluminiumsalze von L-Ascorbinsäuresulfatestern; Alkalimetallsalze wie Natrium- und Kaliumsalze von L-Ascorbinsäureestern; Erdalkalimetallsalze wie Magnesium- und Calciumsalze von L-Ascorbinsäureestern; und trivalente Metallsalze wie Aluminiumsalze von L-Ascorbinsäureestern, jegliche natürliche, naturidentische und artifizielle Peptide wie z.B. Neuropeptide, antimikrobielle Peptide und Matrikine mit und ohne Modifikation durch kovalente Bindung an eine Fettsäure bzw. Veresterung.

Wirkstoffe mit reizender Nebenwirkung, wie alpha-Hydroxysäuren, β-Hydroxysäuren, alpha-Ketosäuren, β-Ketosduren, Retinoide (Retinol, Retinal, Retin-Säure) Anthralinen (Dioxyanthranol), Anthranoide, Peroxide (insbesondere Benzoylperoxid), Minoxidil, Lithiumsalze, Antimetabolite, Vitamin D und seine Derivate; Katechine, Flavonoide, Ceramide, mehrfach ungesättigte Fettsäuren, essentielle Fettsäuren (z.B. gamma-Linolensäure), Wirkstoffe mit liposomalen Strukturen, Carriersysteme, Enzyme, Coenzyme, Enzymhemmstoffe, hydratisierende Mittel, hautberuhigende

Mittel, Detergentien oder schaumbildende Mittel, und anorganische oder synthetische mattierende Füllstoffe, oder dekorative Stoffe wie Pigmente oder Farbstoffe und -partikel für Foundations, Make-up Formulierungen, und andere Mittel zur kosmetischen Verschönerung und farblichen Gestaltung von Augen-, Lippen-, Gesicht etc. sowie abrasive Mittel.

Weiterhin können Pflanzenwirkstoffextrakte bzw. daraus gewonnene Auszüge oder Einzelstoffe erwähnt werden. Allgemein wird der Pflanzenwirkstoffextrakt in der Regel ausgewählt aus der Gruppe bestehend aus festen Pflanzenextrakten, flüssigen Pflanzenextrakten, hydrophilen Pflanzenextrakten, lipophilen Pflanzenextrakten, einzelnen Pflanzeninhaltsstoffen; sowie deren Mischungen, wie Flavonoide und ihre Aglyka: Rutin, Quercetin, Diosmin, Hyperosid, (Neo)hesperidin, Hesperitin, Ginkgo Biloba (z.B. Ginkoflavonglykoside), Crataegus-Extrakt (z.B. oligomere Procyanidine), Buchweizen (z.B. Rutin), Sophora japonica (z.B. Rutin), Birkenblätter (z.B. Quercetinglykoside, Hyperosid und Rutin), Holunderblüten (z.B. Rutin), Lindenblüten (z.B. ätherisches Öl mit Quercetin und Farnesol), Johanniskrautöl bzw. Johanniskrautextrakt, Nachtkerzenöl, (z.B. Olivenölauszug), Calendula, Arnika (z.B. ölige Auszüge der Blüten mit ätherischem Öl, polare Auszüge mit Flavonoiden), Melisse (z.B. Flavone, ätherisches Öl); Immunstimulantien: Echinacea purpurea (z.B. alkoholische Auszüge, Frischpflanzensaft, Preßsaft), Eleutherokokkus senticosus; Alkoloide: Koffein, Teein, Schwarztee bzw. Schwarztee-Extrakt, Theobromin, Capsaicin, Rauwolfia (z.B. Prajmalin), Immergrün (z.B.Vincomin); weitere Phytopharmaka: Aloe, Roßkastanie (z.B. Aescin), Knoblauch (z.B. Knoblauchöl), Ananas (z.B. Bromelaine), Ginseng (z.B. Ginseno-side), Mariendistelfrüchte (z.B. auf Silymarin standardisierter Extrakt), Mäusedornwurzel (z.B. Ruscogenin), Baldrian (z.B. Valepotriate, Tct, Valerianae), Kava-Kava (z.B. Kavalactone), Hopfenblüten (z.B. Hopfenbitterstoffe), Extr. Passi-florae, Enzian (z.B. ethanol. Extrakt), anthrachinonhaltige Drogenauszüge, z.B. aloinhaltiger Aloe Vera-Saft, Pollenextrakt, Algenextrakte, Süßholzwurzelextrakte, Palmenextrakt, Galphimia (z.B.Urtinktur), Mistel (z.B. wässrig-ethanol, Auszug), Phytosterole (z.B. beta-Sitosterin), Wollblumen (z.B. wäßrig-alkohol. Extrakt), Drosera (z.B. Likörweinextrakt), Sanddornfrüchte (z.B. daraus gewonnener Saft oder Sanddornöl), Eibischwurzel, Primelwurzelextrakt, Frischpflanzenextrakte aus Malve, Beinwell, Efeu, Schachtelhalm, Schafgarbe, Spitzwegerich (z.B. Preßsaft), Brennessel, Schöllkraut, Petersilie; Pflanzenextrakte aus Norolaena lobata, Tagetes lucida, Teeoma siems, Momordica charantia, und Aloe Vera Extrakte, Cardiospermum Urtinktur, Dulcamara Extrakt, sowie Gerbstoffe wie Tannin.

Im Unterschied zu den vorstehend beschriebenen im wesentlichen in der Kosmetik verwendeten Wirkstoffen handelt es sich bei den therapeutischen Wirkstoffen (Arzneimitteln) um solche, die im Sinne des Arzneimittelgesetzes unter anderem dazu bestimmt sind, Krankheiten, Leiden, Körperschäden oder krankhafte Beschwerden zu heilen, zu lindern oder zu verhüten. Erfindungsgemäß sind insbesondere solche Mittel bzw. Wirkstoffe geeignet, die für die äußere oder transdermale Anwendung, insbesondere im Bereich der Wundbehandlung und -heilung sowie im Bereich der Behandlung von Brandverletzungen, insbesondere zur Erstversorgung von Brandwunden bestimmt sind.

Bei Wirkstoffen für eine dermale oder transdermale Anwendung handelt es sich insbesondere um hautaktive aber auch um transdermale Wirkstoffe. Sie schließen beispielsweise ein: Mittel zur Behandlung von Brandverletzungen, Mittel zur Behandlung von Hautkrankheiten, äußerlich anwendbare Analgetika, z. B. Dextropropoxyphen, Pentazocin, Pethidin, Buprenorphin; Antirheumatika/Antiphlogistika (Entzündungshemmer) (NSAR), z. B. Weihrauch bzw. Weihrauchextrakt, Indometacin, Diclofenac, Naproxen, Ketoprofen, Ibuprofen, Flurbiprofen, Salicylsäure und -derivate wie Acetylsalicylsäure, Oxicame; Steroidhormone, z. B. Corticoide und Glucocorticoide wie Hydrocortison, Cortisol, Cortisonacetat, Cloprednol, Prednison, Prednisolon, Deflazacort, Fluocorolon, Triamcinolon, Betamethason, Betamethasonvalerat, Mometasonfuroat, Dexamethason, Methylprednisolon, Ethinylestradiol, Medroergotamin, Dihydroergotoxin; Gichtmittel, z. B. Benzbromaron, Allopurinol; Dermatika Externa, Antihistaminika wie Brompheniramin, Bamipin; Antibiotika wie Erythromycin, Clindamycin, Tetracyclin, einschließlich antibakterielle Mittel wie z. B. kolloidales Silber und Silbersalze wie Silberchlorid, Silbernitrat, Silberjodid oder weitere aus dem Stand der Technik bekannte Silber-haltige Wundbehandlungsstoffe; Antimykotika, Peptidarzneistoffe, antivirale Wirkstoffe, entzündungshemmende Wirkstoffe, juckreizstillende Wirkstoffe wie, anästhesierende Wirkstoffe, z. B. Antihistaminika, Benzocain, Polidocanol oder Corticoide und Glucocorticoide; Aknemittel; antiparasitäre Wirkstoffe; äußerlich anwendbare Hormone; Venentherapeutika; Immunsuppresiva wie Calcineurin-Inhibitoren wie Tacrolimus und Pimecrolimus, Mineralstoffe und Spurenelemente, wie z.B. anorganische oder organische Selenverbindungen, Zink und Zinksalze etc. alle für die dermale oder transdermale Anwendung.

Klarstellend sei angemerkt, dass die Einordnung der Wirkstoffe in die Gruppe der kosmetischen oder therapeutischen Wirkstoffe im Rahmen der vorliegenden Erfindung keine ausschließliche Zuordnung darstellt. Insbesondere schließt die hier vorgenommene Einordnung nicht aus, dass die entsprechenden Wirkstoffe sowohl als kosmetische sowie auch als therapeutische Wirkstoffe Anwendung finden.

Bevorzugte Wirkstoffe für die dermale und transdermale Anwendung sind ausgewählt aus der Gruppe die enthält: Mittel zur Behandlung von Hautkrankheiten wie der Neurodermitis, der atopischen Dermatitis, Psoriasis, Rosacea etc., entzündungshemmende Wirkstoffe, juckreizstillende Wirkstoffe, Gerbstoffe, topische Analgetika, Andsthetika und antibakterielle Wirkstoffe.

Besonders bevorzugt ist dabei mindestens ein Wirkstoff ausgewählt aus der Gruppe der hautähnlichen Lipide, umfassend beispielsweise Phospholipide, neutrale Lipide und Sphingolipide sowie Komponenten des natürlichen Feuchthaltefaktors der Haut, des sogenannten Natural Moisturizing Factors (NMF), umfassend beispielsweise Harnstoff

(Urea), Aminosäuren und Carbonsäuren, Pyrrolidoncarbonsäure, Natrium, Kalium, Calcium, Magnesium, Laktat (Milchsäure), Citrat, Chlorid, Phosphat etc., Harnsäure und andere organische Säuren.

Weiterhin sind insbesondere solche Wirkstoffe bevorzugt, die im Bereich der Wundbehandlung, insbesondere zur Behandlung von chronischen Wunden, Dekubitus, Ulcus cruris, diabetischem Fußsyndrom etc. eingesetzt werden wie beispielsweise Analgetika, z.B. Immunsuppressiva, Hormone, anästhesierende Wirkstoffe, antiparasitäre, fungizide bzw. antimykotische und antibakterielle Wirkstoffe wie insbesondere Silber-haltige Wirkstoffe wie z. B. Silbernitrat, Silberchlorid, Silberjodid, Microsilber-Partikel oder weitere aus dem Stand der Technik bekannte Silber-haltige Wundbehandlungsstoffe, Wirkstoffe zur Unterstützung und Regulierung des Wundmilieus wie insbesondere Elektrolyte, Kieselerde, Mineralstoffe und Spurenelemente wie z. B. Kalium, Magnesium, Calcium, Selen, Iod etc., Wirkstoffe zur Erzielung eines Wunddebridements wie z. B. Kollagenasen oder andere, im Stand der Technik bekannte, geeignete proteolytischen Enzyme sowie Wirkstoffe zur Unterstützung der Wundheilung wie z.B. Wachstumsfaktoren, Enzyminhibitoren, Matrixproteine oder extrazelluläre Matrixbestandteile oder lösliche (niedermolekulare) Protein- und Peptidbestandteile, andere Collagentypen als die in der erfindungsgemäß eingesetzten Collagensuspension bereits enthaltenen Collagene Typ I, III und V,

Besonders bevorzugte Wirkstoffe aus dem Bereich der Wundbehandlungsmittel sind ausgewählt aus Silberhaltigen Wirkstoffen wie insbesondere Silbernitrat, Silberchlorid, Microsilber-Partikel, Tacrolimus, Pimecrolimus, Antihistaminika, Polidocanol, Weihrauch/Weihrauchextrakt, Capsaicin, Tannin, Johanniskrautöl/Johanniskrautextrakt, Nachtkerzenöl, Dexpanthenol sowie anorganischen oder organischen Selenverbindungen, Zink und Zinksalzen.

Weitere bevorzugte Wirkstoffe sind solche aus der Gruppe der proteinogenen Wirkstoffe, umfassend bevorzugt Wachstumsfaktoren, proteinogene Hormone, Enzyme, Coenzyme, Glykoproteine, Blutkoagulationsfaktoren, andere Cytokine und rekombinant hergestellte Varianten der vorstehend genannten Wirkstoffe.

Erfindungsgemäß einsetzbare Wachstumsfaktoren werden bevorzugt aus der Gruppe ausgewählt, die aus VEGF (Vascular Endothelial Growth Factor), bFGF (basic Fibroblast Growth Factor), FGF-1 (saurer Fibroblast Growth Factor), TGF-$\beta$, TGF-$\alpha$ (Transforming Growth Factor $\beta$ bzw. $\alpha$), EGF (Endothelial Growth Factor), HGF (Hepatocyte Growth Factor), TNF-$\alpha$ (Tumor Necrosis Factor $\alpha$), IGF I bzw. II (Insulin-like Growth Factor / Insulin Binding Growth Factor I bzw. II), Heparin Binding Growth Factor I bzw. II, PDGF (Platelet Derived Growth Factor), PD-ECGF (Platelet Derived Endothelial Cell Growth Factor), BMP (Bone Morphogenetic Growth Factor), GHRP (Growth Hormone Release Factor), Cartilage Inducing Factor A bzw. B, Bone Growth Factors, Interleukin 8, Angiopoietin, Angiogenin, Aprotinin, und vWF (von Willebrand Factor) besteht.

Glykoproteine als Wirkstoffe schließen beispielsweise Immunglobuline und Antikörper ein.

Andere Cytokine als Wirkstoffe schließen beispielsweise Interleukine und Interferon ein.

Weitere bevorzugte Wirkstoffe sind solche, die eine blutstillende oder hämostatische Wirkung aufweisen, wie Blutkoagulationsfaktoren wie z.B. Thrombin, Fibrinogen oder Cholesterylsulfat (z.B. Natrium-Cholesterylsulfat) oder Wirkstoffe mit aktivierender Wirkung auf Faktoren und Substanzen der extrinsischen und/oder intrinsischen Gerinnungskaskade wie z. B. Phospholipide, Kaolin, Aprotinin, Faktor- oder Faktoren-Konzentrate, Tissue Factor oder Calcium-Ionen.

Des Weiteren ist denkbar, weitere Wirkstoffe wie Bronchialtherapeutika wie Antiasthmatika, Antitussiva, Mucolytica etc., Antidiabetica wie z. B. Glibenclamid, Hormone, Steroidhormone wie Dexamethason, Herzglycoside wie Digitoxin, Herz- und Kreislauf-therapeutika wie z. B. Beta-Blocker, Antiarrhythmika, Antihypertonika, Calcium-Antagonisten etc., Psychopharmaka und Antidepressiva wie z. B. trizyklische Antidepressiva (NSMRI), Serotonin-Wiederaufnahme-Hemmer (SSRI), Noradrenalin-Wiederaufnahme-Hemmer (NRI), Serotonin-Noradrenalin-Wiederaufnahme-Hemmer (SNRI), Monoaminooxidase-Hemmer (MAO-Hemmer) etc., Neuroleptika, Antikonvulsiva oder Antiepileptika, Hypnotika, Sedativa, Anästhetika, Magen-, Darmtherapeutika, Lipidsenker, Analgetika wie z. B. Antimigränemittel, Paracetamol, Salicylsäure und -derivate wie Acetylsalicylsäure, Diclophenac, Ibuprofen, Ketoprofen, Naproxen etc., Antiphlogistika, Vasodilatatoren, Diuretica, Gichtmittel, Zytostatika, Muskelrelaxantien, Kontrazeptiva z. B. in Form von Hormonpflastern, Suchtentwöhnungsmittel in Form von beispielsweise Nicotinpflastern, Pflanzenextrakte, Provitamine wie z. B. Beta-Carotin, Vitamine wie z. B. Vitamin C, A, B, E etc., über eine transdermale Applikation in einer erfindungsgemäßen Zusammensetzung z. B. in Form eines transdermalen Wirkstoff-Patches zu verabreichen.

Erfindungsgemäß ist es ganz besonders bevorzugt, als weiteren Strukturbildner oder Wirkstoff solche Substanzen zuzugeben, die aus der Gruppe der Matrixproteine, extrazellulären Matrixbestandteile oder löslichen (niedermolekulare) Protein- und Peptidbestandteile, bevorzugt aus der Gruppe umfassend Elastin, Elastin-Hydrolysate, Glykosaminoglykane, wie Heparansulfat, Chondroitinsulfat, Dermatansulfat, Keratansulfat, Heparin und Hyaluronsäure, Proteoglykanen, wie Aggrecan, Fibromodulin, Decorin, Biglycan, Versican, Perlecan, Basalmembran-Proteoglykan hoher Dichte, Syndecan und Serglycin, Fibrin, Fibronectin, Glucane, wie Paramyion etc. ausgewählt sind. Ganz besonders bevorzugte derartige extrazelluläre Matrixbestandteile bzw. Strukturbildner sind Elastin und Elastinhydrolysate, Hyaluronsäure und Fibronectin,

Auch das Collagenmaterial an sich kann gewisse therapeutische Wirkungen, wie insbesondere eine blutstillende Wirkung oder einen positiven unterstützenden Effekt in der Wundheilung aufweisen. Es ist jedoch kein Wirkstoff im

Sinne der Erfindung.

Die vorstehend genannten Wirkstoffe sind allein oder in Kombination mehrerer Wirkstoffe in den vernetzten Collagenmatrices bevorzugt in einer Menge von zweckmäßig bis zu 40 Gew.-%, bevorzugt bis zu 60 Gew.-%, bevorzugter bis zu 80 Gew.-% bezogen auf das gefriergetrocknete Endprodukt vorhanden.

Strukturbildner bzw. Wirkstoffe aus der Gruppe der Matrixproteine, extrazellulären Matrixbestandteile, proteinogenen Wirkstoffe und löslichen Protein- oder Peptidbestandteile können in den gefriergetrockneten Collagenmatrices zusammen mit etwa vorhandenen säurelöslichen Collagen- und Peptidbestandteilen aus der Aufarbeitung der eingesetzten Collagensuspension bevorzugt insgesamt einen Anteil von bis zu 10 Gew.-%, bevorzugter bis 20 Gew.-% bezogen auf die Trockenmasse des gefriergetrockneten Endprodukts, gemessen nach hierin definierter Bestimmungs-Methode (BCA), ausmachen.

Die erfindungsgemäßen Collagenmatrices können wahlweise mindestens einen Hilfsstoff umfassen.

Hilfsstoffe schließen ein: pH-Einstellungsmittel, wie Pufferstoffe, anorganische und organische Säuren oder Basen; Fettsubstanzen, wie Mineralöle, wie Paraffinöle oder Vaselineöle, Siliconöle, Pflanzenöle wie Kokosöl, Süßmandelöl, Aprikosenöl, Maisöl, Jojobaöl, Olivenöl, Avocadoöl, Sesamöl, Palmöl, Eukalyptusöl, Rosmarinöl, Lavendelöl, Kiefernöl, Thymianöl, Minzöl, Kardamonöl, Orangenblütenöl, Sojaöl, Kleieöl, Reisöl, Rapsöl und Rizinusöl, Weizenkeimöl und daraus isoliertes Vitamin E, Nachtkerzenöl, Pflanzenlecithine (z.B. Sojalecithin), aus Pflanzen isolierte Sphingolipide/Ceramide, tierische Öle oder Fette, wie Talg, Lanolin, Butteröl, Neutralöl, Squalan, Fettsäureester, Ester von Fettalkoholen wie Triglyceride, und Wachse mit einem der Hauttemperatur entsprechenden Schmelzpunkt (tierische Wachse, wie Bienenwachs, Carnaubawachs und Candelillawachs, mineralische Wachse, wie mikrokristalline Wachse, und synthetische Wachse, wie Polyethylen- oder Silikonwachse), sowie sämtliche für kosmetische Zwecke geeigneten Öle (sogenannte kosmetische Öle), wie beispielsweise in der CTFA-Abhandlung, Cosmetic Ingredient Hand-book, 1. Auflg., 1988, The Cosmetic, Toiletry and Fragrance Association, Inc., Washington, erwähnt, oberflächenaktive Mittel neben den oben erwähnten Waschtensiden, wie Dispergiermittel, Netzmittel, Emulgatoren etc.; Füllstoffe; Stabilisatoren; Cosolventien; pharmazeutisch und kosmetisch gebräuchliche oder sonstige Farbstoffe und Pigmente, insbesondere solche, die primär zur farblichen Gestaltung der Hydrogel-Zusammensetzung eingesetzt werden und nicht zur Applikation und farblichen Gestaltung am menschlichen Körper, wie solche Pigmente und Farbstoffe wie die unter der Gruppe der Wirkstoffe aufgeführten dekorativen Farbstoffe; Konservierungsmittel; Weichmacher; Schmiermittel bzw. Gleitmittel; etc.

Erfindungsgemäß bevorzugte Hilfsstoffe sind Fette und Öle. Dabei sind insbesondere kosmetische Öle wie vorstehend aufgezählt, insbesondere Triglyceride, besonders bevorzugt Capryl/Capronsäure-Triglyceride, Squalan oder Jojobaöl sowie Nachtkerzenöl bevorzugt.

Die vorstehend genannten Hilfsstoffe sind allein oder in Kombination mehrerer Hilfsstoffe in den vernetzten Collagenmatrices bevorzugt in einer Menge von zweckmäßig bis zu 80 Gew.-%, bevorzugt bis zu 60 Gew.-%, bevorzugter bis zu 40 Gew.-% bezogen auf das gefriergetrocknete Endprodukt vorhanden.

Generell schließt die Einordnung der vorstehend erwähnten Stoffe in die Kategorie der Hilfsstoffe im Rahmen der vorliegenden Erfindung nicht aus, dass diese Hilfsstoffe auch gewisse kosmetische und/oder therapeutische Wirkungen entfalten können, was in besonderem Maß für die genannten bevorzugt eingesetzten kosmetischen Öle gilt.

Enthalten die erfindungsgemäßen Collagenmaterialien eine Kombination von zusätzlichen Inhaltsstoffen aus mindestens zwei der genannten Gruppen, Strukturbildner, Wirkstoffe und/oder Hilfsstoffe, so beträgt der Gesamtanteil dieser zusätzlichen Inhaltsstoffe in der porösen Collagenträgermatrix insgesamt bis 40 Gew.-%, bevorzugt bis 60 Gew.-%, bevorzugter bis 80 Gew.-%, jeweils bezogen auf das gefriergetrocknete Collagenmaterial,

Dabei beträgt der Anteil derartiger Strukturbildner, Wirkstoffe und/oder Hilfsstoffe, entweder allein oder in Kombination aus mindestens zwei der genannten Gruppen vorzugsweise nicht weniger als 0,1 Gew.-%, bevorzugter nicht weniger als 1 Gew.-%.

Wie bereits ausgeführt, liegt ein weiterer Vorteil des erfindungsgemäßen Verfahrens darin, dass durch die besondere Verfahrensführung, insbesondere auch durch die verfahrenstechnisch bedingte geringe Epoxid-Restaktivität und die Möglichkeit der Inaktivierung möglicher Restaktivitäten durch die Feuchtigkeitseinstellung nach der Gefriertrocknung, keine Auswaschung des Epoxid-Vernetzers durchgeführt werden muss, wie dies beispielsweise aus Verfahren nach dem Stand der Technik bekannt ist (z.B. Zeeman-Gruppe). Dies ist nicht nur von Bedeutung, um die säurelöslichen Collagen- und Peptidbestandteile der eingesetzten Collagensuspension freisetzbar in dem gefriergetrockneten, vernetzten Material zu erhalten, sondern gleichfalls auch für weitere Strukturbildner bzw. Wirkstoffe aus der Gruppe der Matrixproteine, extrazellulären Matrixbestandteile, proteinogenen Wirkstoffe und löslichen Protein- oder Peptidbestandteile, wie hierin definiert, z. B. Elastin etc., die der Collagensuspension zusätzlich zugesetzt werden. Als Voraussetzung, dass solche Bestandteile bei der Anwendung ihre positive Wirkung entfalten können, müssen diese bei der Anwendung aus der vernetzten Collagenmatrix, die quasi als Trägermaterial fungiert, freisetzbar sein. Dafür ist unerlässlich, dass solche proteinogenen Wirkstoffe und Bestandteile überwiegend unvernetzt im Material erhalten bleiben und nicht beispielsweise durch die Epoxy-Zugabe im Collagenmaterial mit vernetzt und damit unlösbar gebunden werden. Durch das erfindungsgemäße Verfahren ist es möglich, diese löslichen Matrix-Proteinbestandteile unvernetzt in das Collagenmaterial einzubringen und darin überwiegend unvernetzt zu erhalten, unter anderem auch da in dem erfindungsgemäßen Verfahren

extrem wenig Epoxid-Vernetzungsmittel zur Verfügung steht. Durch diese geringe Menge Vernetzungsmittel erfolgt die Vernetzungsreaktion maßgeblich zwischen den geringen Mengen des Epoxid-Vernetzungsmittels und dem im Überschuss vorliegenden Collagen polypeptiden als Reaktionspartner, anstatt mit den niedermolekularen proteinogenen Matrix-Molekülen.

Insgesamt beträgt der Anteil derartiger löslicher, unvernetzter, in der Anwendung freisetzbarer proteinogener Bestandteile in den erfindungsgemäßen Collagenmatrices mindestens 0,1 Gew.-%, bevorzugt mindestens 2 Gew.-%, besonders bevorzugt ≥ 5 Gew.-%., jeweils bezogen auf die Trockenmasse des gefriergetrockneten Collagenmaterials. Der Anteil solcher freisetzbarer löslicher Bestandteile, umfassend säurelösliche Collagen- und Peptidbestandteile und/oder Strukturbildner bzw. Wirkstoffe aus der Gruppe der Matrixproteine, extrazellulären Matrixbestandteile, proteinogenen Wirkstoffe und löslichen Protein- oder Peptidbestandteile beträgt vorteilhafter Weise maximal bis zu 20 Gew.-%.

Dabei ist der quantitative Anteil solcher löslicher freisetzbarer Collagen- und Peptidbestandteile beispielsweise mittels BCA-Assay (Bi-Chinoic-Acid-Assay) im Vergleich zu Rinder-Serum-Albumin als Standard im Überstand nach Extraktion in 0,9%-NaCl über 24h bei 37°C bestimmbar, wie hierin im Detail dargestellt.

Bei Verfahren nach dem Stand der Technik würden solche löslichen proteinogenen Bestandteile aufgrund der hohen Menge Vernetzungsmittel mit vernetzt und damit unlösbar an das Trägermaterial gebunden, Etwaige unvernetzte Reste würden außerdem durch die im weiteren Verfahrensablauf notwendige Auswaschung des Rest-Epoxid-Vernetzer zur Verringerung der Epoxy-Restaktivität mit aus dem Material ausgewaschen werden.

In einer besonders bevorzugten Ausführungsform des Verfahrens wird der Collagensuspension kein synthetisches hydrophiles Polymer, insbesondere kein funktionell aktiviertes synthetisches hydrophiles Polymer, wie beispielsweise ein Glykol, wie insbesondere ein difunktionell aktiviertes Polyethylenglycol, vor der Zugabe des Vernetzungsmittels zugefügt. Entsprechend betreffen besonders bevorzugte Ausführungsformen der erfindungsgemäßen vernetzten Collagenmatrices solche, die kein Konjugat eines Collagens mit einem synthetischen Polymer umfassen.

Wie vorstehend ausgeführt, weisen die erfindungsgemäßen gefriergetrockneten Epoxy-vernetzten Collagenmatrices spätestens nach dem Schritt der Feuchtigkeitseinstellung keine nachweisbare Epoxy-Restaktivität, gemessen nach hierin beschriebener Methode, mehr auf.

Daneben zeigte sich außerdem, dass die nach dem erfindungsgemäßen Verfahren erhältlichen Collagenmaterialien auch in in-vitro Standard-Verfahren zur Toxizitätsbestimmung (XTT-Test) keine Toxizität aufweisen, was ein weiterer Beleg für die hohe Biokompatibilität derartiger vernetzter Collagenmaterialien ist.

Die erfindungsgemäßen gefriergetrockneten Epoxy-vernetzten Collagenmatrices zeichnen sich außerdem durch eine verbesserte Degradationsstabilität, entsprechend einer verringerten Degradationsrate (verringerter Collagenase-Abbau / Collagenaseverdau), eine verbesserte Hydrolysestabilität sowie eine erhöhte mechanische Stabilität im Sinne einer verbesserten Nassreißfestigkeit, jeweils wie nachfolgend definiert und beschrieben, aus,

Da die Degradationsgeschwindigkeit bzw. Degradationsrate die Stabilität von Collagenmatrices gegen enzymatischen Abbau, insbesondere gegen Collagenaseverdau, wiederspiegelt, ist diese beispielsweise mittels Collagenaseverdautest bestimmbar. Dabei wird der Abbau der Collagenfasern und -fibrillen durch das Enzym Collagenase (Collagenase aus Clostridium histolyticum (Typ 1) in PBS (Phosphate Buffered Saline)-Puffer) unter kontrollierten Bedingungen gezielt herbeigeführt und nach einer definierten Reaktionsdauer mittels UV/VIS Messung in einem Spektralphotometer der Anteil der Zersetzungsprodukte bestimmt, wie in den nachfolgenden Beispielen im Detail dargestellt.

Die erfindungsgemäßen degradationsstabilisierten Collagenmatrices zeichnen sich danach durch eine Verringerung der Degradationsrate, im Sinne einer Verringerung des Anteils an löslichen Zersetzungsprodukten, im Vergleich zu unvernetztem bzw. lediglich dehydrothermal vernetztem gefriergetrocknetem Collagen, bestimmbar mittels Collagenaseverdautest wie hierin beschrieben, aus.

Dabei ist die Degradationsrate einerseits abhängig von der verwendeten Vernetzungsmittelmenge, andererseits jedoch auch vom Anteil möglicherweise zugesetzter löslicher Proteinbestandteile (Matrixproteine etc.). Durch die verwendeten Verfahrensparameter sind die Degradationseigenschaften in Abhängigkeit vom gewünschten Anwendungsgebiet steuerbar.

Die erfindungsgemäßen Collagenmatrices zeigen bevorzugt eine Degradationsrate, gemessen zum Reaktionszeitpunkt 6 Stunden nach Zugabe der Collagenase im Vergleich zu unvernetztem bzw. lediglich dehydrothermal vernetztem gefriergetrocknetem Collagen (Degradationsrate 100 %) von maximal 85 %, bevorzugt maximal 70 %, bevorzugter maximal 50 %. Dies bedeutet eine Verbesserung der Degradationsstabilität um mindestens 15 %, bevorzugt mindestens 30 %, besonders bevorzugt mindestens 50 % im Vergleich zu unvernetztem bzw. nur dehydrothermal vernetztem Collagen.

In einer weiteren Methode zur Bestimmung der Degradationsrate der Collagenmatrix wird die Degradationsgeschwindigkeit der Collagenmatrix bestimmt durch Bestimmung des Gewichtes einer Probe vor und nach enzymatischem Abbau durch Collagenase aus *Clostridium histolyticum* (Typ I, Worthington Biochemicals). Dazu werden definierte Stücke der Collagen-Matrices in eine Lösung eingetaucht, die definierte Einheiten Collagenase in 1 ml PBS (pH 7,2) enthält und unter gelindem Schütteln bei 37°C für 2 Stunden inkubiert. Der Abbau wird durch Zugabe von 0,2 ml einer 0,25 M EDTA-Lösung gestoppt, und es wird für 10 Minuten auf Eis gekühlt. Nachfolgend wird die Probe mit 5 ml PBS-Puffer (pH 7,2) dreimal für 15 Minuten, mit 5 ml entmineralisiertem Wasser dreimal für 15 Minuten gewaschen, bei -80°C über Nacht eingefroren und anschließend gefriergetrocknet. Nach der Gefriertrocknung wird das Gewicht der partiell abgebauten Collagenträgerprobe bestimmt und die Degradationsgeschwindigkeit wie folgt bestimmt:

$$\text{Degradationsgeschwindigkeit (\%)} = 100 \times (\text{Ursprungsgewicht} - \text{Gewicht nach Abbau})/\text{Ursprungsgewicht}$$

Die erfindungsgemäßen Collagenmatrices zeichnen sich danach durch eine Degradationsgeschwindigkeit von höchstens etwa 60 %, bevorzugter höchstens etwa 50 %, noch bevorzugter höchstens etwa 40 % auf, und bevorzugt beträgt die Degradationsgeschwindigkeit mindestens etwa 2 %, bevorzugter mindestens etwa 4 %, noch bevorzugter mindestens etwa 8 %, noch bevorzugter mindestens etwa 10 %.

Je nach Grad der Vernetzung stellen die erfindungsgemäßen vernetzten Collagenmatrices mehr oder weniger schnell resorbierbare Materialien dar. D.h., ein Material mit geringem Vernetzungsgrad wird beispielsweise nach subkutaner bzw. intramuskulärer Anwendung unter den im Organismus herrschenden Bedingungen enzymatisch abgebaut. Das Material kann also nach dem Inkontaktbringen mit der Wunde auf bzw. in dieser verbleiben und dort die Wundheilung fördern. Ein Entfernen des Materials entfällt vorteilhaft. Eine derartige Anwendung ist insbesondere vorteilhaft bei der Verwendung als Wundbehandlungsmittel. Sind solche Wundbehandlungsmittel zum Verbleib in der Wunde vorgesehen, werden sie auch als degradierbare bzw. abbaubare Implantate bezeichnet.

Ein vernetztes Collagenmaterial weist neben einer erhöhten mechanischen Stabilität (Reißfestigkeit) eine erhöhte Stabilität gegen biologische Abbaubarkeit (beispielsweise enzymatische Degradation) auf und ist insbesondere geeignet als semi-permanentes Implantat zur Geweberekonstruktion oder zur Defektauffüllung oder als Gerüststruktur für die Zellbesiedelung, beispielsweise im Bereich des Tissue Engineering,

Eine geringe Degradationsgeschwindigkeit und damit hohe Degradations-Stabilität der Collagenmatrices ist insbesondere auch für die Verwendung als kosmetische Auflage, Zellbesiedelungsgerüst oder in der Verwendung als Wundexsudat-saugendes Schwamm-Material z. B. in einer Vakuum-unterstützten Wundbehandlungstherapie, vorteilhaft.

Auf der anderen Seite ist jedoch eine zu geringe Degradationsrate der Matrix für die Anwendung als Wundbehandlungsmittel und als abbaubares Wundbehandlungs-Implantat unerwünscht, da es zu einer Abkapselung des unvollständig abgebauten Materials in der Wunde bzw. im Körper und damit zum Auftreten von unerwünschter Gewebeverhärtung kommen kann. Es ist daher erfindungsgemäß von besonderer Bedeutung, die Degradationsgeschwindigkeit/-rate für die gewünschte Anwendung optimal einzustellen. Die Degradationseigenschaften des Collagenmaterials werden dabei sowohl durch die Beschaffenheit des, wie vorstehend beschrieben, erhaltenen Materials als auch durch die darauf angewendeten Vernetzungs- bzw. Trocknungsbedingungen bei der Herstellung der erfindungsgemäßen Collagenmatrices beeinflusst. Erfindungsgemäß können mit dem, wie vorstehend beschrieben hergestellten, Collagenmaterial insbesondere in Kombination mit den nachfolgend beschriebenen bevorzugten Herstellungsbedingungen der Collagenmatrix besonders günstige Degradationseigenschaften in Abhängigkeit vom gewünschten Anwendungsgebiet erreicht werden.

Dabei weisen insbesondere Collagenmaterialien für die Anwendung als kosmetische Auflage, als Gerüststruktur für Zellbesiedelungen im Tissue Engineering sowie als Auflagenmaterial zur Verwendung in einer Vakuum-unterstützten Wundbehandlungstherapie eine geringere Degradationsrate von maximal 50 %, entsprechend einer Verbesserung der Degradationsstabilität um mindestens 50 % im Vergleich zu unvernetztem bzw. nur dehydrothermal vernetztem Collagen auf. Hingegen weisen Collagenmaterialien für die Anwendung als Wundbehandlungsmittel oder als degradierbares Wundbehandlungsmittel zum Verbleib in der Wunde (degradierbares Implantat) oder als Implantat zur Geweberekonstruktion sowie zur Unterfütterung tiefer Hautdefekte eine geringere Degradationsrate von maximal 85 bis 70 %, entsprechend einer Verbesserung der Degradationsstabilität um mindestens 15 bis 30 % im Vergleich zu unvernetztem bzw. nur dehydrothermal vernetztem Collagen.

Im Sinne der vorliegenden Erfindung meint Vernetzungseffekt bzw. Vernetzungsgrad einerseits die durch die Vernetzung erreichbare Verringerung der Degradationsrate bzw. eine Erhöhung der Degradationsstabilität der Collagenmatrix sowie andererseits die Verbesserung der mechanischen Stabilität über die Erhöhung der Reißfestigkeit (Nassreißfestigkeit), welche prinzipiell mit üblichen Bestimmungsmethoden, insbesondere mit den hierin beschriebenen Methoden bestimmt werden können, sowie außerdem die Erhöhung der Steifigkeit des Materials, quantifizierbar über die Messung des elastischen Moduls.

So ist beispielsweise die Resistenz einer Collagenstruktur gegenüber Collagenase, beispielsweise bestimmbar nach der hierin beschriebenen Methode (Collagenaseverdau), ein Maß für die Vernetzung, wobei nicht vernetztes Material erheblich schneller enzymatisch abgebaut wird, als dies bei vernetztem Material der Fall ist.

Auch die Hydrolysestabilität stellt ein Maß für die Vernetzung einer Collagenmatrix dar und zeigt sich beispielsweise durch Einlegen einer definierten Menge Collagenmaterials in eine wässrige Lösung und Bestimmung der Veränderung der Materialeigenschaften über die Zeit. Während unvernetzte bzw. dehydrothermal vernetzte Collagenmaterialien unter den gewählten Bedingungen (wässrige Lösung von 50 °C) nach < 18 Tagen eine vollständige Hydrolyse (strukturelle Auflösung der Matrixstruktur) zeigten, wurden bei den erfindungsgemäßen vernetzten Collagenmaterialien unter gleichen Bedingungen keine strukturellen Veränderungen der Matrix beobachtet.

Die Nassreißfestigkeit der erfindungsgemäßen Collagenmatrices kann nach DIN EN ISO 3376 bestimmt werden und beträgt bevorzugt > 50 cN/mm Schichtdicke, bevorzugter >100 cN/mm, noch bevorzugter >300 cN/mm.

Bevorzugt wird jedoch die Nassreißfestigkeit unter Verwendung einer internen Messmethode (UV8801) wie in den nachfolgenden Beispielen beschrieben, bestimmt. Bevorzugte Nass-Reißfestigkeiten der erfindungsgemäßen vernetzten Collagenmatrices, bestimmt nach dieser internen Methode (UV 8801), betragen > 200 cN/mm Schichtdicke, bevorzugter > 400 cN/mm, noch bevorzugter > 500 cN/mm.

Die erfindungsgemäßen gefriergetrockneten Epoxy-vernetzten Collagenmatrices zeigen außerdem eine deutlich verbesserte Absorptions-, Flüssigkeitsaufnahme- und -speicherkapazität sowie eine erhöhte Befeuchtungs- oder Benetzungsgeschwindigkeit gegenüber unvernetzten Collagenmatrices, sowie gegenüber solchen, die lediglich mittels Dehydrothermalvernetzung oder mit anderen bekannten chemischen Vernetzungsmitteln vernetzt wurden.

Die Flüssigkeitsaufnahme- bzw. -speicherkapazität der erfindungsgemäßen Collagenmatrices bezeichnet dabei die Fähigkeit zur Aufnahme von Flüssigkeitsmengen insbesondere in Kombination mit der Fähigkeit diese aufgenommenen Flüssigkeitsmengen zu speichern und zu halten. Dabei sind erfindungsgemäß solche vernetzten Collagenmatrices bevorzugt, die in der Lage sind Flüssigkeitsmengen des 1 bis 200fachen, bevorzugt des 10 bis 100fachen ihres Eigengewichtes aufzunehmen und zu speichern.

Ein Maß für die Menge der Flüssigkeit, die durch das Material aufgenommen werden kann, bezeichnet der Massenquellungsgrad ($Q_m$):

$$Q_m = \frac{m_{\text{Gel}}}{m_{\text{tr. Pr.}}}$$

$Q_m$ bezeichnet dabei das Verhältnis der Masse des gequollenen Materials ($m_{Gel}$) zur Masse des trockenen Materials vor der Quellung ($m_{tr.Pr.}$).

Zur Messung des Massenquellungsgrades wird das gefriergetrocknete Material somit gewogen und anschließend in eine Schale mit einem Überschuss an destilliertem Wasser mit einer Temperatur von 15 - 25 °C auf die Wasseroberfläche gelegt und für 10 Minuten quellen gelassen. Das überschüssige Wasser wird ohne mechanische Einwirkung abgegossen. Nach erneuter Gewichtsmessung der gequollenen Zusammensetzung wird der Massenquellungsgrad gemäß vorstehender Formel ermittelt.

Die erfindungsgemäßen gefriergetrockneten Zusammensetzungen weisen bevorzugt einen Massenquellungsgrad von 15 - 100 auf.

Es ist außerdem möglich, das Flüssigkeitshaltevermögen bezogen auf das Gewicht der Zusammensetzung anzugeben. Dazu wird die im vorstehend beschriebenem Versuchsaufbau ermittelte Gewichtszunahme der gequollenen Materialproben, nachdem die überschüssige Flüssigkeit abgegossen wurde, auf das dieser Gewichtszunahme entsprechende Flüssigkeitsvolumen umgerechnet und dieses aufgenommene Flüssigkeitsvolumen bezogen auf 1 g der eingesetzten Zusammensetzung angegeben.

Die erfindungsgemäßen Collagenmatrices zeichnen sich auch durch eine höhere optische Dichte gegenüber unvernetzten oder lediglich Dehydrothermal-vernetzten Collagenmatrices aus.

Dabei bezeichnet optische Dichte die quantitative Einheit optische Dichte, gemessen als dekadischer Logarithmus des Quotienten von durchgelassener Lichtintensität zu eingestrahlter Lichtintensität, ermittelt mit einem Heiland SW Densitometer TD 03 an schichtförmigen Collagenmatrices mit einer Schichtdicke von 1 m. Die erfindungsgemäßen Collagenmatrices der vorliegenden Erfindung weisen bevorzugt eine optische Dichte von ≥ 0,02, bevorzugter ≥ 0,03, noch bevorzugter ≥ 0,05 pro mm Schichtdicke auf.

Neben vorstehend genannten Vorteilen, die die erfindungsgemäßen Collagenmatrices aufweisen und die maßgeblich aus dem erfindungsgemäßen Verfahren resultieren, weist das erfindungsgemäße Epoxid-vernetzte Collagenmaterial gegenüber unvernetztem oder lediglich Dehydrothermal-vernetztem Collagenmaterial, sowie auch gegenüber mit anderen bekannten chemischen Vernetzungsmitteln vernetzten Collagenmaterialien insbesondere die folgenden vorteilhaften Eigenschaften auf:

- Bessere Haptik: Das Material ist samtiger, weicher, fühlt sich bei identischer Schichtdicke voller bzw. voluminöser an als beispielsweise unvernetztes / dehydrothermal-vernetztes Material. Dies ist insbesondere im kosmetischen Bereich vorteilhaft

- Höhere optische Dichte: vorteilhaft im Bereich der kosmetischen Applikation sowie für Ausführungsformen, die durch farbliche Bedruckungen aufgewertet werden sollen
- Höhere Rückstellkraft: das vernetzte Material kollabiert im nassen Zustand auf mechanische Einwirkungen hin nicht so leicht wie beispielsweise unvernetztes / dehydrothermalvernetztes Material und nimmt leichter wieder sein ursprüngliches Volumen ein, ähnlich einem Schwamm
- Verbesserte Wasseraufnahmekapazität
- Verbesserte Benetzungsgeschwindigkeit
- hohe Elastizität und Flexibilität
- Steuerbarkeit der Zellbesiedelung/Zellreaktion (Antwort der Zelle auf das Material)

Zur Steuerbarkeit der Zellbesiedelung / Zellreaktion ist auszuführen, dass durch das erfindungsgemäße Verfahren eine Möglichkeit gefunden wurde, eine gezielte Steuerung der Materialeigenschaften in Hinblick auf Steifigkeit und Flexibilität, enzymatische Degradierbarkeit (Degradationsrate) und die Bereitstellung freisetzbarer löslicher Collagen- und Peptidbestandteile bzw. weiterer Matrixproteine und löslicher PeptidBestandteile etc., wie vorstehend ausgeführt, vorzunehmen.

In Hinblick auf die Steuerung der Steifigkeit und Flexibilität ist auszuführen, dass bekannt ist, dass die Steifigkeit/Flexibilität von besiedelbaren Matrices einen maßgeblichen Einfluss auf die Eigenschaften der darauf anhaftenden Zellen hat. Der Einfluss der zellulären Umgebung auf die Entwicklung des Zellphänotyps und dessen Expressionsmuster, Migrations- und Proliferationsverhalten ist in den letzten Jahren mehr und mehr in den Vordergrund von Grundlagenforschung im Bereich Tissue Engineering gerückt. Der Steifigkeit (stiffness or rigidity) bzw. Elastizität der extrazellulären Matrix (ECM) ist neben ihrer Zusammensetzung dabei ein Hauptaspekt der jeweiligen zellulären Umgebung.

Die Steifigkeit der extrazellulären Matrix ist ein Regulationssignal für das Verhalten von Zellen. Beispielsweise ist die Differenzierung von Stammzellen direkt von der Steifigkeit des Substrats abhängig, ebenso beruht z.B. das Verhalten von Myofibroblasten auf einer bestimmten Steifigkeit der zellulären Umgebung. Die Zellen reagieren direkt auf die mechanische Rückmeldung ("mechanical feedback") der sie umgebenden ECM ("mechano-chemical sensing")

Zur Erforschung des Zusammenhangs zwischen Steifigkeit des zell-umgebenden Materials und der darauf beruhenden Zell-Antwort ist als Stand der Technik die Verwendung von Gelen unterschiedlicher Steifigkeit und Zusammensetzung bekannt (Mih et al. in PLoS ONE 6 (5): e19929; 2011). Beispiele für synthetische Substrate sind Polyacrylamid Hydrogele unterschiedlichen Polymerisationsgerades sowie Polyelektrolyt-Multilayers (PEM), ebenso werden auch Gele auf Proteinbasis verwendet wie z.B. Collagengele (z.B. nach Yang et al. in Biophysical Journal Vol. 97, 2051-2060; 2009). Die Steifigkeit des Collagengels aus löslichem Collagen hängt dabei direkt von der Konzentration des gelbildenden Proteins ab und kann durch eine zusätzliche Vernetzung z.B. mit Glutaraldehyd oder auch Genipin weiter modifiziert werden.

Zur Quantifizierung der Elastistizität dient das sogenannte Elastizitätsmodul (auch: Young's Modulus) des jeweiligen Materials, E. Dies wird wie folgt definiert:

> Der Elastizitätsmodul (auch: Youngscher Modul, benannt nach dem Physiker Thomas Young) ist ein Materialkennwert aus der Werkstofftechnik, der den Zusammenhang zwischen Spannung und Dehnung bei der Verformung eines festen Körpers bei linear elastischem Verhalten beschreibt. Der Elastizitätsmodul wird mit E-Modul oder als Formelzeichen mit E abgekürzt.

Der Betrag des Elastizitätsmoduls ist desto größer, je mehr Widerstand ein Material seiner Verformung entgegensetzt. Ein Material mit hohem Elastizitätsmodul ist also steif, ein Material mit niedrigem Elastizitätsmodul ist nachgiebig.

Bekannt ist, dass die ECM Steifigkeit im gesunden und kranken bzw. verletzen Gewebe eine breite Verteilung hat.

Beispielsweise liegt die Steifigkeit von Nervengewebe (Gehirn) bei E= ca. 2,5 kPa, die von Muskelgewebe bei E= 12 kPa und die von Knochen bei E = 18 GPa. Für Granulationsgewebe, wie es in der Wundheilung vorliegt, wird eine Steifigkeit von initial 0.1 - 1 kPa und nach 7 Tagen von 18kPa beschrieben (Krishnan et al. in Cell Adhesion & Migration 2:2, 83-94; 2008).

Es besteht ein Interesse daran, Gerüststrukturen für die 3D-Kultivierung im Bereich der Biotechnologie, Grundlagenforschung und Tissue Engineering bereitzustellen, die neben einer hohen Biokompatibilität und einer entsprechenden Degradationsstabilität auch eine für das jeweilige Gewebe spezifische, gezielt einstellbare Steifigkeit aufweisen, um den zu kultivierenden Zellen die optimale Zellumgebung bieten zu können.

Die erfindungsgemäßen Collagenmatrices weisen eine mittels des erfindungsgemäßen Verfahrens einstellbare Steifigkeit im Bereich von 0.01 - 100 kPa, bevorzugt im Bereich zwischen 0,1 und 60 kPa, besonders bevorzugt im Bereich zwischen 2 und 40 kPa auf.

Dadurch besteht mit dem vorliegenden Verfahren zur Vernetzung von Collagenmatrices die Möglichkeit, die

Materialeigenschaften gezielt an die Anforderungen für eine optimale Zellbesiedelbarkeit, Zelldifferenzierung, insbesondere für das sogenannte Expressionsprofil der mit der Matrix in direktem Kontakt stehenden Zellen, anzupassen. Expressionsprofil bezeichnet dabei z.B. die Expression bestimmter MatrixProteine, Cytoskelett-proteine (Aktine), Cytokine, Proteasen etc. Eine derartige Steuerung der Zelldifferenzierung bzw. des Expressionsprofils besiedelter Matrices ist insbesondere im Bereich des Tissue Engineering und der Bioimplantate, aber auch bei der Erstellung von Modellsystemen in der Grundlagenforschung, Diagnostik und Analytik vorteilhaft.

Aufgrund der vorstehend beschriebenen vorteilhaften Eigenschaften der erfindungsgemäßen vernetzten Collagenmatrices sind diese insbesondere geeignet zur kosmetischen und medizinischen, pharmazeutischen oder biotechnologischen Verwendung.

Gegenstand der Erfindung ist somit auch die kosmetische Verwendung der erfindungsgemäßen Collagenmatrices als kosmetisches Mittel, insbesondere als kosmetische Auflage oder Maske.

Dabei erfolgt insbesondere die Anwendung einer erfindungsgemäßen gefriergetrockneten Epoxy-vernetzten Collagenmatrix als kosmetische Auflage oder Maske derart, dass diese entweder trocken auf die zu behandelnde Körperpartie aufgebracht und dort mit Wasser oder einer wässrigen Lösung eines oder mehrerer Wirkstoffe und/oder gegebenenfalls Hilfsstoffe angefeuchtet wird oder worin die Collagenmatrix in Form einer kosmetischen Auflage oder Maske vor dem Aufbringen auf die zu behandelnde Körperpartie mit einer wässrigen Lösung eines oder mehrerer Wirkstoffe und/oder gegebenenfalls Hilfsstoffe angefeuchtet wird.

Eine entsprechende trockene oder vorbefeuchtete Anwendung kann auch bei der Verwendung der erfindungsgemäßen Collagenmatrices als Mittel zur Wundbehandlung erfolgen.

Desweiteren betrifft die vorliegende Erfindung die erfindungsgemäßen gefriergetrockneten Epoxy-vernetzten Collagenmatrices zur Verwendung als pharmazeutisches Mittel (umfassend auch Medizinprodukte), insbesondere zur topischen bzw. dermalen Anwendung oder zur Implantation bei Menschen oder Tieren.

Insbesondere bevorzugt sind die erfindungsgemäßen Collagenmatrices zur Verwendung als Implantat, als dermales oder transdermales Hautbehandlungsmittel und/oder als Mittel zur Blutstillung sowie als Gerüststruktur zur Zellbesiedelung im Bereich Tissue Engineering.

Weiterhin betrifft die vorliegende Erfindung die erfindungsgemäßen Collagenmatrices zur Verwendung in mindestens einer Indikation bzw. Anwendung, die aus der folgenden Gruppe ausgewählt ist, die besteht aus: Behandlung von akuten oder chronischen Wunden, Verbesserung der Wundheilung, Ausgleich von Gewebedefekten, Unterfütterung tiefer Hautdefekte mit Volumenaufbau, Unterstützung der Geweberegeneration, Regeneration der Dermis, Behandlung von Brandwunden, Verwendung in der plastischen Chirurgie, Verwendung nach Narbenexzision, Kombinationstherapie mit autologen Spalthauttransplantaten, Unterstützung der Bildung von Granulationsgewebe, Unterstützung der Angiogenese, Gewährleistung einer besseren Narbenqualität, Behandlung chronischer Wunden wie Ulcus Cruris, Dekubitus und diabetischer Fuß, Behandlung offener Wunden, Behandlung von Wundheilungsstörungen, Behandlung von Erkrankungen mit tiefen Hautdefekten, Herstellung eines Kieferimplantats, Herstellung eines Knochenimplantats, Herstellung eines Knorpelimplantats, Herstellung eines Gewebeimplantats, Herstellung eines Hautimplantats, Herstellung einer medizinischen Auflage, Herstellung einer transdermalen Auflage, Herstellung eines Wundpflasters, Herstellung eines Wundverbandmaterials, Herstellung einer Wundauflage und Herstellung einer Zellzuchtmatrix zur Zellvermehrung für die Implantation von Zell-Matrix-Einheiten sowie in der Biotechnologie bei der Erstellung von Modellsystemen für die in-vitro-Nachstellung von Gewebesystemen (z.B. Hautmodell) für Grundlagenforschung, Diagnostik und Analytik.

Des weiteren können die erfindungsgemäßen Collagenmatrices auch in einer Vakuum-unterstützten Wundbehandlungstherapie, wie sie prinzipiell aus dem Stand der Technik bekannt ist und wie beispielsweise in der US 2007/0027414 beschrieben, verwendet werden. Dabei können die erfindungsgemäßen Collagenmatrices in einer solchen Vakuum-Behandlung aufgrund ihrer hohen Flexibilität gut in das Wundbett eingebracht werden und dort aufgrund ihrer guten Absorptions- und Hydratationseigenschaften den Abtransport des überschüssigen Wundsekrets positiv unterstützen. Dabei wird der Sekret-Transport durch das permeable, poröse Collagenmatrix-Material einerseits bereits durch dessen grundsätzlich hohe Hydrophilie und Quellbarkeit erreicht. Darüber hinaus verfügen die erfindungsgemäßen Collagenmatrices aufgrund des Gefriertrocknungsprozesses über eine hohe Porosität, was den Flüssigkeitsdurchtritt zusätzlich erleichtert. Dabei ist außerdem vorteilhaft, dass die erfindungsgemäßen Collagenmatrices, insbesondere auch aufgrund der darin enthaltenen freisetzbaren löslichen Collagen-, Peptid und Proteinbestandteile, an sich bereits einen positiven Einfluss auf den Wundheilungsverlauf aufweisen.

Die erfindungsgemäßen Collagenmatrices sind für die vorstehenden Anwendungen insbesondere auch aufgrund ihrer hohen Biokompatibilität, entsprechend der geringen Epoxy-Restaktivität und der enthaltenen freisetzbaren löslichen Collagen-, Peptid und Proteinbestandteile, besonders geeignet.

Für die vorstehend beschriebenen bevorzugten Anwendungsgebiete der erfindungsgemäßen vernetzten Collagenmatrices liegen diese bevorzugt in Form schichtförmiger Masken, Sheets, Matrices, Auflagen, Pads, Lagen oder ähnlichen flächigen Ausgestaltungen vor. Solche schichtförmigen Ausführungsformen eignen sich besonders für die äußerliche und flächige Behandlung auch größerer betroffener Regionen der Haut.

In einer weiteren möglichen Ausführungsform können solche schichtförmigen Collagenmatrices auch vollständig oder teilweise laminiert sein bzw. in Form mehrschichtiger, miteinander verbundener Lagen ("sandwich-layer") vorliegen. Als Laminate können übliche, aus dem Stand der Technik bekannte Materialien wie z.B. Fasern, Vliese, Netze, Filme oder Folien aus geeigneten Materialien wie z.B. Rayon, Cellulose, Polyethylen (PE) oder Polyurethan (PU) oder anderen synthetischen oder semi-synthetischen Polymeren / Copolymeren verwendet werden, die mit herkömmlichen Methoden, z.B. durch Kleben, Heißlaminieren, Vernetzen etc. mit den Trägermaterialien im Sinne der vorliegenden Erfindung fest verbunden werden können. Eine derartige Laminierung ist besonders geeignet, um die mechanische Stabilität der erfindungsgemäßen schichtförmigen Collagenmaterialien zusätzlich zu erhöhen, sowie deren Handhabbarkeit bei der Applikation, besonders im befeuchteten Zustand zu verbessern. Eine bevorzugte Laminierung umfasst eine Laminierung mit einer selbstklebenden Schicht bwz. einem schichtförmigen Pflastermaterial, welches bevorzugt so auf den schichtförmigen Collagenmaterialien angebracht wird, dass die selbstklebende Laminatschicht randseitig ganz oder teilweise über das Collagenmaterial hinausragt, so dass die laminierten Collagenmaterialien ähnlich einer herkömmlichen Pflasteranordnung mittels der randseitig überstehenden selbstklebenden Laminierung auf den zu behandelnden Hautarealen leicht fixiert werden können. Bei solchen selbstklebenden laminierten Collagenmaterialien sind insbesondere solche selbstklebenden Laminierungsbeschichtungen bevorzugt, die über eine besonders hohe Hautverträglichkeit, geringe Irritations- und Allergieneigung sowie eine leichte Ablösbarkeit verfügen, um möglicherweise vorgeschädigte oder irritierte Hautareale beim Ablösen der Klebeschicht nicht zusätzlich zu verletzen. Je nach gewünschtem Anwendungsgebiet können solche Laminierungen okklusiv, semi-okklusiv oder hydrophil und nicht-okklusiv sein, wobei hydrophile, nicht-okklusive bzw. maximal (im Sinne von "allenfalls noch") semi-okklusive Laminierungen bevorzugt sind, um die Befeuchtung der laminierten Collagenmaterialien für die Anwendung zu ermöglichen. Desweiteren ist bei der Wahl solcher selbstklebender Laminierungsbeschichtungen darauf zu achten, dass die Klebeschicht nicht wasserlöslich ist, damit beim Befeuchten des Materials der Kleber nicht herausgelöst werden kann und damit die Klebe- bzw. Fixierungswirkung verloren geht.

Damit umfasst die vorliegende Erfindung insbesondere auch solche schichtförmigen vernetzten Collagenmaterialien, welche ganz oder teilweise mit einer weiteren Schicht ausgewählt aus Fasern, Vliesen, Netzen, Filmen oder Folien oder einer selbstklebenden Schicht versehen ist, die derart auf dem schichtförmigen Collagenmaterial aufgebracht ist, dass sie mit diesem randseitig abschließt oder randseitig vollständig oder partiell über das Collagenmaterial hinausragt.

Wie vorstehend ausgeführt, können die erfindungsgemäßen Collagenmatrices sowohl vor der kosmetischen als auch vor der medizinischen Anwendung vorbefeuchtet bzw. rehydratisiert werden. Eine solche Befeuchtung bzw. Rehydratisierung erfolgt vorzugsweise mit einer wässrigen Lösung die ausgewählt ist aus der Gruppe umfassend Wasser sowie ggf. entmineralisiertes Wasser oder sogenanntes Thermalwasser, physiologische Lösungen sowie wässrige Lösungen, die mindestens einen Wirk- und/oder Hilfsstoff enthalten. Die zur Befeuchtung bzw. Rehydratisierung verwendeten wässrigen Lösungen werden auch als Aktivatorlösungen bezeichnet.

Bei solchen Aktivatorlösungen kann es sich beispielsweise um Lösungen von leicht-flüchtigen Wirk- und/oder Hilfsstoffen handeln, die aufgrund des Herstellverfahrens z.B. durch die Gefriertrocknung nicht in ein gefriergetrocknetes Material eingebracht werden sollen bzw. können, wie z.B. gewisse Anteile ätherischer Öle, Parfums etc. Auch können solche Wirk- und/oder Hilfsstoffe enthalten sein, die eine zusätzliche befeuchtende Wirkung erzielen und die aufgrund dieser befeuchtenden Wirkung oder aufgrund von Hygroskopie-Neigungen nicht oder nur in geringen Mengen in die erfindungsgemäßen gefriergetrockneten Collagenmatrices eingearbeitet werden können, da dadurch entweder die Stabilität des gefriergetrockneten Collagenmaterials selbst oder die Stabilität eventuell enthaltener feuchtigkeitslabiler Wirkstoffe nicht mehr aufrechterhalten werden kann.

Grundsätzlich können in den Aktivatorlösungen einer oder mehrere der vorstehend genannten Wirk- und/oder Hilfsstoffe enthalten sein. Insbesondere sind Wirkstofflösungen besonders geeignet, die einen oder mehrere der vorstehend genannten bevorzugten Wirk- oder Hilfsstoffe zur therapeutischen Anwendung in den erfindungsgemäß bevorzugten Indikationen enthalten.

In einer besonders bevorzugten Ausführungsform wird eine wässrige Aktivatorlösung verwendet, die im Wesentlichen frei von Konservierungsstoffen und/oder Hilfsstoffen aus der Gruppe der Polyether, Polyethylenglykole (PEG's), Polypropylenglykole (PPG's) und Polyglykole (PG's) ist.

In einer weiteren bevorzugten Ausführungsform der Erfindung liegt das erfindungsgemäße Epoxy-vernetzte Collagenmaterial mit der Aktivatorlösung in einer zusammengehörenden, räumlichen Anordnung (Kombinationspräparat, Anwendungspaket, Set, Kit-of-Parts etc.) vor. Derartige Kombinationspräparate oder Kit-of-parts Anordnungen umfassen bevorzugt mindestens eine der erfindungsgemäßen Collagenmatrices, bevorzugt solche in Form schichtförmiger Auflagen, Pads oder Masken, sowie mindestens eine wässrige Lösung, die einen oder mehrere Wirkstoffe und/oder mindestens einen oder mehrere Hilfsstoffe enthalten kann (Aktivatorlösung).

Die Ausgestaltung solcher Kombinationspräparate oder Kit-of-parts Kombinationen aus erfindungsgemäßem Collagenmaterial einerseits und Aktivatorlösung andererseits kann dabei vorsehen, dass die beiden Bestandteile separat aus der Kit-of-parts Anordnung entnommen werden und außerhalb davon für die weitere Verwendung zusammengeführt werden. Es ist jedoch auch denkbar, dass eine Zusammenführung der Komponenten innerhalb der Kit-of-parts-Verpackung, z.B. in dafür vorgesehenen Kammern, selbst erfolgt und die rehydratisierte Zusammensetzung sodann aus dieser direkt der weiteren äußerlichen oder transdermalen Verwendung zugeführt wird. Dies kann bevorzugt direkt durch den Endverbraucher durchgeführt werden.

Die Erfindung wird durch die nachfolgenden Beispiele näher erläutert.

## BEISPIELE

### Beispiel 1:

### Herstellbeispiel 1a

**Reine Collagenmatrix mit Epoxid-Vernetzung**

**(5 % Epoxid-Vernetzungsmittel/Trockenmasse)**

Beispiel zur Herstellung einer erfindungsgemäßen Epoxy-vernetzten Collagen-Biomatrix ohne Zusatz weiterer löslicher Protein- oder Peptidbestandteile, Wirk- und/oder Hilfsstoffe unter Verwendung einer Epoxid-Vernetzungsmittelmenge von 5% bezogen auf die Trockenmasse der Collagensuspension.

a) Bereitstellen von 3000 g einer Collagensuspension (Trockengehalt Collagen: 1.6%, Collagengehalt: 48g), hergestellt nach dem Verfahren gemäß DE 4048622 A1 und insbesondere DE 10350654 A1
b) Einstellen des pH-Werts der Collagensuspension auf pH 3,3.
c) (entfällt)
d) Bei einer Temperatur unterhalb von 10°C wird die Collagensuspension unter Rühren mit einem Flügelrührer (eurostar, IKA) bei 600U/s innerhalb von 5 Minuten tropfenweise mit 2.4g 1,4-Butandioldiglycidylether (BDDGE, Sigma-Aldrich) versetzt. Die erhaltene Masse wird in einem Vakuum-Mischgerät (Smartmix, Amann/Girrbach) in Portionen von ca. 750g entgast.
e) Die entgaste Suspension wird innerhalb von 2h nach Einmischen des Vernetzungsmittels in Platten gegossen und eingefroren, wobei die Collagensuspension bis zum Einfrieren weiterhin bei einer Temperatur < 18 °C gehalten wird.
f) Anschließend wird die gefrorene Masse für 24 Stunden bei -20 °C gelagert und anschließend einer Gefriertrocknung unterzogen, wobei die Gefriertrocknungstemperatur unterhalb von 100 °C gehalten wird.
g) Die lyophilisierte Collagenmatrix wird bei einer Luftfeuchtigkeit von 60 - 70% rel. Luftfeuchtigkeit über 24 - 48 Std. auf einen Feuchtigkeitsgehalt bis 25 %, bezogen auf die getrocknete Collagenmatrix, rückbefeuchtet.
h) Die so erhaltene Collagenmatrix wird in Schichten von 1-2 mm gespalten, konfektioniert und gegebenenfalls gamma-sterilisiert (20kGy).

### Herstellbeispiel 1b

**Reine Collagenmatrix mit Epoxid-Vernetzung**

**(10 % Epoxid-Vernetzungsmittel/Trockenmasse)**

Beispiel zur Herstellung einer erfindungsgemäßen Epoxy-vernetzten Collagen-Biomatrix ohne Zusatz weiterer löslicher Protein- oder Peptidbestandteile, Wirk- und/oder Hilfsstoffe unter Verwendung einer Epoxid-Vernetzungsmittelmenge von 10 % bezogen auf die Trockenmasse der Collagensuspension.

Die Herstellung erfolgt analog zu Herstellbeispiel 1a, worin in Schritt d) 4.8 g 1,4-Butandioldiglycidylether (BDDGE, Sigma-Aldrich) zugesetzt wird und in Schritt g) die Rückbefeuchtung bis zu 96 Std. lang erfolgt.

**Beispiel 2:**

**Herstellbeispiel 2a**

**Epoxid-vernetzte Collagenmatrix mit zusätzlichen löslichen Proteinbestandteilen aus der Gruppe der Matrixproteine (Elastin-Hydrolysat)**

**(5 % Epoxid-Vernetzungsmittel/Trockenmasse)**

Beispiel zur Herstellung einer erfindungsgemäßen Epoxy-vernetzten Collagen-Biomatrix mit Zusatz weiterer löslicher Protein-/ Peptidbestandteile (Matrixproteine; Elastin) unter Verwendung einer Epoxid-Vernetzungsmittelmenge von 5% bezogen auf die Trockenmasse der wässrigen Collagensuspension.

a) Bereitstellen von 3000 g einer Collagensuspension (Trockengehalt Collagen: 1.6%, Collagengehalt: 48g), hergestellt nach dem Verfahren gemäß DE 4048622 A1 und insbesondere DE 10350654 A1
b) Einstellen des pH-Werts der Collagensuspension auf pH 3,3.
c) Bei einer Temperatur unterhalb von 10°C wird die Collagensuspension unter Rühren mit einem Flügelrührer (eurostar, IKA) bei 550 U/s innerhalb von 5 Minuten tropfenweise mit 2.4g 1,4-Butandioldiglycidylether (BDDGE, Sigma-Aldrich) versetzt.
d) Anschließend Zugabe von 30g Elastin-Hydrolysat (Elastin spezial BIN, Fa. GfN, Herstellung von Naturextrakten GmbH) unter gleichen Bedingungen. Die erhaltene Masse (wässrige Collagenmischung) wird in einem Vakuum-Mischgerät (Smartmix, Amann/Girrbach) in Portionen von ca. 750g über 2x 5 min entgast.
e) Die entgaste Collagenmischung wird innerhalb von 2h nach Einmischen des Vernetzungsmittels und des Elastin-Hydrolysats in Platten gegossen und eingefroren, wobei die Collagenmischung bis zum Einfrieren weiterhin bei einer Temperatur < 10 °C gehalten wird.
f) Anschließend wird die gefrorene Masse für 24 Stunden bei -20 °C gelagert und anschließend einer Gefriertrocknung unterzogen, wobei die Gefriertrocknungstemperatur unterhalb von 100 °C gehalten wird.
g) Die lyophilisierte Collagenmatrix wird bei einer Luftfeuchtigkeit von 60 - 70% rel. Luftfeuchtigkeit über 24 - 48 Std. auf einen Feuchtigkeitsgehalt bis 25 %, bezogen auf die getrocknete Collagenmatrix, rückbefeuchtet.
h) Die so erhaltene Collagenmatrix wird in Schichten von 1-2 mm gespalten, konfektioniert und gegebenenfalls gamma-sterilisiert (20kGy).

**Herstellbeispiel 2b**

**Epoxid-vernetzte Collagenmatrix mit zusätzlichen löslichen Proteinbestandteilen aus der Gruppe der Matrixproteine (Elastin-Hydrolysat)**

**(10 % Epoxid-Vernetzungsmittel/Trockenmasse)**

Beispiel zur Herstellung einer erfindungsgemäßen Epoxy-vernetzten Collagen-Biomatrix mit Zusatz weiterer löslicher Protein-/ Peptidbestandteile (Matrixproteine; Elastin) unter Verwendung einer Epoxid-Vernetzungsmittelmenge von 10% bezogen auf die Trockenmasse der wässrigen Collagensuspension.

Die Herstellung erfolgt analog zu Herstellbeispiel 2a, worin in Schritt c) 4.8g 1,4-Butandioldiglycidylether (BDDGE, Sigma-Aldrich) zugesetzt wird und in Schritt g) die Rückbefeuchtung bis zu 96 Std. lang erfolgt.

**Beispiel 3:**

**Herstellbeispiel 3**

**Epoxid-vernetzte Collagenmatrix mit zusätzlichen Wirkstoffen/Hilfsstoffen aus der Gruppe der kosmetischen Fette und Öle (Triglyceride / Neutralöl)**

**(4,8 % Epoxid-Vernetzungsmittel/Trockenmasse)**

Beispiel zur Herstellung einer erfindungsgemäßen Epoxy-vernetzten Collagen-Biomatrix mit Zusatz weiterer Wirk-/Hilfsstoffe aus der Gruppe der Fette und Öle (Triglyceride / Neutralöl) unter Verwendung einer Epoxid-Vernetzungsmittelmenge von 4,8 % bezogen auf die Trockenmasse der wässrigen Collagensuspension.

a) Bereitstellen von 3900 g einer Collagensuspension (Trockengehalt Collagen: 1.6%, Collagengehalt: 62,4g), hergestellt nach dem Verfahren gemäß DE 4048622 A1 und insbesondere DE 10350654 A1

b) Einstellen des pH-Werts der Collagensuspension auf pH 3,3.

c) Bei einer Temperatur unterhalb von 10°C wird die Collagensuspension unter Rühren mit einem Flügelrührer (eurostar, IKA) bei 550 U/s innerhalb von 5 Minuten tropfenweise mit 2.95g 1,4-Butandioldiglycidylether (BDDGE, Sigma-Aldrich) versetzt.

d) Anschließend Zugabe von 3,8g Neutralöl unter gleichen Bedingungen. Die erhaltene Masse (wässrige Collagenmischung) wird in einem Vakuum-Mischgerät (Smartmix, Amann/Girrbach) in Portionen von ca. 750g über 2x 5 min entgast.

e) Die entgaste Collagenmischung wird innerhalb von 2h nach Einmischen des Vernetzungsmittels und des Neutralöls in Platten gegossen und eingefroren, wobei die Collagenmischung bis zum Einfrieren weiterhin bei einer Temperatur < 10 °C gehalten wird.

f) Anschließend wird die gefrorene Masse für 24 Stunden bei -20 °C gelagert und anschließend einer Gefriertrocknung unterzogen, wobei die Gefriertrocknungstemperatur unterhalb von 100 °C gehalten wird.

g) Die lyophilisierte Collagenmatrix wird bei einer Luftfeuchtigkeit von 60 - 70% rel. Luftfeuchtigkeit über 24 - 48 Std. auf einen Feuchtigkeitsgehalt bis 25 %, bezogen auf die getrocknete Collagenmatrix, rückbefeuchtet.

h) Die so erhaltene Collagenmatrix wird in Schichten von 1-2 mm gespalten, konfektioniert und gegebenenfalls gamma-sterilisiert (20kGy).

**<u>Beispiel 4:</u>**

**Qualitative Bestimmung**

**von löslichen Proteinen mittels SDS-PAGE**

Kurzerklärung: SDS-PAGE

SDS = Sodium-Dodecyl-Sulfat, Detergenz, Natriumsalz einer langkettigen Fettsäure
PAGE = Poly-Acrylamid-Gel-Elektrophorese, Elektrophorese in einem Polymergel aus Acrylamid

Elektrophorese: Auftrennung von geladenen Teilchen in einer Trägersubstanz durch Anlegen einer elektrischen Spannung, die eine Wanderung der Teilchen zur Folge hat

Kurzbeschreibung: Durch Anlagerung von ionischen Detergenzien bei pH>7 werden aus ungeladenen Proteinen geladene Teilchen, die sich in einem Gel durch Anlegen einer elektrischen Spannung in dem daraus resultierenden elektrischen Feld anhand ihrer Größe und Gestalt entlang einer Wanderungsstrecke auftrennen. Proteine geringer Masse wandern schneller als große Proteine mit höherer Masse. Kugelförmige Proteine wandern schneller als langgestreckte, fädige Proteine. Die nach Größe getrennten Proteingruppen bilden im Gel schmale Streifen (genannt Banden), die mit Farbstoffen sichtbar gemacht werden können. Die Größe von Proteinen oder Proteinfragmenten wird anhand ihrer Wanderungsstrecke im Vergleich zu Referenzsubstanzen (Molekulargewichtsstandard = Proteine bekannter Größe) visuell beurteilt.

Geeignete Testsysteme sind herkömmliche käuflich erhältliche Standard-Testsysteme wie beispielsweise das Criterion-System der Firma Biorad:

Criterion XT Precast Gel 4-12% Acrylamid,
Puffersystem: Bis-Tris, 12 + 2 Well Comb, 45$\mu$l pro Well
Geldicke: 1mm, Catalog 345-0123 Fa.Bio Rad
Puffer B: Criterion XT Mes (Buffer, 20x) Control 210007145
Probenpuffer: XT Sample Buffer; 4x, 10ml Control 310008088

Die Färbung der fertigen Proteingele wird durch das Fertigreagenz GelCode Blue Safe Stain (Thermo Scientific) erreicht, welches auf dem protein-sensitiven Farbstoff Coomassie-Blau basiert.

**Beispiel 5:**

**Quantitative Bestimmung**

**löslicher Protein-Bestandteile mittels BCA-Methode**

Der Nachweis von löslichen Proteinen und Proteinbestandteilen mittels BCA-Test beruht darauf, dass Proteine mit $Cu^{2+}$-Ionen in alkalischer Lösung einen Komplex bilden (Biuret-Recktion). Die $Cu^{2+}$ Ionen dieses Komplexes werden zu $Cu^{+}$-Ionen reduziert, die mit Bicinchinon-Säure (BCA) einen violetten Farbkomplex bilden. Die Absorption dieses Farbkomplexes wird spektrometrisch bei 562 nm gemessen. Die Reduktion erfolgt durch die Seitenketten von Cystein, Tyrosin, Tryptophan und die Peptidbindung, wobei die Intensität der Farbstoffbildung (das Redoxverhalten der beteiligten Gruppen) u.a. von der Temperatur abhängt, so dass durch deren Variation die Sensitivität des Tests modifiziert werden kann.

Geeignete Testsysteme sind herkömmliche käuflich erhältliche Standard-Testsysteme wie beispielsweise der Pierce BCA Protein Assay Kit (Thermo Scientific).

Die Bestimmung erfolgt im Vergleich zu einer Standardlösung von BSA (Bovine Serum Albumin).
Reaktionsbedingungen zur Farbentwicklung: 60 °C, 1 h Reaktionszeit.

**Versuchsergebnisse:**

Abbildung 1 zeigt den prozentualen Anteil löslicher Protein-Bestandteile in einer Epoxy-vernetzten Collagenmatrix mit zusätzlichen löslichen Matrixproteinen (Elastin-Hydrolysat), entsprechend der Zusammensetzung nach Herstellbeispiel 2a und 2b, die mit Epoxid-Konzentrationen von 5% und 10%, jeweils bezogen auf die Trockenmasse der Collagensuspension, vernetzt wurden im Vergleich zu einer ausschließlich dehydrothermal vernetzten Collagenmatrix mit Elastin-Hydrolysat (100%). (In der Abbildung bezeichnet dabei "unvernetzt" eine dehydrothermal vernetzte (d.h. nicht chemisch vernetzte) Collagenmatrix.). Daraus zeigt sich deutlich, dass trotz chemischer Vernetzung ein hoher Anteil löslicher Proteinbestandteile und Matrixproteine in dem Material erhalten bleibt. Die Messung zeigte außerdem für die untersuchten Materialien die folgenden Gewichtsanteile an löslichen Proteinbestandteilen, jeweils bezogen auf das gefriergetrocknete Endprodukt:

Dehydrothermal vernetzte Collagenmatrix mit Elastin ("unvernetzt"): > 15 Gew.-%

Herstellbeispiel 2a mit 5 % Epoxid: > 10 Gew.-%
Herstellbeispiel 2b mit 10 % Epoxid: > 4 Gew.-%.

**Beispiel 6:**

**Bestimmung der Degradationsrate Collagenaseverdautest**

Die Bestimmung der enzymatischen Stabilität (Degradationsstabilität) von Collagenmatrices erfolgt mittels einer Methode, die auf dem Abbau der Collagenfasern durch das Enzym Collagenase beruht. Dabei wird der Abbau der Collagenfasern und -fibrillen durch das Enzym Collagenase (Collagenase aus Clostridium histolyticum (Typ 1) in PBS-Puffer (Phosphate Buffered Saline) unter kontrollierten Bedingungen gezielt herbeigeführt und nach einer definierten Reaktionsdauer mittels UV/VIS Messung in einem Spektralphotometer der Anteil der Zersetzungsprodukte bestimmt.

Verwendete Chemikalien:

- TRIS- Pufferlösung: TRIZMA- Base (Fluka Art Nr. 93350 LOT 450756/1), $CaCl_2*6H_2O$ (Riedel de Haen Art Nr. 12074 LOT 12610),
- HCl 25%ig (Merck Art Nr. 1.00316.1000 LOT Z730816335)
- EDTA- Lösung: Ethylendiamin-tetraessigsäure Dinatriumsalz Dihydrat 0,2mol/L (Fluka Art Nr. 03679 LOT 1104109 10505293)
- Collagenase: Sigma C 688T LOT 122k8607, 700 U/mg
- Collagenmatrices: z.B. gemäß Herstellbeispiel 1 bis 3

Herstellung der Reagenzien:

TRIS- Pufferlösung 0,1M/ 25mM $CaCl_2$:

1,21g TRIZMA Base wird zusammen mit 0,55g $CaCl_2$ *6$H_2O$ in 80mL RO-Wasser gelöst und mit ca. 1mL 25% HCl auf einen pH- Wert von 7,4 eingestellt, anschließend wird die Lösung in einen 100mL Messkolben überführt und mit RO-Wasser aufgefüllt.

EDTA- Lösung 0,2 mol/L:

Es wird 3,7224g Ethylendiamin-tetraessigsäure Dinatriumsalz Dihydrat in 50mL RO- Wasser gelöst (auf dem Magnetrührer ca. 30min).

Enzymlösung:

5mg des Enzyms wird in 1mL TRIS- Pufferlösung gelöst. Für die einzelnen Versuche werden Aliquote von 50 bzw. 100U (chargenabhängiges Volumen, hier = 35 u. 70µL) dem Versuchssystem zugeführt.

Essigsäure 0,95%ig:

Es wird 0,95mL Eisessig mit RO- Wasser auf 1000mL aufgefüllt.

Durchführung:

Aus 2 mm dicken Collagensheets werden Stücke mit einem Radius von 10 mm gestanzt (getr. Gewicht ca. 15mg) und 12 Stunden im Exsikator getrocknet. Die Proben werden in 2mL Eppendorf Gefäße (Safe Lock Tubes 0030.120.094) eingewogen und mit 1,5mL der TRIS- Pufferlösung versetzt. Sobald die Zugabe von 35µL Enzymlösung erfolgt ist, werden die Proben bei 37°C im Thermoblock (Stuart Scientific Block Heater) temperiert.

Nach der entsprechenden Verdauungszeit (1,5 Stunden, 3 Stunden, 6 Stunden, und 23 Stunden) werden die Eppendorf Gefäße mit 200µL EDTA-Lösung versetzt und 40 min. mit max. Leistung bei 18°C zentrifugiert. Zur Verdünnung mit 7,5mL Essigsäure 0,95% werden Aliquote von 800µL in 10mL Normschliff Reagenzgläser überführt, mit einem Stopfen verschlossen und zur Homogenisierung umgeschwenkt.

Photometrische Messung:

Die Messung erfolgt im UV/VIS Spektralphotometer bei einer Wellenlänge von 234nm. Es werden 10mm Quarz Küvetten verwendet. Als Leerwert wird 7,5mL Essigsäure mit 800µL TRIS- Pufferlösung vermessen. Bevor die Proben vermessen werden sollte als Nullwert eine entsprechende Enzymlösung vermessen werden (hier: 35µL Enzymlsg. In 1,5mL TRIS- Puffer im Eppendorf Gefäß, davon 800µL in 7,5mL Essigsäure), dessen Absorption wird als Nullpunkt in der Grafik gesetzt (Absorption ca. 0,0166).

Für die Bestimmung der Absolutwerte wird außerdem nach obenstehender Vorschrift eine Kalibrierlösung erstellt und vermessen.

Herstellung der Kalibrierlösung:

0,1006g Kollagen wurde in 8ml TRIS-Puffer mit 233µl Collagenaselösung (entsprechend 600U) über Nacht bei 37°C vollständig abgebaut. Diese Lösung wurde am nächsten Tag mit 1333µl EDTA-Lösung versetzt und im Messkolben mit TRIS-Puffer auf 10ml aufgefüllt. Von dieser Lösung wurden entsprechende Volumina nach folgender Tabelle mit 0,95% Essigsäure auf 10 ml verdünnt und die Absorption dieser Lösung bei 234 nm gemessen.

| 1%ige Kollagen-Lösung [ml/10ml] | Masse Kollagen-abbaupro-dukt in g | Konzentration Kollagen [µg/ml] | A (234nm) (MW; n=2) |
|---|---|---|---|
| 0 | 0 | 0 | -0,0146 |
| 0,3 | 0,0003 | 300 | 0,2782 |
| 0,6 | 0,0006 | 600 | 0,6095 |
| 0,9 | 0,0009 | 900 | 0,9754 |

(fortgesetzt)

| 1%ige Kollagen-Lösung [ml/10ml] | Masse Kollagen-abbaupro-dukt in g | Konzentration Kollagen [μg/ml] | A (234nm) (MW; n=2) |
|---|---|---|---|
| 1,2 | 0,0012 | 1200 | 1,3254 |

Die entsprechende Kalibriergerade hat die lineare Gleichung
A (234nm) = 0,0011c-0,0407 ($R^2$=0,9983).

**Versuchsergebnisse:**

Abbildung 2a zeigt die relativen Degradationsraten (%) einer Epoxy-vernetzten Collagenmatrix ohne zusätzliche Inhaltsstoffe gemäß Herstellbeispiel 1a sowie einer Epoxy-vernetzten Collagenmatrix mit zusätzlichen löslichen Matrixproteinen (Elastin-Hydrolysat) gemäß Herstellbeispiel 2a (Epoxid-Konzentration jeweils 5%) im Vergleich zu einer ausschließlich dehydrothermal vernetzten Collagenmatrix mit Elastin-Hydrolysat. (In der Abbildung bezeichnet dabei "unvernetzt" eine dehydrothermal vernetzte (d.h. nicht chemisch vernetzte) Collagenmatrix.) Die Abbildung zeigt deutlich die Verbesserung der Degradation im Sinne einer Verringerung der Degradationsrate (Verringerung des enzymatischen Abbaus) der Epoxid-vernetzten erfindungsgemäßen Collagenmaterialien.

Hier wurde die nach 6 Stunden vollständig aufgelöste Matrix als 100% gesetzt. Die Absorptionswerte wurden auf 10 mg Matrix normiert.

Über die Bestimmung der Kalibriergerade kann man aus den Absorptionswerten die Absolutwerte der gelösten Mengen berechnen:

| Konzentration der gelösten Matrix in mg/ml bzw. Degradation in % | t=0 | t=1,5 h | t=3 h | t=6 h | t=23 h |
|---|---|---|---|---|---|
| unvernetzt | 0<br>0% | 9,37<br>93,7% | 10<br>100% | 10<br>100% | 10<br>100% |
| Bsp. 1a | 0<br>0% | 2,64<br>26,4% | 4,18<br>47,8% | 4,45<br>44,5% | 7,64<br>76,4% |
| Bsp. 2a | 0<br>0% | 5,88<br>58,8% | 6,84<br>68,4% | 7,09<br>70,9% | 8,73<br>87,3% |

Abbildung 2b zeigt die entsprechenden absoluten Konzentrationen der gelösten Matrix normiert auf 10 mg eingesetzte Matrix über einen Zeitraum bis zu 23 Stunden.

Die Abbildung 2b zeigt außerdem die Entwicklung der Degradation über einen Zeitraum bis 23 Stunden als prozentualer Anteil der Degradation. Danach zeigen die erfindungsgemäß vernetzten Collagenmatrices auch nach 23 Stunden Collagenaseverdau noch eine deutlich bessere Degradationsstabilität als eine chemisch unvernetzte Matrix.

**Beispiel 7:**

**Bestimmung der Hydrolysestabilität**

Die Untersuchung der Hydrolysestabilität von Collagenmatrices erfolgt durch Einlegen eines definierten Collagenmaterials in eine wässrige Lösung und Lagerung bei 50 °C und Bestimmung der Veränderung der Materialeigenschaften über die Zeit.

Eine beispielhafte wässrige Lösung zur Untersuchung der Hydrolysestabilität weist die folgende Zusammensetzung auf:

| | Hersteller | Anteil (%) |
|---|---|---|
| Glycerin | Merck | 2,50 |
| 1,5 Pentandiol | Merck | 2,40 |
| Rokonsal MEP | ISP | 0,250 |
| Natriumchlorid | AppliChem | 0,10 |

(fortgesetzt)

| | Hersteller | Anteil (%) |
|---|---|---|
| Reinstwasser | | 94,75 |

Herstellung:

1) Glycerin, Pentandiol und Rokonsal MEP in Becherglas auf Magnetrührer lösen
2) mit Reinstwasser (SHC 101) unter rühren auf ca. 98% der Endmenge auffüllen
3) NaCl zugeben und auf Magnetrührer lösen
4) pH-Wert mit 1 %iger Citronensäure-Lösung auf pH 5,0 einstellen
5) mit Reinstwasser (SHC 101) auf die Endmenge auffüllen
6) pH-Wert kontrollieren und evtl. nochmals auf pH 5,0 einstellen

Die Hydrolysestabilität kann einerseits durch Messung der Flächenreduzierung, bedingt durch die Schrumpfung der Matrices, ermittelt werden.

Außerdem kann die Hydrolysestabilität durch anhand der Massenabnahme bestimmt werden nach:

$$\text{Hydrolysestabilität (\%)} = M_{tx} \times 100 / M_{t0}$$

Mit $M_{tx}$ = Masse des getrockneten intakten/zusammenhängenden Matrixmaterials
$M_{t0}$ = Masse des trockenen Matrixmaterials vor Untersuchungsbeginn

**Versuchsergebnisse:**

Untersucht wurde die Hydrolysestabilität unter den vorstehend beschriebenen Bedingungen anhand der Bestimmung der Flächenreduzierung von

a) dehydrothermal vernetzte Collagenmatrix ("unvernetzt") enthaltend Triglyceride/Neutralöl, die bei einer Gefriertrocknungstemperatur > 120 °C gefriergetrocknet wurde.
b) erfindungsgemäße Epoxy-vernetzte Collagenmatrix gemäß Herstellbeispiel 3 (enthaltend Triglyceride/Neutralöl) sowie
c) Epoxy-vernetzte Collagenmatrix (5 % Epoxid/TM) entsprechend der Zusammensetzung nach Herstellbeispiel 3 (enthaltend Triglyceride/Neutralöl), die bei einer Gefriertrocknungstemperatur > 120 °C gefriergetrocknet wurde.

Abbildung 3 zeigt, dass lediglich dehydrothermal vernetzte Collagenmaterialien ("unvernetzt) gemäß a) unter den gewählten Versuchsbedingungen nach 5 Tagen bereits eine vollständige Hydrolyse (strukturelle Auflösung der Matrixstruktur) zeigen, wohingegen bei den erfindungsgemäßen Epoxy-vernetzten Collagenmaterialien gemäß b) unter gleichen Bedingungen nach 18 Tagen noch keine strukturellen Veränderungen der Matrix beobachtet werden. Epoxy-vernetzte Collagenmaterialien die bei Gefriertrocknungstemperaturen > 100 °C getrocknet wurden gemäß c) zeigen gegenüber den erfindungsgemäßen Materialien nach b) zum gleichen Zeitpunkt eine beginnende Hydrolyse (Flöchenschrumpfung). Nach ca. 4 Wochen zeigt auch eine erfindungsgemäße Matrix nach b) eine beginnende Hydrolyse, wobei die höher getrockneten Materialien gemäß c) zu diesem Zeitpunkt eine deutlich schlechtere Hydrolysestabilität zeigen. Die Ergebnisse spiegeln damit auch den Einfluss der Gefriertrocknungstemperatur auf die Hydrolysestabilität wider. Danach weisen Matrices, die einer Gefriertrocknungstemperatur > 100 °C ausgesetzt wurden, eine schlechtere Hydrolysestabilität auf gegenüber den erfindungsgemäß hergestellten Materialien (Gefriertrocknungstemperatur < 100 °C).

Exemplarisch veranschaulicht Abbildung 4 den Grad der Hydrolyse einer dehydrothermal vernetzten Matrix gemäß a) im Vergleich zu einer erfindungsgemäßen Matrix gemäß b) nach 18 Tagen Lagerung unter den vorstehend beschriebenen Versuchsbedingungen.

**Beispiel 8:**

**Bestimmung der Nassreißfestigkeit**

**interne Methode (UV8801)**

Bei der Methode zur Bestimmung der Reißfestigkeit mittels Stempel (interne Messmethode UV8801) wird an erfindungsgemäßen Collagenmatrices mit Hilfe eines mechanischen Prüfgerätes (Zwick Materialprüfgerät B Z 2.5 / TN 1S) ein Metallstempel mit kugelförmigem Kopf (25 mm im Durchmesser) auf eine schichtförmige Ausführungsform der Collagenmatrices gedrückt und Weg und Kraft, die der Stempel zurücklegt bzw. ausübt, aufgezeichnet.
Für die Bestimmung wird eine schichtförmige Collagenmatrix mit einer Dicke von 1,5 mm auf eine Größe von 8 x 8 cm zugeschnitten und in die Probenaufnahme des Gerätes eingebracht und darin vollständig befeuchtet. Anschließend wird die Messung gestartet und der kugelförmige Stempel drückt auf die Probe, bis es zu einem Reißen des Materials kommt.
Die Kraft, bei dem es zu einem Riß des Materials kommt, wird mittels elektronischer Datenaufzeichnung aufgezeichnet, berechnet und ausgegeben.

**Versuchsergebnisse:**

Exemplarisch zeigt Abbildung 5 die Verbesserung der Nassreißfestigkeit (gemäß interner Methode UV8801) von Epoxy-vernetzten Collagenmatrices mit einer Zusammensetzung gemäß Herstellbeispiel 1a im Vergleich zu lediglich dehydrothermal vernetzten Collagenmatrices (unvernetzt, Trocknung 100 °C, wobei "unvernetzt" ausschließlich dehydrothermal vernetzt (d.h. nicht chemisch vernetzt) bezeichnet.
Die Abbildung 5 zeigt auch den Einfluss der Gefriertrocknungstemperatur auf die Nassreißfestigkeit. Danach weisen Matrices, die einer Gefriertrocknungstemperatur ≥ 100 °C oder einer Nachtrocknung bei > 100 °C ausgesetzt wurden schlechtere Nassreißfestigkeiten auf als die erfindungsgemäß hergestellten Materialien.
Darüberhinaus veranschaulicht Abbildung 6 außerdem den Einfluss der Epoxid-Vernetztermittel Konzentration auf die Nassreißfestigkeit. Dabei zeigt sich, dass mit Zunahme der Epoxidkonzentration > 5% (in TM der wässrigen Collagensuspension/-mischung) die Nassreißfestigkeit abnimmt. Optimale Nassreißfestigkeiten werden unter den gegebenen Verfahrensbedingungen mit Epoxid-Konzentrationen von 5% bis 10 % (TM) erzielt. Die Abbildung zeigt außerdem die erwartungsgemäße Zunahme der Nassreißfestigkeit von lediglich dehydrothermal vernetzten Collagenmaterialien mit Erhöhung der Gefriertrocknungstemperatur.
Abbildung 7 veranschaulicht den Einfluss der Standzeiten und der dabei eingehaltenen Temperatur der wässrigen Collagensuspension/-mischung vor dem Einfrieren auf die Nassreißfestigkeit. Daraus wird deutlich, dass sich sowohl längere Standzeiten als auch höhere Temperaturen nachteilig auf die Nassreißfestigkeit der Collagenmaterialien auswirken.
Dabei zeigten alle untersuchten Epoxy-vernetzten Matrixmaterialien Nassreißfestigkeiten von > 400 cN/mm Schichtdicke.
Das dehydrothermal vernetzte Material wies eine Nassreißfestigkeit von < 200 cN/mm Schichtdicke auf.

**Beispiel 9:**

**Nachweis reaktiver Epoxy-Verbindungen Bestimmung der Epoxy-Restaktivität**

**(NBP-Assay)**

Die Bestimmung der Epoxy-Restaktivität kann mittels eines modifizierten NBP-Assays (Nitrobenzyl-Pyridin-Assays), basierend auf "Detection of Epoxides with 4-(p-Nitrobenzylpyridine" von Agarwal et al. (1979) Bull. Environm. Contam. Toxicol. 23, p. 825-829, modifiziert nach Zocher et al. (2000) "Epoxide hydrolase activity of Streptomyces strains" J. Biotechnol. Feb 17; 77(2-3), p. 287-92 erfolgen.

1. Prinzip

Das Prüfverfahren wird für die quantitative Bestimmung von löslichen, reaktiven Epoxyverbindungen im gefriergetrockneten Endprodukt bei mit Epoxiden stabilisierten Biomatrices angewendet.
p-Nitrobenzylpyridin reagiert mit alkylierenden Verbindungen (u.a. Epoxiden) zu einem farblosen, schwerlöslichen Salz, das bei Umsetzung mit einer Base zu einem blauen, schwerlöslichen Farbstoff deprotoniert wird. Die Farbintensität lässt sich spektrometrisch bei einer Wellenlänge von 570nm detektieren.

**2. Reagenzien / Chemikalien**

para-Nitrobenzylpyridin (NBP) (Merck, zur Synthese)
Ethylenglycol (Fluka, purum)
Aceton (Fluka, für UV-Spektroskopie)
Triethylamin (Fluka, purum)
1,4-Butonediol diglycidyl ether oder BDDG oder Epoxid (Sigma Aldrich, purum)
RO-Wasser
daraus hergestellt:

Substratlösung: 1g NBP in 5ml Ethylenglykol und 1.25ml Aceton bei 50°C lösen
(ausreichend für 10 Proben)
Basenlösung: 50% Triethylamin in Aceton (v/v)
(ausreichend für 10 Proben)

**3. Geräte**

Reagenzglas mit Schliff, Schliffstopfen, Schliffklemme oder 50ml
Laborflasche mit Schraubdeckel
Wärmeschrank und Heizblock (50°C)
Reaktionsgefäße 2 ml mit Sicherheitsverschluß oder Schraubverschluß
Mikropipetten für 200$\mu$l und 1000$\mu$l (Eppendorf), passende Pipettenspitzen UV-Spektrometer (570nm)
1,5ml Einmal-Plastikküvetten (Plastikbrand, Nr. 7591 50)

**4. Proben**

Stanzen (12 mm) des zu untersuchenden Collagenmaterials, pro Probe (Dreifachansatz) 1-3 Stanzen

Vergleichsprobe: gefriergetrocknete Biomatrix ohne chemische Vernetzer
(z.B. lediglich Dehydrothermal vernetzte Collagenmatrix)

**5. Durchführung**

Substratlösung:

Für jeweils 10 Proben wird 1g NBP in ein Reagenzglas mit Schliff eingewogen und mit 5ml Ethylenglycol und 1.25ml Aceton vermischt. Bei höherer Probenzahl die Menge entsprechend vervielfachen und eine Schraubdeckelflasche verwenden.
Nach sorgfältigem Verschließen (Schliffstopfen/Schliffklammer bzw. Schraubdeckel) wird das Substratgemisch in einem Wärmeschrank bei ca. 50°C unter gelegentlichem Schwenken erwärmt, bis sich der Feststoff vollständig aufgelöst hat (1/2 - 1h). Danach wird die Substratlösung auf Raumtemperatur abgekühlt. Das Salz bleibt dabei gelöst.

Baselösung:

3ml Triethylamin und 3ml Aceton im Reagenzglas mit Schliff verschließen und mischen.

Präparation der Standardproben für Eichgerade:

Vorverdünnung:

1g Epoxid wird auf 0.001g genau in einen 100ml Meßkolben mittels einer Pasteurpipette (Glas) direkt eingewogen, mit RO-Wasser auf 100ml aufgefüllt und gründlich gemischt (1:100)
Von der Vorverdünnung werden mit der 1000$\mu$l-Pipette genau 1000$\mu$l in einen 100ml Messkolben überführt, auf 100ml aufgefüllt und gründlich gemischt. Dies ist die Epoxid-Verdünnung [1:10.000] zur Generierung der Standardmeßwerte (Eichgerade)

Standard messwerte:

gleiche Menge 12mm- Stanzen des Vergleichsmaterials (ohne Vernetzer) in 2ml-Reaktionsgefäß geben, Reaktionsgefäße beschriften. Unter Nutzung der 200$\mu$l-Pipette Zugabe jeweils unterschiedlicher Volumina von RO-Wasser auf das Vlies (Volumen siehe Zeile A, Tabelle 1), nachfolgend Zugabe von 600$\mu$l Substratlösung.

Tabelle 1 Standardmeßwerte

| | Menge Epoxid (nmol) | 0 | 12,5 | 25 | 50 | 75 |
|---|---|---|---|---|---|---|
| A | Volumen RO-Wasser ($\mu l$) | 200 | 175 | 150 | 100 | 50 |
| B | Volumen Epoxid-Verdünnung ($\mu$l) | 0 | 25 | 50 | 100 | 150 |

Als letztes - erst nach Fertigstellung der Untersuchungsproben - Zugabe der Epoxid-Verdünnung 1:10.000 (Volumen siehe Zeile B, Tabelle 1) zu der Standardreihe im Reaktionsgefäß. Wiederum kurz schwenken.

Untersuchungsgut:

1-3 12mm-Stanzen des zu untersuchenden Materials werden im Dreifach-Ansatz in 2ml Reaktionsgefäßen auf 0.1 mg genau eingewogen und mit 200$\mu$l RO-Wasser befeuchtet. Danach erfolgt die Zugabe von 600$\mu$l Substratlösung zu den angefeuchteten Stanzen.

Mehrmaliges sanftes Anschnippen der Reaktionsgefäße bis sich die zunächst trübe Suspension aufklart. Wenn sich Reaktionslösung unter dem Deckel befindet, durch kurzes Zentrifugieren Flüssigkeit zurück in den Boden befördern.

Alle Proben sorgfältig verschlossen für 1h bei 50°C im Wärmeschrank oder im Heizblock inkubieren.

Abkühlen auf Raumtemperatur, anschließend Zugabe von 600$\mu$l der Basenlösung und kurzes Schütteln.

Dabei muss die Messung der Absorption innerhalb von 10 Minuten erfolgen.

Abnehmen des gesamten Überstandes der Collagenstanze mit der 1000$\mu$l-Pipette und Überführen in eine 1,5ml Plastikküvette.

Messung der Absorption bei 570nm innerhalb von 10min nach Zugabe der Basenlösung.

Messprogramm: Methods/Concentration/ENBP
Kolibrierpunkt: Standardmeßwert 50 (= 25nmol Epoxid)
Parallelstrahl und Blank: leere Plastikküvette

**6. Auswertung**

Mittels der Eichgeraden (Relation Konzentration/Absorption) wird die Menge des reaktiven Epoxides bestimmt. Das Ergebnis der Messung wird in Relation zur Einwaage des untersuchten Matrixmaterials gesetzt und ergibt sich zu:

Gehalt an Epoxid: ng/mg Collagen

**Versuchsergebnisse:**

Exemplarisch zeigt Abbildung 8 die Epoxid-Restaktivität der erfindungsgemäß hergestellten Collagenmaterialien anhand einer Matrix gemäß Herstellbeispiel 1a sowie deren exponentielle Abnahme im Verlauf der Zeit nach der Gefriertrocknung (Abbaukurve), Die darin untersuchten Matrices wurden bei einer Gefriertrocknungstemperatur von max. 60°C getrocknet. Aus der Abbildung wird außerdem insbesondere der Einfluss der Rückbefeuchtung auf die Abnahme der Epoxid-Restaktivität deutlich Für die Untersuchung wurden die Collagenmaterialien unmittelbar nach der Gefriertrocknung unter kontrollierten Klimabedingungen der Rückbefeuchtung zugeführt. Verglichen wurde die Abnahme der Restaktivität von Materialien mit einer Rückbefeuchtungsdauer von

a) 24 h,
b) 48 h,
c) 72 h und
d) 96 h.

Dabei wurde die Bestimmung jeweils unmittelbar nach Beendigung der Rückbefeuchtung durchgeführt. Lediglich Probe a) wurde vor Durchführung der ersten Messung für 24 Stunden bei normalen Umgebungsbedingungen gelagert. Dadurch ergeben sich die dargestellten Messpunkte nach 48 h (2 Tagen) für Proben a) und b), 72 h (3 Tagen) für Proben a), b) und c), nach 96 h (4 Tagen) für Proben a), b), c) und d), sowie nach 168 h (7 Tagen) und nach 336 h (14 Tagen) jeweils für alle Proben a) bis d).

Dabei zeigt sich deutlich, dass die Epoxid-Restaktivität mit zunehmender Rückbefeuchtung deutlich schneller abgebaut und auf ein nicht mehr nachweisbares Maß verringert werden kann.

**Beispiel 10:**

**In vitro Cytotoxizitätstest**

**(XTT-Test)**

**an Epoxy-vernetztem Collagenmaterial gemäß Herstellbeispiel 3**

**1. MATERIAL UND METHODE**

**1.1 Testmaterial:**

Gefriergetrocknetes Epoxy-vernetztes Collagenmaterial gemäß Herstellbeispiel 3

**1.2. HERSTELLUNG DER EXTRAKTE**

Aus dem Testmaterial wurden aus 3 unterschiedlichen Collagensheets jeweils Stücke identischer Größe (9,6 $cm^2$) ausgeschnitten. Die ausgeschnittenen Stücke wurden für 24 Stunden in 9,6 ml Zellkulturmedium (RPMI 1640 Medium), ergänzt mit 10% FCS (Gibco, Invitrogen, Ref, Nr. 10270-106), 1 mM Natriumpyruvat, 4mN L-Glutamin und 100 $\mu$g /ml Penicillin/Streptomycin (vollständiges Medium) bei 37$\pm$ 1,5 °C unter Schütteln extrahiert. Postiv- und Negativkontrollen wurden auf die gleiche Weise extrahiert.

Die Testmaterialien wurden gemäß ISO 10993-12 bei einem Oberfläche/Volumenverhältnis von 3 $cm^2$/ml extrahiert.

**1.3. KONTROLLEN**

Größere Mengen der Extrakte der Negativ- und Positivkontrollen wurden im Vorfeld hergestellt und bei -20 °C gelagert, 100% Extrakte wurden unmittelbar vor der Behandlung aufgetaut und verdünnt. Die Negativ- und Positivkontrollen wurden im vollständigen Medium gemäß ISO 10993-12 mit einem Oberfläche/Volumenverhältnis von 6 $cm^2$/ml extrahiert.

**1.3.1 NEGATIVKONTROLLE**

RM-C (hoch-dichtes Polyethylen)
Hersteller: Hatano Research Institut, Hatano/Japan
Lot: C - 042

**1.3.2 POSTIVKONTROLLE**

Name. Latex
Lieferant: VWR International GmbH (64295 Darmstadt, Deutschland)
Lot: 03200694110385

### 1.4 TESTSYSTEM

#### 1.4.1 KRITERIEN FÜR DIE AUSWAHL DER ZELLLINIE L929

Die ATCC, CCL 1 NCTC Klon 929 Zelllinie (Klon von Stamm L, Mausbindegewebe) wurde erfolgreich über mehrere Jahre in in-vitro-Experimenten eingesetzt. Insbesondere die hohe Proliferationsrate (Verdopplungszeit: 16 Stunden, gemessen am 22. Oktober 1992) und die gute Viabilität unbehandelter Zellen (in der Regel mehr als 70%), beides notwendig für eine geeignete Studiendurchführung, sprachen für die Auswahl dieser Zelllinie.

#### 1.4.2 ZELLKULTUREN

Große Mengen der L929-Zelllinie (geliefert durch LMP, Technische Universität Darmstadt, D-64287 Darmstadt) wurden in flüssigem Stickstoff in der Zellbank Harlan Cytotest Cell Research GmbH gelagert, was die wiederholte Verwendung der gleichen Zellkulturcharge in den Experimenten ermöglicht. Dadurch wird aufgrund der reproduzierbaren Charakteristika der Zellen die Einhaltung gleichbleibender Parameter in den Experimenten gewährleistet.

Aufgetaute Stammzellkulturen wurden bei 37 ± 1,5 °C in Plastikflaschen (Greiner, D-72634 Frickenhausen) vermehrt, Besiedlung wurde mit ca. $2x10^5$ Zellen/Kulturflasche in 6ml vollständigem Medium durchgeführt. Die Zellen wurden zweimal wöchentlich subkultiviert. Die Zellkulturen wurden bei 37 ± 1,5 °C und 5,0 ± 0,5% Kohlendioxidatmosphäre inkubiert.

### 1.5 TESTGRUPPEN

| | |
|---|---|
| **1.5.1 Mediumkontrolle:** | **vollständiges Medium** |
| **1.5.2. Negativkontrolle:** | **RM-C**<br>extrahiert mit vollständigem Medium für 24 Stunden; 100% Extrakt |
| **1.5.3. Positivkontrolle:** | **Latex**<br>extrahiert mit vollständigem Medium für 24 Stunden; 3% Extrakt, 10 % Extrakt, 30% Extrakt, 70% Extrakt, 100% Extrakt |
| **1.5.4. Testmaterial:** | **Collagenmatrix gemäß Herstellbeispiel 3**<br>extrahiert mit vollständigem Medium für 24 Stunden; 3% Extrakt, 10% Extrakt, 30% Extrakt, 100% Extrakt |

### 1.6 VERSUCHSDURCHFÜHRUNG

#### 1.6.1. ZELLKULTIVIERUNG

Exponentiell wachsende Stammkulturen, mehr als 50% konfluent, wurden mit Ca-Mg-freier Salzlösung gespült und mit Trypsin bei 37 ± 1,5 °C für 5 min (Gibco BRL Trypsin/EDTA-Lösung 10x Kat, Nr. 35400-019) behandelt. Dann wurde die enzymatische Ablösung durch die Zugabe des vollständigen Mediums gestoppt und eine einzige Zellsuspension wurde hergestellt.

Die Ca-Mg-freie Salzlösung zeigte die folgende Zusammensetzung (pro l):

| | |
|---|---|
| NaCl | 8000mg |
| KCl | 400mg |
| Glucose | 1000mg |
| $NaHCO_3$ | 350mg |

Einzelne Vertiefungen einer 96-Loch Zellkulturmicrotiterplatte (Greiner) wurden mit 0,1 ml Medium enthaltend ca. 15.000 Zellen versetzt. Das Medium war vollständiges Medium.

Die Platten wurden für 24 Stunden inkubiert, um Zellanhaftung zu ermöglichen.

#### 1.6.2 BEHANDLUNG

Danach wurde das Medium entfernt und die Zellen mit 0,1 molarem Behandlungsmedium versetzt, enthaltend verschiedene Konzentrationen der Extrakte der Testmaterialien, der Negativ- und der Positivkontrollextrakte sowie der

Mediumkontrolle.

Alle Inkubationen wurden bei 37 ± 1,5 °C in einer Feuchtatmosphäre mit 5,0 ± 0,5% $CO_2$-Atmosphäre durchgeführt.

### 1.6.3 XTT MARKIERUNG UND MESSUNG

Nach der Inkubationszeit von 24 Stunden wurden 50 $\mu$l der XTT-Markierungsmischung hinzugefügt. Die Mischung enthält das XTT-Markierungsreagenz und ein Elektronenkopplungsreagenz (Volumenverhältnis 1:100). Die Zellen wurden für ca. 1 Stunde 35 Minuten inkubiert und anschließend in den Mikroplattenreader transferriert (Versamax® Molecular Devices, D-85737 Ismaning) ausgestattet mit einem 450nm Filter zur Messung der Absorption (Referenzwellenlänge 690 nm).

### 1.7 DATENAUFZEICHNUNG

Die generierten Daten wurden als Rohdaten aufgezeichnet. Die Ergebnisse werden in tabellarischer Form enthaltend die Testgruppen mit den Testmaterialien, Negativmedium und Kontrollgruppe dargestellt.

### 1.8 AUSWERTUNG DER ERGEBNISSE

Eine Abnahme der Anzahl lebender Zellen resultiert in einer Abnahme der Gesamtaktivität der mitochondrialen Dehydrogenase in den Proben. Diese Abnahme korreliert direkt mit der Menge von gebildetem orangenem Formazan, gemessen über die Absorption. Die Ergebnisse für die dosisabhängige Cytotoxizitätsantwort kann als arithmetisches Mittel ± Standardabweichung dargestellt werden. Um die Konzentration des toxischen Materials zu berechnen, das notwendig ist, um die Absorption im Vergleich zur Mediumkontrolle um 50% zu reduzieren ($XTT_{50}$), wurde die folgende Formel verwedet:

$$XTT_{50} = Conc._{>50} - \frac{(Conc._{>50} - Conc_{<50}) \cdot (\%_{>50} - 50)}{(\%_{>50} - \%_{<50})}$$

| | |
|---|---|
| a) Conc. > 50= | max. gemessene Konzentration mit % der Mediumkontolle >50% |
| b) Conc. <50= | min. gemessene Konzentration mit % der Mediumkontrolle <50% |
| c) %>50= | relative Absorption bei a) in % |
| d) %<50= | relative Absorption bei b) in % |

Je geringer der $XTT_{50}$-Wert, umso höher ist das cytotoxische Potenzial des Testmoterials,

Eine Reduktion der Viabilität auf <70% und ≥50% der Mediumkontrolle bedeutet eine geringe Cytotoxizität. Bei einer Reduktion der Viabilität auf <50% der Mediumkontrolle liegt cytotoxisches Potenzial vor.

## 2. ERGEBNISSE

### 2.1. TABELLE DER ERGEBNISSE

Ergebnisse nach 24 Stunden Extraktion im vollständigen Medium

| Test Gruppe | Extrakt Konzentration [%] | Absorption* | Standard-Abweichung | Chem. Leerwerte | Absorption in % der Medium Kontrolle** |
|---|---|---|---|---|---|
| **Medium Kontrolle** | | 1.388 | 0.155 | 0.101 | 100.00 |
| **Testmaterial** | 3 | 1.246 | 0.141 | 0.101 | 88.97 |
| **Testmaterial** | 10 | 1.283 | 0.057 | 0.102 | 91.76 |
| **Testmaterial** | 30 | 1.258 | 0.046 | 0.103 | 89.74 |

(fortgesetzt)

| Test Gruppe | Extrakt Konzentration [%] | Absorption* | Standard-Abweichung | Chem. Leerwerte | Absorption in % der Medium Kontrolle** |
|---|---|---|---|---|---|
| **Testmaterial** | 100 | 1.320 | 0.028 | 0.100 | 94.79 |
| **Negativ Kontrolle** | 100 | 1.192 | 0.107 | 0.105 | 98.37 |
| **Medium Kontrolle** | | 1.206 | 0.079 | 0.101 | 100.00 |
| **Positiv Kontrolle** | 3 | 1.273 | 0.144 | 0.105 | 105.70 |
| **Positiv Kontrolle** | 10 | 0.903 | 0.040 | 0.106 | 72.13 |
| **Positiv Kontrolle** | 30 | 0.130 | 0.002 | 0.109 | 1.90 |
| **Positiv Kontrolle** | 70 | 0.129 | 0.002 | 0.111 | 1.72 |
| **Positiv Kontrolle** | 100 | 0.134 | 0.001 | 0.114 | 1.81 |

Angegebene Absorptionen zeigen gerundete Werte. Relative Absorptionen wurden unter Heranziehen der korrekten Absorptionswerte berechnet.

Schattierte Testgruppen repräsentieren Testmaterialkonzentration bei denen mikroskopische Untersuchungen nach der Behandlung morphologische Veränderungen der Zellen aufgrund aufgetretener Cytotoxizität zeigen.

* mittlere Absorption (absolut) von 7 wells

** relative Absorption [gerundete Werte]:

$$\frac{100 \times (absorbance_{specimen} - absorbance_{chem.blanks})}{(absorbance_{solvent\ control} - absorbance_{chem.blanks})}$$

$XTT_{50}$-Werte der Testmaterialien: konnten nicht berechnet werden, da die Viabilität der Zellen nicht relevant reduziert wurde.

$XTT_{50}$-Werte der Positivkontrolle: 16,3% (v/v)

**3. DISKUSSION**

Diese in vitro-Untersuchung wurde durchgeführt, um das cytotoxische Potenzial von gefriergetrocknetem Epoxy-vernetztem Collagenmaterial gemäß Herstellbeispiel 3 mittel XTT-Test unter Verwendung der Mauszelllinie L929 zu untersuchen.

Von drei Testmaterialien wurden jeweils identische Stücke (9,6cm$^2$) geschnitten, die geschnittenen Stücke wurden wie vorstehend beschrieben extrahiert.

Die folgenden Konzentrationen von Testextrakten wurden untersucht:

3%, 10%, 30% und 100% (v/v)

die folgenden Konzentrationen von Extrakten (Positivkontrolle) wurden getestet:

3%, 10%, 30%, 70% und 100 % (v/v)

Zwischen der Mediumkontrolle (vollständiges Medium) und der Negativkontrolle (extrahierte Negativkontrolle RM-C) konnten keine relevanten Unterschiede beobachtet werden.

Die Positivkontrolle (Latex) zeigte eine deutliche dosisabhängige Reduktion der Zellviabilität und Zellproliferation. Mit dem unverdünnten Referenz-Standardextrakt (100%) zeigte sich eine Reduktion der Zellviabilität und/oder Zellproliferation in den jeweiligen wells von um 1,81%. Der errechnete $XTT_{50}$-Wert ist 16,3% (v/v).

Bei keinem der untersuchten Extrakte des Collagenmaterials gemäß Herstellbeispiel 3 konnten cytotoxische Effekte nach der Inkubation beobachtet werden, Der $XTT_{50}$-Wert konnte nicht errechnet werden, da die Viabilität der Zellen nicht relevant reduziert wurde.

**4. ERGEBNIS**

Zusammenfassend kann festgestellt werden, dass im Rahmen dieser Untersuchung unter den dargestellten experimentellen Bedingungen Extrakte des Testmoterials gemäß Herstellbeispiel 3 keinerlei cytotoxisches Potenzial bis zur höchsten Testkonzentration gezeigt werden konnte.

**<u>Beispiel 11:</u>**

**Messung der Steifigkeit / Elastizitäts-Modul E**

**(nach Stok et al.; J Biomed Mater Res; 2009)**

**an Epoxy-vernetztem Collagenmaterial gemäß Herstellbeispiel 2a und 2b**

**(5 % und 10 % Epoxid-Vernetzungsmittel/Trockenmasse)**

Die Steifigkeit wurde nach Stok *et al.* 2009 durch Kompressionstests mit einer Materialprüfmaschine (Zwick/Roell 1456, Ulm) mit einem 5 N Kraftaufnehmer gemessen. Eine geeignete Größe für den Prüfstempel wurde nach Spilker et al.; Journal of Biomechanical Engineering, Jg. 114, H, 2, S. 191-201; 1992 ermittelt; der Durchmesser betrug 1,38 mm. Die Probe befand sich in einer Halterung auf einem Mikrokoordinatentisch (siehe Abb.), wo sie mit PBS überschichtet wurde. Eine Plexiglasabdeckung verhinderte das Hochbiegen der Probe.

Der Stempel wurde stufenweise 5%, 15% und 25% der Probendicke tief in die Probe gefahren und gehalten, bis das System sich entspannt hatte. Der E-Modul wurde aus dem Mittelwert der letzten zehn Werte der Gleichgewichtsphase von drei Eindrückstufen bestimmt:

E-Modul [kPa] ist die Steigung aus der Dehnung (x) und Spannung (y)
Dehnung = Eindringtiefe [mm]/Dicke der Probe [mm]
Spannung = Kraft [mN] / Fläche des Stempels [$mm^2$]

Die Probe wurde für wenigstens 15 Minuten in PBS getränkt, in die Halterung gegeben und dort, mit PBS überschichtet, für wenigstens 5 Minuten ruhen gelassen. Als Oberfläche der Probe wurde die Einstellung definiert, bei der der Sensor 0,1 mN Widerstand erfasste. Die Ermittlung der Werte E erfolgte durch Dreifachmessung (n=3).

Die Versuchsanordnung ist beispielhaft dargestellt in Abbildung 9.

Ergebnis:

Collagenmatrices gemäß Herstellbeispiel 2a (5% Epoxid) zeigen eine Steifigkeit von 9 kPa.

Collagenmatrices gemäß Herstellbeispiel 2b (10% Epoxid) zeigen eine Steifigkeit von 22 kPa.

Eine unvernetzte Collagen-Elastin-Matrix (dehydrothermal vernetzt) zeigte eine Steifigkeit von lediglich 6 kPa.

**<u>Beispiel 12:</u>**

**Verwendung der vernetzten Collagenmatrices**

Die erfindungsgemäßen Materialien nach den Herstellbeispielen 1 bis 3 sind besonders geeignet zur Verwendung als kosmetisches und pharmazeutisches Mittel.

**Anwendungsbeispiel 12a**

**Verwendung als kosmetische Maske**

Die erfindungsgemäßen Materialien nach den Herstellbeispielen 1 bis 3 werden für die Behandlung auf die gewünschte Form und Größe der zu behandelnden Hautoreale zugeschnitten und entweder

a) auf die zu behandelnde Haut trocken aufgelegt und durch Besprühen mit Wasser oder einer Aktivatorlösung bis zur Sättigung befeuchtet oder
b) vor Auflegen auf die Haut mit Wasser oder einer Aktivatorlösung bis zur Sättigung befeuchtet und anschließend im rehydratisierten Zustand auf die zu behandelnden Hautoreale aufgelegt.

Die rehydratisierten Collagenauflagen wurden für einen Zeitraum von ca. 20 bis 30 min auf den behandelten Hautstellen belassen.
Während dieser Behandlung zeigte sich ein deutliches Abklingen vorhandener Rötungen oder Reizungen, sowie von vorhandenem Juckreiz, ein frischeres Aussehen, eine höhere Hautelastizität sowie eine verbesserte Hautfeuchte.

**Anwendungsbeispiel 12b**

**Verwendung als subkutanes Implantat**

Die erfindungsgemäßen Materialien nach den Herstellbeispielen 1 und 2 können als subkutanes Implantat im Bereich der plastischen oder rekonstruktiven Chirurgie mit dem Ziel des rekonstruktiven Volumenaufbaus eingesetzt werden, z.B. bei Verlust von Hautvolumen im Mittelgesichtsbereich aufgrund unterschiedlichster Ätiologie, rhinoplastischen Eingriffen oder auch bei ästhetisch rekonstruktiven Operationen, wie beispielsweise erforderlich nach Tumor-Operationen oder Traumata am gesamten Körper.
Vor der Implantation kann das Material im trockenen Zustand für das zu unterlegende Hautareal zugeschnitten und entsprechend angepasst werden.
Vor dem Einbringen der Matrix in die vorbereitete Hauttasche muß diese in reichlich steriler physiologischer Kochsalz- oder Ringerlösung rehydratisiert werden. Das Einlegen der Matrix in die präparierte subdermale Hauttasche erfolgt zügig unmittelbar nach abgeschlossener Rehydrierung der Matrix in steriler physiologischer Kochsalz- oder Ringerlösung. Der Wundschluss sollte entsprechend dem operativen Verfahren angepasst sein und ist durch den behandelnden Arzt zu entscheiden.

Wirkung der Matrix:

Bei Implantation wirkt die Matrix als Gerüststruktur und ermöglicht die Ansiedlung von Zellen, welche am Volumenaufbau der Haut beteiligt sind. Die eingewanderten Zellen bauen im Rahmen des Heilungsprozesses körpereigene Gewebestrukturen auf und sorgen so für den gewünschten Volumenaufbau im Bereich des eingebrachten Implantats. Mit dem Fortschreiten der Gewebeneogenese kann die Struktur der Matrix vollständig resorbiert und remodelliert werden.
Das nativ strukturierte Collagengerüst ist optimal dazu geeignet, das Einwachsen von Zellen und Gefäßen und damit die Gewebeneogenese zu unterstützen.
Der Einsatz der Matrix zwischen zwei unterschiedlichen Gewebeschichten kann zudem präventiv gegen Adhäsion und sekundäre Kontrakturen wirken.

**Anwendungsbeispiel 12c**

**Verwendung als Dermisersatz (Implantat)**

**(akute und chronische Wunden)**

Die erfindungsgemäßen Materialien nach den Herstellbeispielen 1 und 2 können bei tief dermalen Defekten und Vollhautwunden in der Verbrennungschirurgie, in der plastisch-rekonstruktiven Chirurgie
und in der Behandlung transplantationspflichtiger schlecht heilender Wunden (z.B. chronische Wunden) zum Dermisaufbau in Kombination mit autologen Spalthauttransplantaten verwendet werden.

Anwendung:

Das entsprechende Stück Material wird steril aus der Verpackung entnommen und gemäß dem Hautdefekt grob zurechtgeschnitten.
Nun wird das trockene Material auf die Wunde aufgelegt und zunächst in den Wundbereich mittels eines Bauchtuches eingepresst.
Das Material beginnt sich mit Wundsekret anzufeuchten und haftet blasenfrei sicher an dem Wundgrund. Die Ränder werden bis auf ein schmales Überlappen von ca. 2 mm zirkulär zurückgeschnitten.
Liegt das Material regelrecht im Wundgebiet, so wird mittels einer Spritze vorsichtig Kochsalzlösung aufgeträufelt; alternativ kann ein sehr nasses, mit Kochsalzlösung getränktes Bauchtuch aufgedrückt und für einige Minuten belassen werden. Nach dieser Zeit ist das Material komplett rehydriert und liegt entsprechend der vorherigen Platzierung exakt im Wundbett.
Die Transplantation der Spalthaut in das Wundareal erfolgt unmittelbar auf dem Material. Eine zusätzliche Befestigung des Materials gemeinsam mit der Spalthaut wird durch Nähte oder Klammern erreicht. Wird die Matrix erst nach zeitlicher Verzögerung durch das Spalthauttransplantat abgedeckt, sollte sichergestellt werden, dass sie nicht austrocknet. Hierfür eignet sich z.B. eine sterile, nichtadhärente Gaze getränkt mit physiologischer Kochsalzlösung.
Zur Wundabdeckung wird eine nicht okklusive Silikonfolie oder mehrere Lagen wirkstofffreier Fettgaze empfohlen, um ein feuchtes Wundmilieu zu gewährleisten, In der bisherigen Praxis hat es sich als zweckmäßig erwiesen, einen strammen Verband aus einer Kombination von 5-6 Schichten Fettgaze und 3-4 Lagen Mullverband zu verwenden. Die Verbandtechnik ist darauf auszulegen, einen guten Kontakt zwischen Spalthaut, Matrix und Wundgrund zu gewährleisten und Scherkräfte abzufangen. Die Anlage eines Unterdruckverbandes auf behandelten Wunden richtet sich nach der ärztlichen Entscheidung im einzelnen Fall. In vielen Fällen wurden gute Ergebnisse mit dieser Verbandtechnik erzielt.
Ziel der Behandlung ist der Aufbau einer Neodermis, um die Qualität der wiederhergestellten Haut zu verbessern, Vernarbung zu reduzieren und Wundkontraktion zu verhindern.

**Anwendungsbeispiel 12d**

**Verwendung als Mittel zur Blutstillung (Hämostyptikum)**

**(akute und chirurgische Wunden)**

Die erfindungsgemäßen Materialien nach den Herstellbeispielen 1 und 2 können als lokal anzuwendendes blutstillendes Mittel eingesetzt werden, bevorzugt bei Operationen mit venösen und diffus sickernden Blutungen z.B. in der Visceralchirurgie, Herz-Thorax-Gefäßchirurgie, Neurochirurgie, Kieferchirurgie und allgemeinen Stomatologie, HNO, Urologie und Gynäkologie,
**[0296]** Das Material wird vorzugsweise trocken auf die abgetupfte Wunde aufgelegt und leicht anmodelliert, es kann aber auch vor der Anwendung angefeuchtet werden. Bei stärkeren Blutungen kann mit einer feuchten Kompresse antamponiert werden. Größere Wundflächen werden mit mehreren Stücken versorgt, für kleine Flächen kann es mit der Schere entsprechend zugeschnitten werden.

Wirkungsweise:

Das Material nimmt das Mehrfache seines eigenen Gewichtes an Flüssigkeit auf. Die Thrombozyten aggregieren an der großen inneren Oberfläche und tragen durch freigesetzte Gerinnungsfaktoren zur lokalen Hämostase bei. Das entstehende Fibrin verankert die Collagenmatrix mit dem Wundgrund und bildet so einen stabilen Wundverschluss.
Durch diese Behandlung kann eine schnelle Blutstillung erreicht werden.

**Anwendungsbeispiel 12e**

**Verwendung als dermale / transdermale Wundauflage für exsudierende Wunden**

**(chronische Wunden)**

Die erfindungsgemäßen Materialien nach den Herstellbeispielen 1 und 2 können als Wundauflage für die lokale interaktive Wundbehandlung eingesetzt werden.
Zur optimalen Wirkung sollte das Material direkt auf das gesamte Wundbett aufgebracht werden. In Wunden mit geringer oder keiner Exsudation kann das Material mit Kochsalz- oder Ringerlösung angefeuchtet werden.
Die Wundauflage muss mit einem geeigneten Sekundärverband abgedeckt werden, um ein feuchtes Wundheilungsmilieu aufrecht zu erhalten. Nach dem ersten Aufbringen sollte die Wunde in Abhängigkeit von der Stärke des Exsudats in Abständen von bis zu 72 Stunden erneut mit dem Material behandelt werden. Noch nicht resorbierte Matrix kann dabei in der Wunde verbleiben.

Wirkungsweise:

Das Material kann das Mehrfache seines eigenen Gewichtes an Flüssigkeit aufnehmen und ist daher bestens zum Management der Wundflüssigkeit geeignet. Mit Aufnahme des Wundsekrets werden auch von der Wunde abgestoßene Nekrosen, Bakterien und Fibrinbeläge aufgenommen, wodurch die Bildung des Granulationsgewebe gefördert und beschleunigt werden kann. Beide Effekte tragen zu einer Beschleunigung der Wundheilung bei.

**Anwendungsbeispiel 12f**

**Verwendung in der Vakuum-unterstützten Wundbehandlungstherapie**

**(chronische Wunden)**

Für die Anwendung werden die erfindungsgemäßen Materialien nach den Herstellbeispielen 1 und 2 auf die zu behandelnden Körperpartien oder auf die Wunde trocken aufgebracht, wobei das Material, falls notwendig, auf die Form der Wunde zugeschnitten wird. Das Material wird dann entweder durch das vorhandene Wundsekret oder mit Wasser bzw. einer wässrigen Lösung oder physiologischer Kochsalzlösung durchfeuchtet und rehydratisiert. Es ist außerdem möglich, die erfindungsgemäßen Materialien vor dem Aufbringen auf die zu behandelnde Körperpartie anzufeuchten.

Anschließend wird die Wunde luftdicht mit einer herkömmlichen vakuumfesten Abdeckungsfolie verschlossen. Über herkömmliche Vorrichtungen zum Abführen der Wundflüssigkeit (Drainageeinheit) wird nach Anlegen eines geeigneten Unterdrucks (Vakuum) die Wundflüssigkeit nach herkömmlichen Vakuum-Therapieverfahren abgesaugt.

Durch diese Behandlung kann eine Verkleinerung der Wundfläche, ein Zusammenziehen der Wundränder, eine Verstärkung der Bildung von Granulationsgewebe und darüber eine Beschleunigung der Wundheilung erreicht werden.

Erläuterungen der Abbildungen:

Abb. 1: BCA-Methode: Bestimmung der löslichen Protein-Bestandteile [%] in einer Epoxy-vernetzten Collagenmatrix mit zusätzlichen löslichen Matrixproteinen (Elastin-Hydrolysat) gemäß Herstellbeispiel 2 im Vergleich zu einer ausschließlich dehydrothermal vernetzten Collagenmatrix mit Elastin-Hydrolysat ("unvernetzt).

Abb. 2a: Collagenaseverdau-Test: Bestimmung der Degradationsrate [%] erfindungsgemäßer Epoxy-vernetzter Collagenmatrices gemäß Herstellbeispielen 1a und 2a im Vergleich zu einer ausschließlich dehydrothermal vernetzten Collagenmatrix mit Elastin-Hydrolysat ("unvernetzt); innerhalb von 6 Stunden.

Abb. 2b: Collagenaseverdau-Test: Bestimmung der absoluten Menge des gelösten Anteils erfindungsgemäßer Epoxy-vernetzter Collagenmatrices (normiert auf 10 mg) gemäß Herstellbeispielen 1a und 2a im Vergleich zu einer ausschließlich dehydrothermal vernetzten Collagenmatrix mit Elastin-Hydrolysat ("unvernetzt); innerhalb von 23 Stunden.

Abb. 3: Hydrolysestabilitöt: a) dehydrothermal vernetzte Collagenmatrix ("unvernetzt") mit Triglyceriden/Neutralöl (Gefriertrocknungstemperatur > 120 °C), b) erfindungsgemäße Epoxy-vernetzte Collagenmatrix gemäß Herstellbeispiel 3 (5 % Epoxid/TM; mit Triglyceriden/Neutralöl; Gefriertrocknungstemperatur < 100 °C); c) Epoxy-vernetzte Collagenmatrix entsprechend der Zusammensetzung nach Herstellbeispiel 3 (5 % Epoxid/TM; mit Triglyceriden/Neutralöl; Gefriertrocknungstemperatur > 120 °C).

Abb. 4: Hydrolysegrad nach 18 Tagen a) einer dehydrothermal vernetzten Collagenmatrix im Vergleich zu b) einer erfindungsgemäßen Collagenmatrix nach Herstellbeispiel 3.

Abb. 5: Einfluss der Gefriertrocknungstemperatur auf die Nassreißfestigkeit (interne Methode UV8801)

Abb. 6: Einfluss der Gefriertrocknungstemperatur und der Epoxid-Vernetzerkonzentration auf die Nassreißfestigkeit (interne Methode UV8801)

Abb. 7: Einfluss der Standzeit und der Temperatur während der Standzeit der wässrigen Collagensuspension/-mischung vor dem Einfrieren auf die Nassreißfestigkeit (interne Methode UV8801).

Abb. 8: Epoxid-Restaktivität und Einfluss der Rückbefeuchtung auf die Abnahme der Epoxid-Restaktivität einer erfindungsgemäßen Collagenmatrix nach Herstellbeispiel 1.

Abb. 9: Versuchsaufbau zur Messung der Steifigkeit/Elastizitätsmodul E

**Patentansprüche**

1. Gefriergetrocknete Epoxy-vernetzte Collagenmatrix enthaltend unvernetzte, in der Anwendung freisetzbare säurelösliche Collagen- und/oder Peptidbestandteile und/oder Strukturbildner bzw. Wirkstoffe aus der Gruppe der Matrixproteine, extrazellulären Matrixbestandteile, proteinogenen Wirkstoffe und löslichen Protein- oder Peptidbestandteile, nachweisbar mittels der BCA-Methode, vorzugsweise zu einem Anteil von insgesamt mindestens 0,1 %, bezogen auf die gefriergetrocknete Collagenmatrix, gemessen nach der BCA-Methode.

2. Gefriergetrocknete Epoxy-vernetzte Collagenmatrix nach Anspruch 1, mit einer Nassreißfestigkeit >50 cN/mm Schichtdicke gemessen mittels DIN EN ISO 3376 oder > 200 cN/mm Schichtdicke gemessen mittels Methode UV8801.

3. Gefriergetrocknete Epoxy-vernetzte Collagenmatrix nach einem der Ansprüche 1 oder 2, mit einer Collagenase Degradationsrate von maximal 85 %, entsprechend dem Anteil an Zersetzungsprodukten nach Collagenaseverdau in phosphatgepufferter Salzlösung (PBS), gemessen mittels UV/VIS Spektralphotometer zum Reaktionszeitpunkt 6 Stunden nach Zugabe der Collagenase.

4. Gefriergetrocknete Epoxy-vernetzte Collagenmatrix nach einem der Ansprüche 1 bis 3, mit einer Hydrolysestabilität größer oder gleich 90 %, bestimmbar über die Massenabnahme nach Einlegen und Lagerung der Collagenmatrix nach einem der Ansprüche 1 bis 3 in einer wässrigen Lösung, enthaltend Wasser, Glycerin, 1,5-Pentandiol, Natriumchlorid und ein Konservierungsmittel, für 18 Tage bei 50 °C über die Formel

$$\text{Hydrolysestabilität (\%)} = M_{tx} \times 100 / M_{t0}$$

mit

$M_{tx}$ = Masse des getrockneten intakten/zusammenhängenden Matrixmaterials
$m_{t0}$ = Masse des trockenen Matrixmaterials vor Untersuchungsbeginn.

5. Gefriergetrocknete Epoxy-vernetzte Collagenmatrix nach einem der Ansprüche 1 bis 4, mit einer Steifigkeit, entsprechend einem Elastizitätsmodul nach Thomas Young (Young's Modul) E von 0,01 bis 100 kPa.

6. Gefriergetrocknete Epoxy-vernetzte Collagenmatrix nach einem der Ansprüche 1 bis 5, mit einer Flüssigkeitsaufnahme- und - speicherkapazität des 1 bis 200fachen ihres Eigengewichts.

7. Gefriergetrocknete Epoxy-vernetzte Collagenmatrix nach einem der Ansprüche 1 bis 6, die mindestens einen weiteren Strukturbildner, mindestens einen kosmetischen oder pharmazeutischen Wirkstoff und/oder mindestens einen Hilfsstoff enthält.

8. Gefriergetrocknete Epoxy-vernetzte Collagenmatrix nach einem der Ansprüche 1 bis 6, die in Form von schichtförmigen Auflagen, Sheets, Pads oder Masken vorliegt, die gegebenenfalls ganz oder teilweise mit einer weiteren Schicht versehen sein können, die ausgewählt ist aus Fasern, Vliesen, Netzen, Filmen oder Folien oder einer selbstklebenden Schicht die derart auf dem schichtförmigen Collagenmaterial aufgebracht ist, dass sie mit diesem randseitig abschließt oder randseitig vollständig oder partiell über das Collagenmaterial hinausragt.

9. Gefriergetrocknete Epoxy-vernetzte Collagenmatrix nach einem der Ansprüche 1 bis 8, enthaltend mindestens einen Wirkstoff ausgewählt aus der Gruppe der dermalen und transdermalen Wirkstoffe zur Hautbehandlung, zur Behandlung von Hauterkrankungen, zur Wundbehandlung oder zur Blutstillung, insbesondere solche Wirkstoffe, die ausgewählt sind aus der Gruppe die umfasst: Matrixproteine, lösliche Peptidbestandteile wie insbesondere Elastin und/oder Wachstumsfaktoren, antibakterielle Wundbehandlungsmittel wie insbesondere Silber-haltige Wirkstoffe, insbesondere Silbernitrat und/oder mindestens einen Hilfsstoff, der ausgewählt ist aus der Gruppe der kosmetischen Öle und Fette, wie insbesondere Triglyceride oder Neutralöl.

10. Gefriergetrocknete Epoxy-vernetzte Collagenmatrix nach einem der Ansprüche 1 bis 9, zur Verwendung als pharmazeutisches Mittel, insbesondere zur Verwendung als Mittel zur Behandlung von akuten und/oder chronischen Wunden, als Implantat, als dermales oder transdermales Hautbehandlungsmittel, als Mittel zur Blutstillung und/oder zur Verwendung in einer Vakuum-unterstützten Wundbehandlungstherapie.

11. Verwendung der gefriergetrockneten Epoxy-vernetzten Collagenmatrix nach einem der Ansprüche 1 bis 9, als kosmetisches Mittel, als kosmetische Auflage oder Maske oder als Gerüststruktur zur Zellbesiedelung.

12. Kombinationspräparat enthaltend mindestens eine gefriergetrocknete Epoxy-vernetzte Collagenmatrix gemäß einem der Ansprüche 1 bis 9, sowie mindestens eine wässrige Lösung, die einen oder mehrere Wirkstoffe und/oder gegebenenfalls einen oder mehrere Hilfsstoffe enthält, in zusammengehöriger, räumlicher Anordnung als Set oder Kit-of-parts Kombination.

ABBILDUNGEN

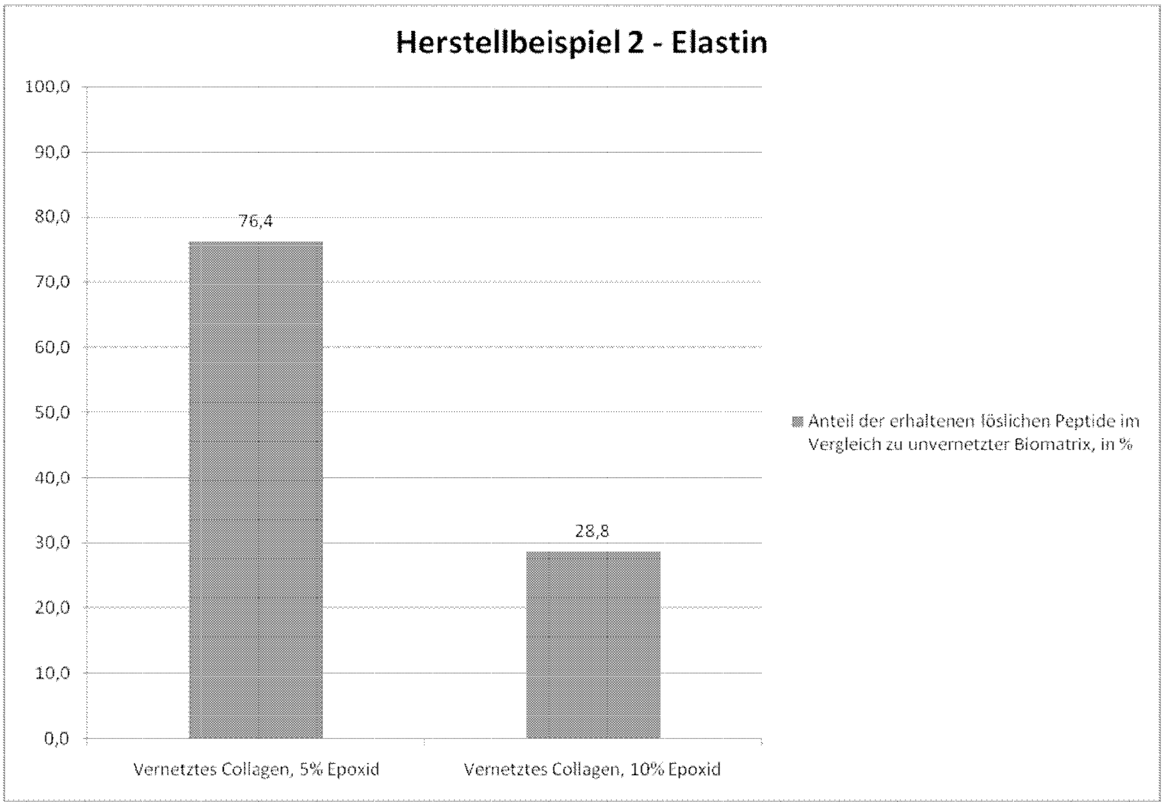


Abb. 1

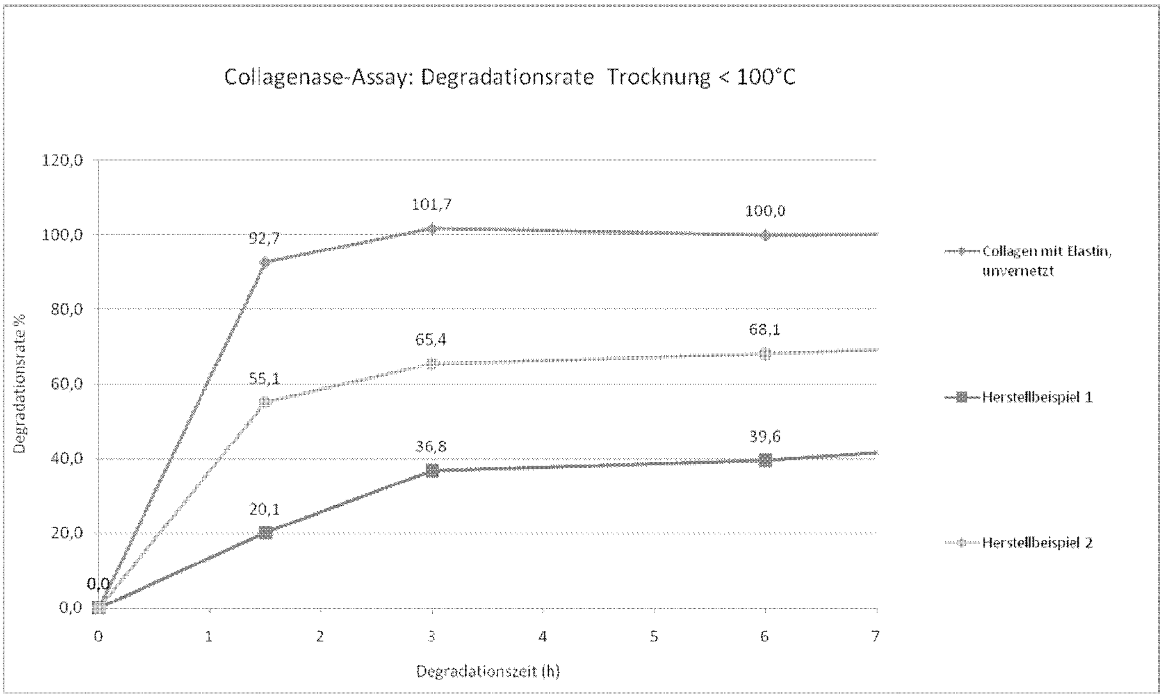


Abb. 2a

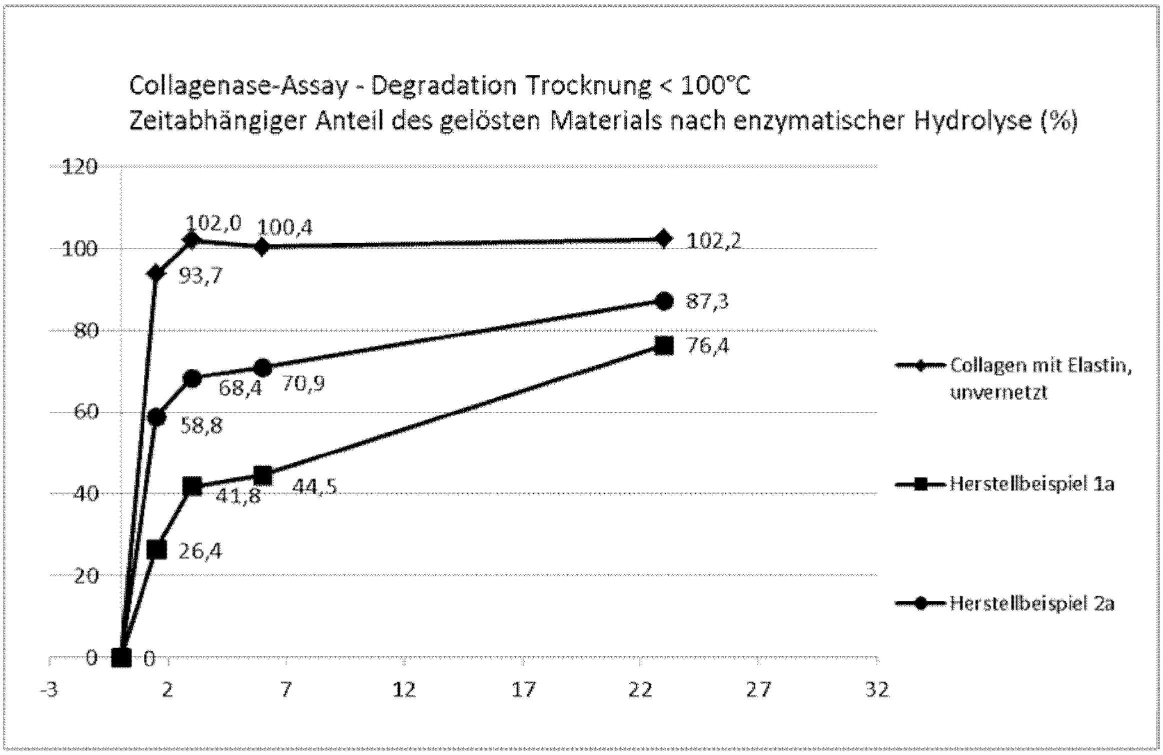
Collagenase-Assay - Degradation Trocknung < 100°C
Zeitabhängiger Anteil des gelösten Materials nach enzymatischer Hydrolyse (%)
120
100
80
60
40
20
0
-3
2
7
12
17
22
27
32
102,0
100,4
102,2
93,7
87,3
76,4
68,4
70,9
58,8
41,8
44,5
26,4
0
Collagen mit Elastin, unvernetzt
Herstellbeispiel 1a
Herstellbeispiel 2a

Abb. 2b

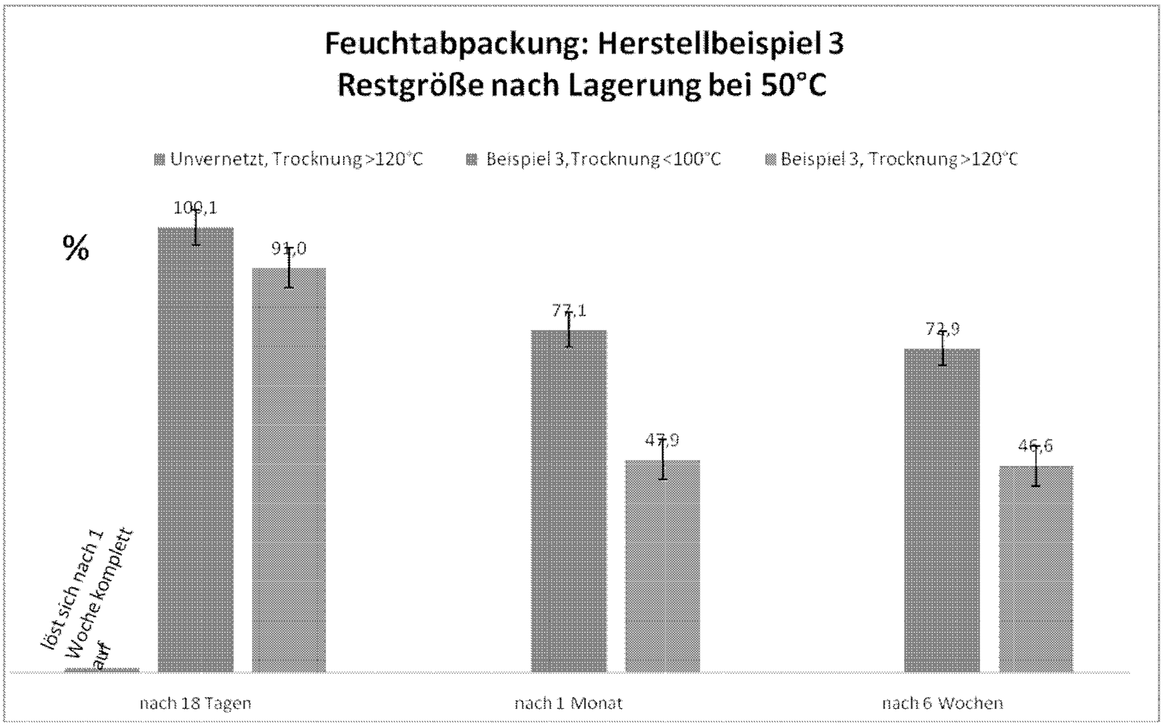
Feuchtabpackung: Herstellbeispiel 3
Restgröße nach Lagerung bei 50°C
Unvernetzt, Trocknung >120°C
Beispiel 3, Trocknung <100°C
Beispiel 3, Trocknung >120°C
%
100,1
91,0
77,1
47,9
72,9
46,6
löst sich nach 1 Woche komplett auf
nach 18 Tagen
nach 1 Monat
nach 6 Wochen

Abb. 3

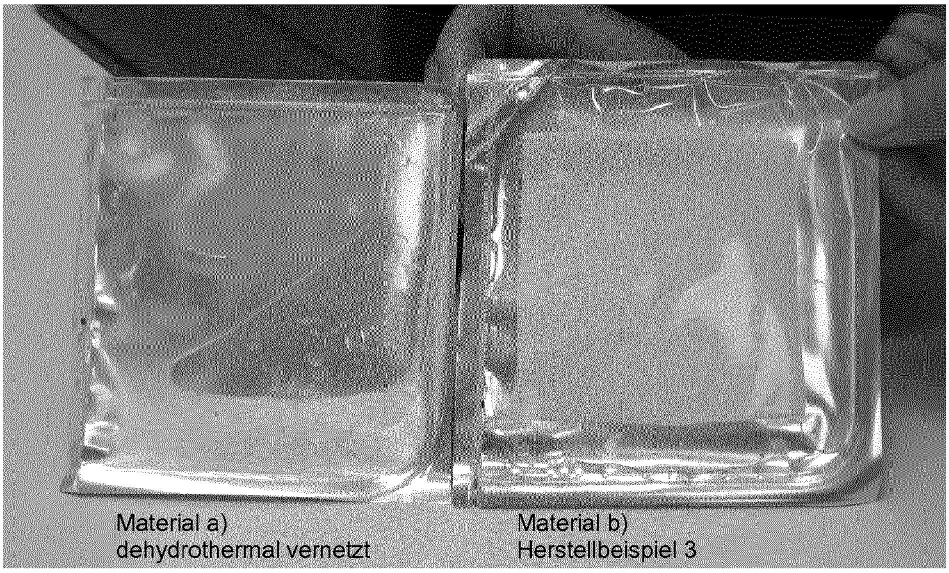
Material a)
dehydrothermal vernetzt
Material b)
Herstellbeispiel 3

Abb. 4

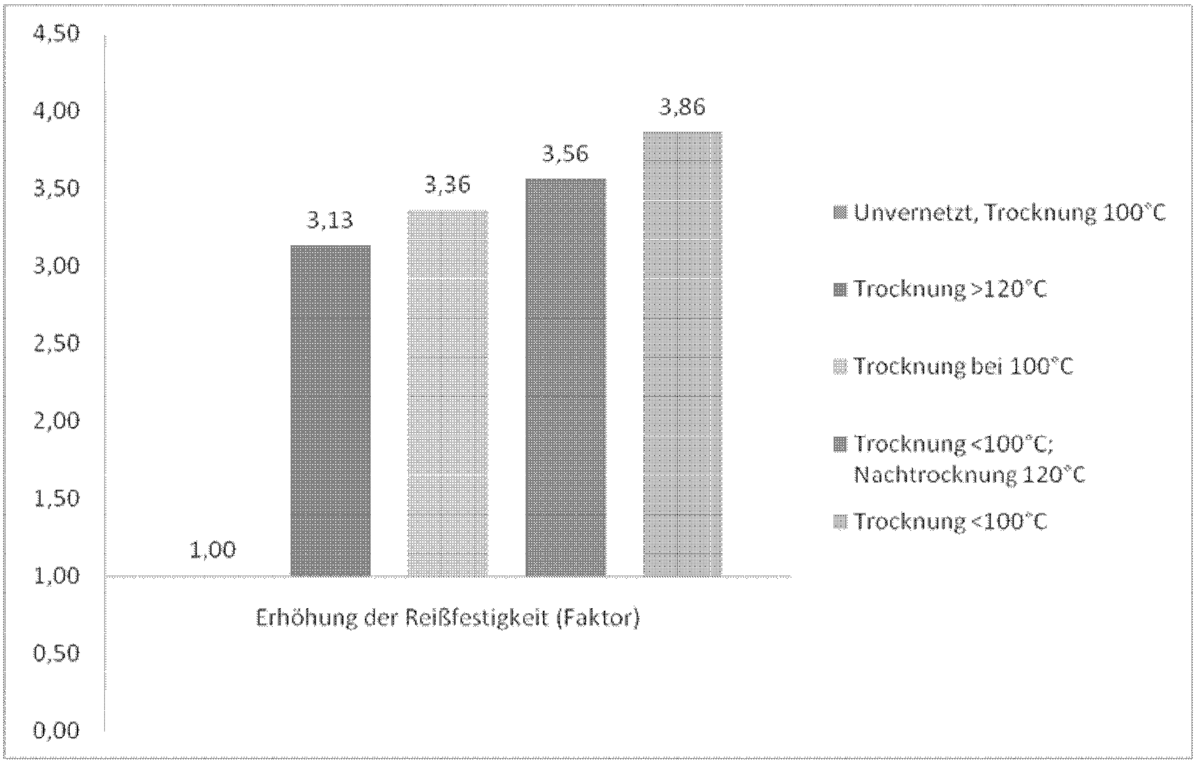
4,50
4,00
3,50
3,00
2,50
2,00
1,50
1,00
0,50
0,00
1,00
3,13
3,36
3,56
3,86
Erhöhung der Reißfestigkeit (Faktor)
Unvernetzt, Trocknung 100°C
Trocknung >120°C
Trocknung bei 100°C
Trocknung <100°C;
Nachtrocknung 120°C
Trocknung <100°C

Abb. 5

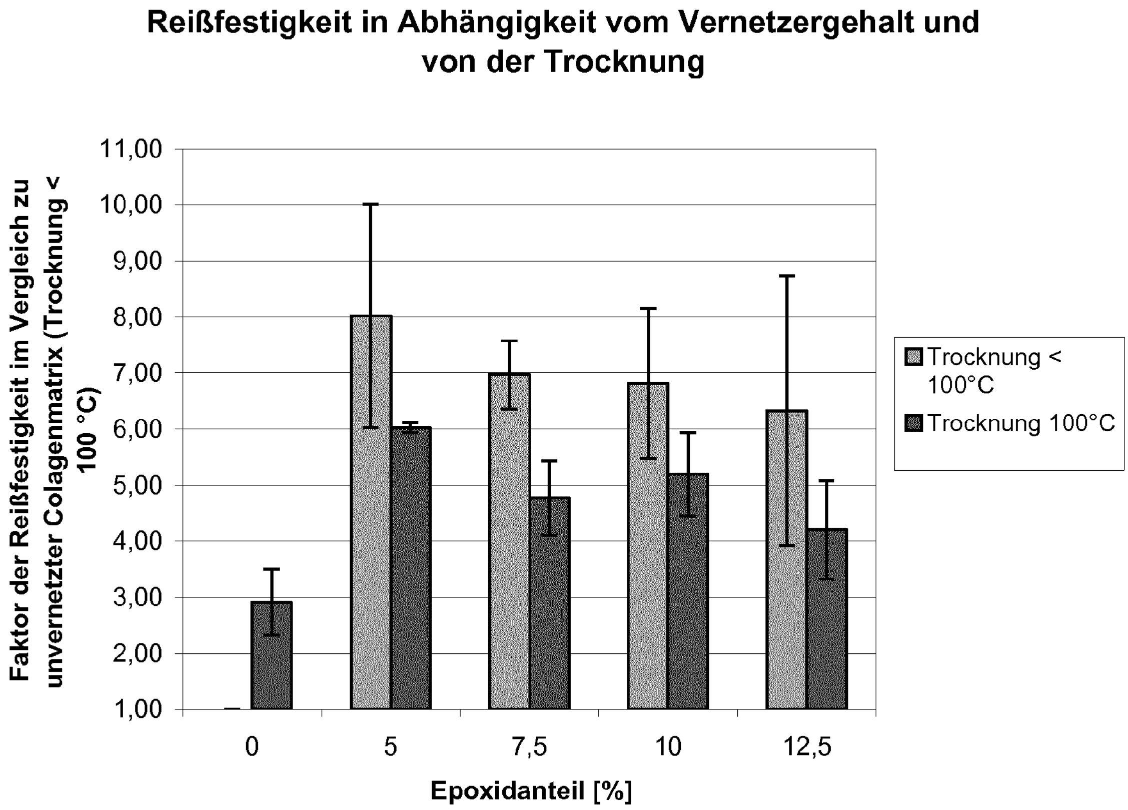
Reißfestigkeit in Abhängigkeit vom Vernetzergehalt und von der Trocknung
Faktor der Reißfestigkeit im Vergleich zu unvernetzter Colagenmatrix (Trocknung < 100 °C)
11,00
10,00
9,00
8,00
7,00
6,00
5,00
4,00
3,00
2,00
1,00
0
5
7,5
10
12,5
Epoxidanteil [%]
Trocknung < 100°C
Trocknung 100°C

Abb. 6

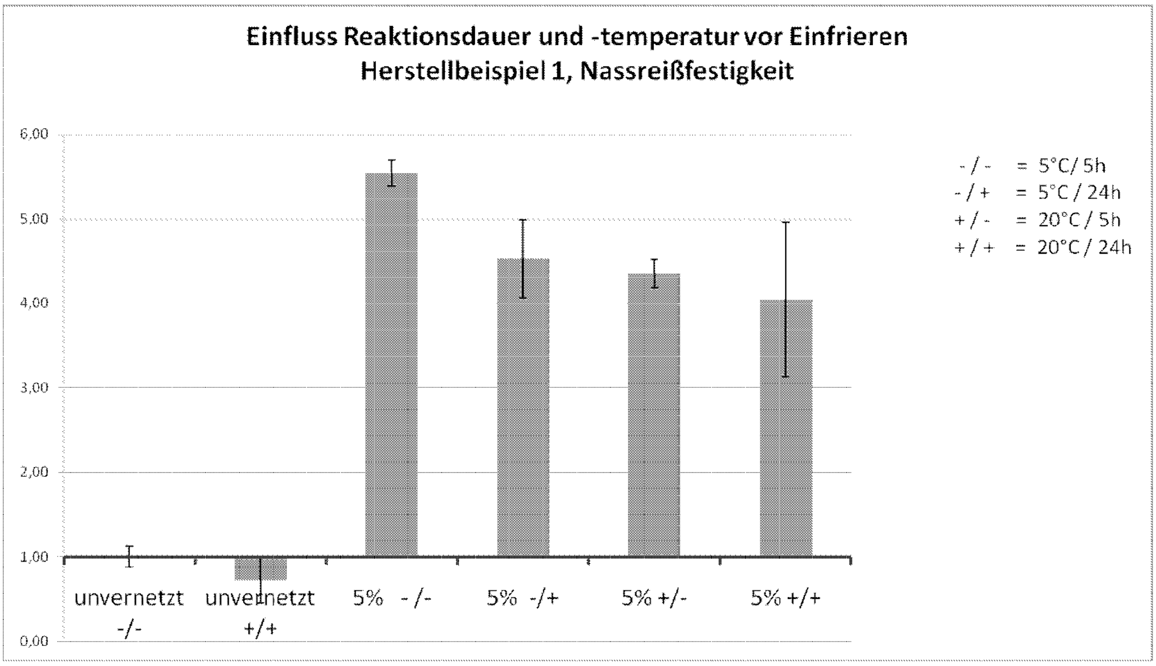
Einfluss Reaktionsdauer und -temperatur vor Einfrieren
Herstellbeispiel 1, Nassreißfestigkeit
6,00
5,00
4,00
3,00
2,00
1,00
0,00
unvernetzt -/-
unvernetzt +/+
5% -/-
5% -/+
5% +/-
5% +/+
-/- = 5°C/5h
-/+ = 5°C/24h
+/- = 20°C/5h
+/+ = 20°C/24h

Abb. 7

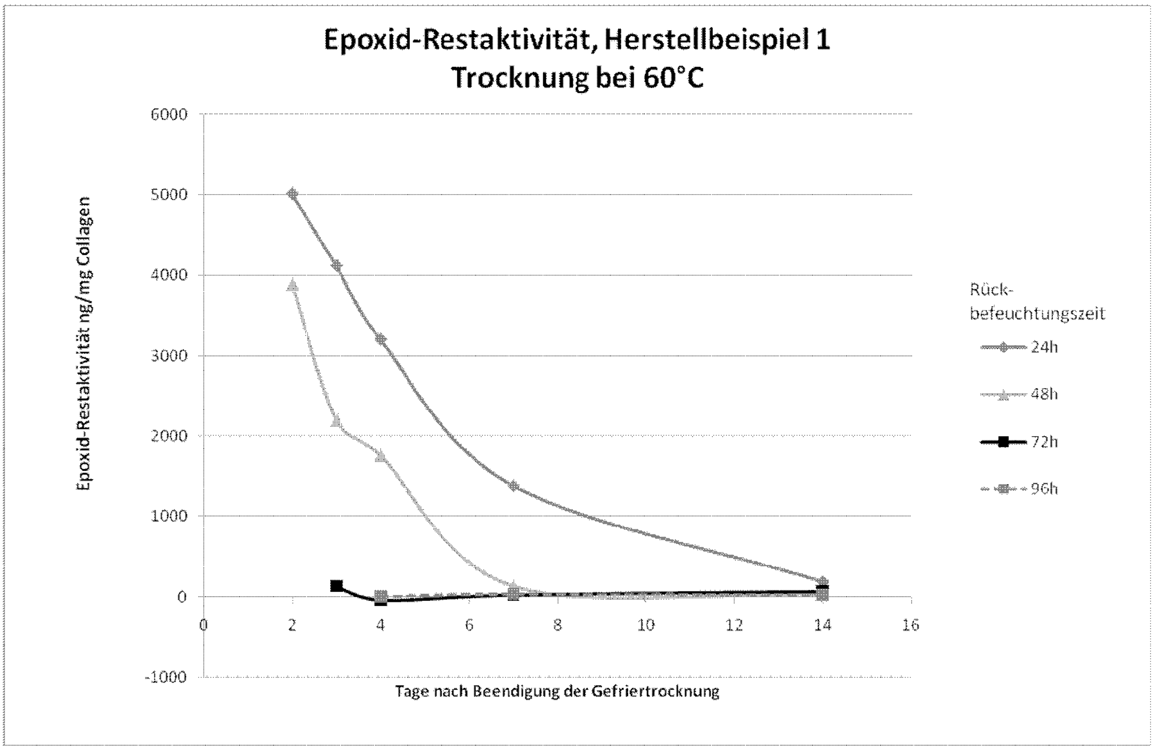
Epoxid-Restaktivität, Herstellbeispiel 1
Trocknung bei 60°C
Epoxid-Restaktivität ng/mg Collagen
6000
5000
4000
3000
2000
1000
0
-1000
0
2
4
6
8
10
12
14
16
Tage nach Beendigung der Gefriertrocknung
Rück-
befeuchtungszeit
24h
48h
72h
96h

Abb. 8

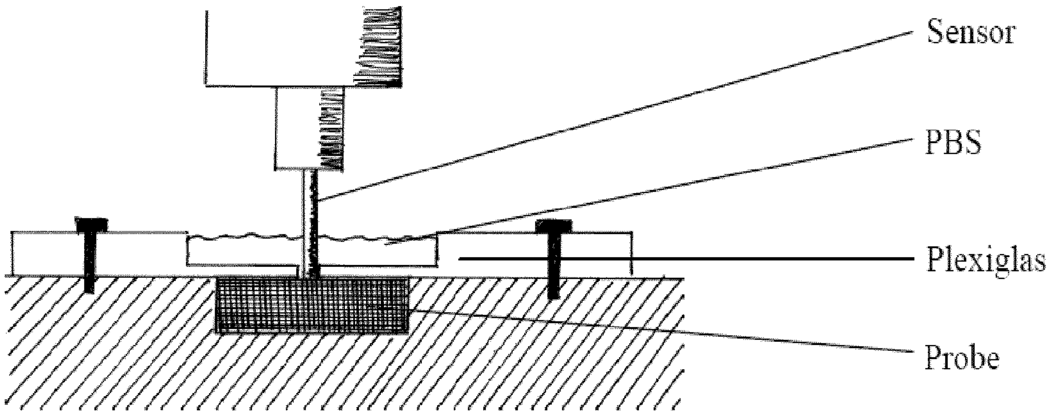
Sensor
PBS
Plexiglas
Probe

Abb. 9

Europäisches Patentamt
European Patent Office
Office européen des brevets

# EUROPÄISCHER RECHERCHENBERICHT

Nummer der Anmeldung
EP 14 16 9413

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 103 50 654 A1 (SUWELACK SKIN & HEALTH CARE AG [DE]) 2. Juni 2005 (2005-06-02)<br>* Absatz [0017] - Absatz [0038] *<br>* Absätze [0090], [0114]; Ansprüche * | 1-12 | INV.<br>A61L15/32<br>A61L27/24<br>A61K8/65<br>A61Q19/00<br>A61K8/02 |
| A | DE 10 2006 038629 A1 (SUWELACK SKIN & HEALTH CARE AG [DE])<br>21. Februar 2008 (2008-02-21)<br>* das ganze Dokument * | 1 | |
| A | EP 0 898 973 A2 (MEDTRONIC INC [US])<br>3. März 1999 (1999-03-03)<br>* das ganze Dokument * | 1-12 | |
| A | US 4 883 864 A (SCHOLZ MATTHEW T [US])<br>28. November 1989 (1989-11-28)<br>* das ganze Dokument * | 1-12 | |

| RECHERCHIERTE SACHGEBIETE (IPC) |
|---|
| A61L<br>A61K<br>A61Q |

Der vorliegende Recherchenbericht wurde für alle Patentansprüche erstellt

1

| Recherchenort | Abschlußdatum der Recherche | Prüfer |
|---|---|---|
| Den Haag | 8. September 2014 | Derrien, Anne-Cécile |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 14 16 9413

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-09-2014

| Im Recherchenbericht angeführtes Patentdokument | Datum der Veröffentlichung | Mitglied(er) der Patentfamilie | Datum der Veröffentlichung |
|---|---|---|---|
| DE 10350654 A1 | 02-06-2005 | KEINE | |
| DE 102006038629 A1 | 21-02-2008 | CA 2660722 A1 | 21-02-2008 |
| | | DE 102006038629 A1 | 21-02-2008 |
| | | EP 2079433 A1 | 22-07-2009 |
| | | JP 5165683 B2 | 21-03-2013 |
| | | JP 2010500986 A | 14-01-2010 |
| | | US 2010226982 A1 | 09-09-2010 |
| | | WO 2008020066 A1 | 21-02-2008 |
| EP 0898973 A2 | 03-03-1999 | DE 69828665 D1 | 24-02-2005 |
| | | DE 69828665 T2 | 12-01-2006 |
| | | EP 0898973 A2 | 03-03-1999 |
| | | US 6117979 A | 12-09-2000 |
| US 4883864 A | 28-11-1989 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 8504413 A
- US 5219576 A
- EP 0428541 B1
- DE 69533285
- WO 2003053490 A1
- EP 1455855 A1
- DE 102006006461 A1
- DE 102004039537 A1
- EP 0898973 B1
- EP 0793511 A1
- EP 0680990 A1
- US 4883864 A
- DE 10350654 A1
- DE 4048622 A1
- DE 3203957 A1
- US 20070027414 A


### In der Beschreibung aufgeführte Nicht-Patentliteratur


- **RUI MIGUEL PAZ.** *Dissertation RWTH Aachen,* 2004
- **R. ZEEMAN.** Cross-linking of Collagen-based materials. *Dissertation,* 1998
- **ZEEMAN et al.** *J Biomed Mater Res,* 1999, vol. 46, 424-433
- *J Biomed Mater Res,* 1999, vol. 47, 270-277
- *Biomaterials,* 1999, vol. 20, 921-931
- *J Biomed Mater Res,* 2000, vol. 51, 541-548
- **AGARWAL et al.** Detection of Epoxides with 4-(p-Nitrobenzylpyridine. *Bull. Environm. Contam. Toxicol.,* 1979, vol. 23, 825-829
- **ZOCHER et al.** Epoxide hydrolase activity of Streptomyces strains. *J. Biotechnol.,* 17. Februar 2000, vol. 77 (2-3), 287-92
- CTFA-Abhandlung, Cosmetic Ingredient Hand-book. The Cosmetic, Toiletry and Fragrance Association, Inc, 1988
- **MIH et al.** *PLoS ONE,* 2011, vol. 6 (5), e19929
- **YANG et al.** *Biophysical Journal,* 2009, vol. 97, 2051-2060
- **KRISHNAN et al.** *Cell Adhesion & Migration,* 2008, vol. 2 (2), 83-94
- **SPILKER et al.** *Journal of Biomechanical Engineering, Jg. 114, H,* 1992, vol. 2, 191-201